(19) Europäisches Patentamt
European Patent Office
Office européen des brevets

(11) EP 4 606 819 A1

(12) **EUROPEAN PATENT APPLICATION**
published in accordance with Art. 153(4) EPC

(43) Date of publication:
**27.08.2025 Bulletin 2025/35**

(21) Application number: **23880209.4**

(22) Date of filing: **18.10.2023**

(51) International Patent Classification (IPC):
***C07K 16/00*** *(2006.01)* ***C07K 16/32*** *(2006.01)*
***A61P 35/00*** *(2006.01)* ***A61K 39/00*** *(2006.01)*

(52) Cooperative Patent Classification (CPC):
**A61K 39/00; A61K 47/68; A61P 35/00; C07K 16/00; C07K 16/18; C07K 16/32; G01N 33/563**

(86) International application number:
**PCT/KR2023/016105**

(87) International publication number:
**WO 2024/085632 (25.04.2024 Gazette 2024/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.10.2022 KR 20220133722**

(71) Applicant: **Crosspoint Therapeutics Inc.**
**Hwaseong-si, Gyeonggi-do 18469 (KR)**

(72) Inventors:
- **JUNG, Sang Taek**
  **Seoul 06217 (KR)**
- **KIM, Su Yeon**
  **Seoul 07211 (KR)**
- **JO, Mi Gyeong**
  **Seoul 04575 (KR)**

(74) Representative: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

Remarks:
The complete document including Reference Table(s) and the Sequence Listing(s) can be downloaded from the EPO website

# (54) HUMAN ANTIBODY FC DOMAIN VARIANT AND USE THEREOF

(57) The present invention relates to a human antibody Fc domain variant and a use thereof and, more specifically, to a human antibody Fc domain variant and a use thereof as an antibody-drug conjugate, wherein the human antibody Fc domain variant has proline (P) substituted for the 235th amino acid of the wild-type human antibody Fc domain and thus lacks or has substantially no binding ability to human FcγRs and C1q, with the consequent deficiency of antibody-dependent cellular cytotoxicity (ADCC), antibody-dependent cell-mediated phagocytosis (ADCP), and complement-dependent cytotoxicity (CDC).

EP 4 606 819 A1

[FIG. 12A]

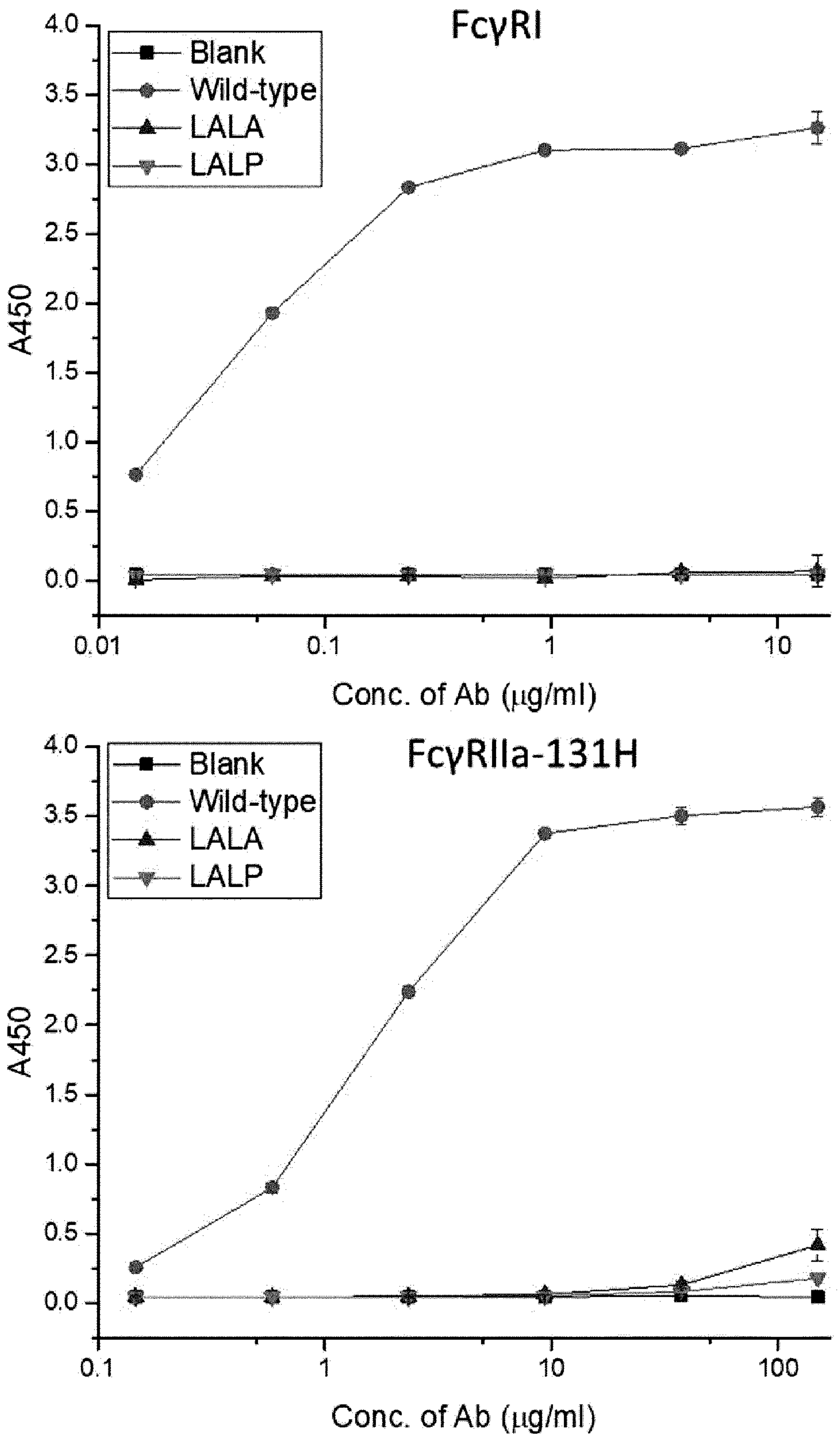
FcγRI
Blank
Wild-type
LALA
LALP
A450
Conc. of Ab (μg/ml)
FcγRIIa-131H
Blank
Wild-type
LALA
LALP
A450
Conc. of Ab (μg/ml)

## Description

[Technical Field]

The present invention relates to a human antibody Fc domain variant and a use thereof.

[Background Art]

Since protein therapeutics show very high specificity for disease targets and low side effects and toxicity, they are rapidly replacing non-specific small molecule compound therapeutics and are widely used in clinical practice. Among protein therapeutics currently used clinically, antibody therapeutics are Fc-fusion protein therapeutics that are fused with the Fc region of an antibody are mainly used.

Therapeutic antibodies are considered one of the most effective cancer treatment methods because they show very high specificity for a target compared to existing small molecule drugs, have low biotoxicity and fewer side effects, and have an excellent blood half-life of about 3 weeks. In fact, large pharmaceutical companies and research institutes around the world are accelerating the research and development of therapeutic antibodies that specifically bind to cancer cells, including cancer-causing factors, and effectively remove the same.

Antibodies provide a link between the humoral and cellular immune systems; while the Fab region of an antibody recognizes an antigen. On the other hand, the Fc domain portion is bound to a receptor (Fc receptor or FcR) to antibodies (immunoglobulins) on cells that are differentially expressed by all immunocompetent cells, and has different mechanisms depending on the type of FcyR expressed on the surface of the immune cell to which it binds. The Fc receptor binding site on the antibody Fc region binds to the Fc receptor (FcR) on the cell, such that, when the antibody binds to the Fc receptor on the cell surface through the Fc region, it triggers several important and diverse biological reactions, including phagocytosis and destruction of antibody-coated particles, removal of immune complexes, lysis of antibody-coated target cells (antibody-dependent cellular cytotoxicity, or ADCC) by killer cells, release of inflammatory mediators, placental migration and control of immunoglobulin production.

The Fc domain plays a critical role in the collection of immune cells, the antibody-dependent cellular cytotoxicity (ADCC) and antibody-dependent cellular phagocytosis (ADCP). In particular, the ADCC and ADCP functions, which are the effector functions of antibodies, rely on interaction with Fc receptor present on the surface of many cells. Human Fc receptors are classified into five types, wherein the type of collected immune cells is determined depending on which Fc receptor the antibody binds to. For example, the Fc domain of an antibody binds to FcγRIIIa to induce ADCC, binds to FcyRI or FcyRIIa to induce ADCP, and binds to blood complement C1q to activate a complement dependent cytotoxicity (CDC) action mechanism, thereby being toxic to the target antigen bound to Fab region. Therefore, it is responsible for the main therapeutic effects of therapeutic antibodies.

The mechanism of action by this antibody Fc region induces a death mechanism of defective cells (e.g., cancer, infected cells), and is an important action mechanism of therapeutic antibodies. However, in the case where a target antigen of a therapeutic antibody such as immune checkpoint inhibitor antibody that binds to immune cells, immune cell-derived double antibody (bispecific immune cell engaging bispecific antibody), etc. is expressed on immune cells rather than defective cells (cancer, infected cells), if the Fc action mechanism remains, normal immune cells bound with the antibody, which are beneficial for immune action, may be destroyed or activate host immune defenses, which may lead to safety issues and undesired side effects.

For example, some therapeutic antibodies, such as immune checkpoint inhibitors that bind to immune cells, bispecific immune cell engaging bispecific antibody (abbrev. to bispecific antibody), etc., have a problem of side effects that destroy immune cells by triggering an immune action mechanism on targeted immune cells. Further, the immune checkpoint inhibitor (e.g., Keytruda, Opdivo, Libtayo, etc.) targeting an immune checkpoint protein expressed on the surface of immune cell having T-cell entails a disadvantage of deteriorating original effects of the antibody due to side effects in that, if Fc-mediated action mechanism remains, the immune cells themselves, which should be used to kill cancer cells, are destroyed.

Further, the bispecific antibody as an antibody therapeutic agent, which binds to an antigen on the surface of a cancer cell on one side while being bound to an immune cell on the other side, and which may act to guide the immune cell to the cancer cell thus to more efficiently remove the cancer cell, causes side effects because the immune cell is destroyed and the cancer cell could not be effectively removed if Fc-mediated immune action mechanism is present in the corresponding antibody. Further, agonist antibodies that bind to target cells to induce cell activation, or antagonist antibodies that block an interaction between the target antigen and the ligand are toxic to target cells and antigens due to the Fc-mediated immune action mechanism, hence causing a problem of deteriorating the original effect of the antibody. In addition, when developing an Fc-fusion protein in which the Fc region is fused to increase the half-life of an active substance such as a protein or compound according to purposes for therapeutic, diagnostic or research, there is a problem of appearing toxicity due to the Fc-mediated immune action mechanism.

Therefore, in order to solve the problem of causing immune cell death side effects/off-target toxicity to normal cells while having excellent efficacy of the antibody due to target antigen-specific binding, removal of the Fc action mechanism is essential. To this end, when developing antibodies, IgG2 antibodies, which have the lowest binding ability to FcγR among the human IgG subclasses and thus have a very low immune action mechanism, are considered. However, IgG2 antibodies have several allotypes due to disulfide bond exchange in a hinge region. Further, there are physical property problems such as aggregation due to the decreased stability. Therefore, IgG4 antibodies with next lower binding ability are being considered and are currently being used in clinical development. However, the IgG4 antibodies also have a disadvantage of causing Fab-arm exchange of IgG antibody molecule due to lack of stability of antibody hinge region. Furthermore, these antibodies still have significant binding ability to FcγRs, and in particular, have quite strong binding ability to FcγRI (equilibrium dissociation constant of several nM), hence involving a problem with activation of various Fc action mechanisms. Accordingly, in order to prevent target cells from being destroyed by the immune action mechanism of the antibody, Fc with removed the binding ability to FcγRs is needed.

[Summary of Invention]

[Problems to be Solved by Invention]

An object of the present invention is to provide a novel human antibody Fc domain variant.
Another object of the present invention is to provide a novel antibody with reduced effector function or a fragment thereof having immunological activity.
In addition, another object of the present invention is to provide a novel antibody-drug conjugate.
Further, another object of the present invention is to provide a pharmaceutical composition for treating or preventing cancer.
Furthermore, another object of the present invention is to provide a method for treating cancer.

[Means for Solving Problems]

1. A human antibody Fc domain variant in which amino acid 235 of the human antibody Fc domain is replaced with proline (P).
2. The human antibody Fc domain variant according to the above 1, wherein the human antibody Fc domain including an amino acid sequence of SEQ ID NO: 15.
3. The human antibody Fc domain variant according to the above 1, wherein amino acid 234 of the human antibody Fc domain is further substituted with alanine (A).
4. The human antibody Fc domain variant according to the above 1, wherein amino acid 265 of the human antibody Fc domain is further substituted with leucine (L), methionine (M) or asparagine (N).
5. The human antibody Fc domain variant according to the above 1, wherein amino acid 329 of the human antibody Fc domain is further substituted with valine (V), leucine (L), or glycine (G).
6. The human antibody Fc domain variant according to the above 1, wherein amino acid 268 of the human antibody Fc domain is further substituted with glutamine (Q).
7. The human antibody Fc domain variant according to the above 1, wherein amino acid 296 of the human antibody Fc domain is further substituted with phenylalanine (F).
8. The human antibody Fc domain variant according to the above 1, wherein amino acid 300 of the human antibody Fc domain is further substituted with phenylalanine (F).
9. The human antibody Fc domain variant according to the above 1, wherein amino acid 333 of the human antibody Fc domain is further substituted with valine (V).
10. The human antibody Fc domain variant according to the above 1, wherein the human antibody is IgG1.
11. An antibody including the human antibody Fc domain variant according to any one of the above 1 to 10, or a fragment thereof having immunological activity.
12. An antibody-drug conjugate in which the antibody according to the above 11 or a fragment thereof having immunological activity is conjugated to one or more drugs.
13. The antibody-drug conjugate according to the above 12, wherein the drug is any one selected from the group consisting of: chimeric antigen receptor (CAR) cell therapeutics, oncolytic drug, immunotherapy agent, cytotoxic agent, angiogenesis inhibitor, kinase inhibitor, costimulatory molecule blocker, adhesion molecule blocker, anti-cytokine agent, nucleic acid therapeutics, anti-CTLA-4 agent, anti-PD-1 agent, anti-PD-L1 agent, anti-PD-L2 agent, TNF-$\alpha$ cross-linking agent, TRAIL cross-linking agent, anti-CD27 agent, anti-CD30 agent, anti-CD40 agent, anti-4-1BB agent, anti-GITR agent, anti-OX40 agent, anti-TRAILR1 agent, anti-TRAILR2 agent, tagretin, interferon-alpha, clobetasol, peg interferon, prednisone, romidepsin, bexarotene, methotrexate, triamcinolone cream, anti-chemokine, vorinostat, gabapentin, cyclosporine, rapamycin, FK506, detectable marker or reporter, TNF antagonist, antirheumatic agent, muscle relaxants, narcotics, non-steroidal anti-inflammatory drugs (NSAIDs), analgesics, anesthetics, sedatives, local anesthetics, neuromuscular blocker, antibacterial agent, psoriasis therapeutics, corticosteroid, anabolic steroid, erythropoietin, immunizations, immunoglobulin, immunosuppressant, growth hormone, hormone replacement drug, radiopharmaceuticals, antidepressant, antipsychotics, stimulant, asthma drug, beta agonist, inhaled steroid, epinephrine or its analogues, cytokine, cytokine antagonist, PD-1 antagonist, adenosine A2AR antagonist, CD73 inhibitor, CTLA-4 inhibitor, TIM-3 inhibitor, LAG-3 inhibitor, anthracycline, antisense oligonucleotide (ASO), siRNA and aptamer.

14. A pharmaceutical composition for treatment or prevention of cancer, including: the human antibody Fc domain variant according to any one of the above 1 to 10; an antibody including the same or a fragment thereof having immunological activity; or the antibody-drug according to the above 12 or 13.

15. The pharmaceutical composition according to the above 14, wherein the cancer is selected from the group consisting of: brain tumor, melanoma, myeloma, non-small cell lung cancer, oral cancer, liver cancer, stomach cancer, colon cancer, breast cancer, lung cancer, bone cancer, pancreatic cancer, skin cancer, head or neck cancer, cervical cancer, ovarian cancer, colon cancer, small intestine cancer, rectal cancer, fallopian tube carcinoma, anal cancer, endometrial carcinoma, vaginal carcinoma, vulvar carcinoma, Hodgkin's disease, esophageal cancer, lymph node cancer, bladder cancer, gallbladder cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, renal or ureteral cancer, renal cell carcinoma, renal pelvic carcinoma, central nervous system tumor, primary central nervous system lymphoma, spinal cord tumor, brain stem glioma and pituitary adenoma.

16. A method for treatment of cancer, including: administering the human antibody Fc domain variant according to any one of the above 1 to 10, an antibody including the same or a fragment thereof having immunological activity; or the antibody-drug conjugate according to the above 12 or 13, to a subject.

17. The method according to the above 16, wherein the cancer is selected from the group consisting of: brain tumor, melanoma, myeloma, non-small cell lung cancer, oral cancer, liver cancer, stomach cancer, colon cancer, breast cancer, lung cancer, bone cancer, pancreatic cancer, skin cancer, head or neck cancer, cervical cancer, ovarian cancer, colon cancer, small intestine cancer, rectal cancer, fallopian tube carcinoma, anal cancer, endometrial carcinoma, vaginal carcinoma, vulvar carcinoma, Hodgkin's disease, esophageal cancer, lymph node cancer, bladder cancer, gallbladder cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, renal or ureteral cancer, renal cell carcinoma, renal pelvic carcinoma, central nervous system tumor, primary central nervous system lymphoma, spinal cord tumor, brain stem glioma and pituitary adenoma.

18. A composition for target detection, including a conjugate in which the antibody according to the above 11 or a fragment thereof having immunological activity and a functional substance are bound.

19. The composition for target detection according to the above 18, wherein the functional material is one or more selected from the group consisting of contrast agents, radioisotopes, inorganic particles, fluorescent markers, dyes, enzymes, polypeptides, nucleic acids, carbohydrates and lipids.

20. A method for diagnosing a disease, including administering the composition for target detection according to the above 18 to an individual.

21. The method according to the above 20, wherein the disease is any one selected from the group consisting of cancer, cardiovascular disease, neurological disorder, diabetes, autoimmune disease, inflammatory disease, viral infectious disease and allergy.

[Advantageous effects]

The human antibody Fc domain variant of the present invention does not bind or does not substantially bind to human FcyRs and C1q.

The human antibody Fc domain variant of the present invention does not exhibit effector functions such as antibody-dependent cellular cytotoxicity (ADCC) and antibody-dependent cellular phagocytosis (ADCP).

The human antibody Fc domain variant of the present invention possesses pH-dependent FcRn binding ability and thermal stability.

The human antibody Fc domain variant of the present invention may reduce drug toxicity and increase efficacy.

The human antibody Fc domain variant of the present invention may maintain the half-life of the drug and remove target toxicity.

[Brief Description of Drawings]

FIGS. 1A and 1B are schematic diagrams of mammalian cell display technology of glycosylated Fc.

FIGS. 2A to 2E show results of SDS-PAGE analysis after expression and purification of highly purified tetrameric FcγRI-streptavidin and tetrameric FcγRIIIa-158V-streptavidin, FcγRI-GST, FcγRIIa-131H-GST, FcγRIIa-131R-GST, FcγRIIb-GST, FcγRIIIa-158V-GST, FcγRIIIa-158F-GST and FcRn-GST.

FIGS. 3A and 3B show results of analyzing the binding activity of fluorescently labeled tetrameric FcγRIIIa-Alexa647 and Protein A-FITC with wild type Fc (WT Fc) expressed in CHO cells.

FIG. 4 shows results of comparing amino acids by aligning the goat IgG Fc sequence and the human IgG Fc sequence.

FIGS. 5A and 5B show results of SDS-PAGE analysis of the prepared glycosylated Fc variants after expression and purification, and the mutation sequences of each glycosylated Fc variant.

FIGS. 6A and 6B show results of ELISA assay for the binding ability, i.e., binding affinity of the prepared glycosylated Fc variants to FcγRI and FcγRIIIa-158V.

FIG. 7 is a schematic diagram illustrating a site-directed mutation library for glycosylated Fc engineering.

FIG. 8 is a schematic diagram of glycosylated Fc engineering using CHO cell display.

FIG. 9 shows results of SDS-PAGE analysis after expressing and purifying the selected glycosylated Fc variants (LP1, LP5, LP6, LP7 and LP8).

FIGS. 10A to 10C show results of ELISA assay for the binding ability of glycosylated trastuzumab Fc variants introduced with the selected glycosylated Fc variants (LP1, LP5, LP7 and LP8), respectively, to FcγRI, FcγRIIa-131H, FcγRIIa-131R, FcγRIIb, FcγRIIIa-158V and FcγRIIIa-158F.

FIG. 11 shows results of SDS-PAGE analysis for a glycosylated Fc variant (LALP) with reduced binding ability to FcγRs after expression and purification, and its mutant sequence.

FIGS. 12A to 12C show results of ELISA assay for the binding ability of the glycosylated Fc variant (LALP) with reduced binding ability to FcγRs, to FcγRI, FcγRIIa-131H, FcγRIIa-131R, FcγRIIb, FcγRIIIa-158V and FcγRIIIa-158F.

FIG. 13 shows results of SDS-PAGE analysis after expression and purification of the selected glycosylated Fc variants (LALP1, LALP5, LALP6, LALP7 and LALP8), as well as their mutant sequences.

FIGS. 14A to 14C show results of ELISA assay for the binding ability of glycosylated trastuzumab Fc variants introduced with the selected glycosylated Fc variants (LALP1, LALP5, LALP6, LALP7 and LALP8), respectively, to FcγRI, FcγRIIa-131H, FcγRIIa-131R, FcγRIIb, FcγRIIIa-158V and FcγRIIIa-158F.

FIG. 15 shows results of DSF analysis for thermal stability of the discovered glycosylated trastuzumab Fc variants (LALP, LALP1, LALP5, LALP6, LALP7 and LALP8).

FIG. 16 shows results of DSF analysis for thermal stability of the discovered glycosylated trastuzumab Fc variants (LP1, LP5, LP6, LP7 and LP8).

FIG. 17 shows results of ELISA assay for FcRn binding ability of the discovered glycosylated trastuzumab Fc variants (LALP, LALP1, LALP5, LALP6 and LALP7).

FIGS. 18A and 18B shows results of ELISA assay for FcRn binding ability of the discovered glycosylated trastuzumab Fc variants (LP1, LP5 and LP6).

FIG. 19 shows results of ELIS assay for C1q binding ability of the discovered glycosylated trastuzumab Fc variants (LALP, LALP1, LALP5, LALP6 and LALP7).

FIG. 20 shows results of ELISA assay for C1q binding ability of the discovered glycosylated trastuzumab Fc variants (LP1, LP5 and LP6).

FIG. 21A shows results of SDS-PAGE analysis after expression and purification of the conventional glycosylated trastuzumab Fc variant (LALAPG), as well as its mutant sequence.

FIG. 21B shows results of comparative analysis for HER2-expressing target cell-based FcγRI binding ability of the discovered glycosylated trastuzumab Fc variants (LP1, LP5, LP6 and LP7) with LALA.

FIG. 22 shows results of analyzing the binding ability of each of the conventional glycosylated trastuzumab Fc variant (LALAPG) and the discovered glycosylated trastuzumab Fc variants (LALP1, LALP5 and LALP6) to hFcγRI.

FIG. 23 shows results of analyzing the binding ability of each of the conventional glycosylated trastuzumab Fc variant (LALAPG) and the discovered glycosylated trastuzumab Fc variants (LALP1, LALP5 and LALP6) to hFcγRIIIa-158F.

Figure 24 shows results of SDS-PAGE analysis after expression and purification of the glycosylated trastuzumab Fc variant (LALPPG) prepared by combining P329G and the mutation discovered in the present invention, as well as its mutant sequence.

FIGS. 25A to 25C show results of ELISA assay for binding ability of the glycosylated trastuzumab Fc variants (LALP and LALPPG) discovered in the present invention to FcγRI, FcγRIIa-131H, FcγRIIa-131R, FcγRIIb, FcγRIIIa-158V and FcγRIIIa-158F, respectively.

FIG. 26 shows results of analyzing reduced PBMC-mediated ADCC function of the glycosylated trastuzumab Fc variants (LALP1, LALP5 and LALP6) discovered in the present invention using real-time cell death analysis.

FIGS. 27A and 27D show results of analyzing reduced PBMC-mediated ADCC function of the glycosylated trastuzumab Fc variants (LALP1, LALP5 and LALP6) discovered in the present invention, which were analyzed by counting the number of dead cells through real-time cell imaging.

FIG. 28 shows an analysis of the reduced NK92-mediated ADCC function of the glycosylated rituximab Fc variants (LALP1, LALP5 and LALP6) discovered in the present invention.

FIG. 29 shows results of analyzing the reduced ADCP function of the glycosylated trastuzumab Fc variants (LALP1, LALP5 and LALP6) discovered in the present invention.

FIG. 30 shows results of analyzing the reduced CDC function of the glycosylated rituximab Fc variants (LALP1, LALP5 and LALP6) discovered in the present invention.

FIGS. 31A and 31B shows results of ELISA assay for FcRn binding ability of the glycosylated trastuzumab Fc variants (LALP1, LALP5 and LALP6) discovered in the present invention in mouse, as well as *in vivo* PK analysis result.

FIGS. 32A and 32B show results of ELISA assay for binding ability of the glycosylated trastuzumab Fc variants (LALP1, LALP5 and LALP6) discovered in the present invention to mFcγRI, mFcγRIIb, mFcγRIII and mFcγRIV in mice, respectively.

FIGS. 33A and 33B show results of ELISA assay for binding ability of the glycosylated trastuzumab Fc variants (LALP1, LALP5 and LALP6) discovered in the present invention to cFcγRI, cFcγRIIa, cFcγRIIb and cFcγRIII in cynomolgus monkeys, respectively.

[Mode for Carrying out Invention]

The present invention provides a human antibody Fc domain variant and a use thereof.

In the present invention, there are provided human antibody Fc domain variants that do not exhibit antibody-dependent cellular cytotoxicity (ADCC) and antibody-dependent cellular phagocytosis (ADCP) because amino acid 235 of a wild-type human antibody Fc domain is substituted with proline (P), resulting in no or substantially no binding ability to human FcγRs and C1q, as well as uses of the variants as antibody-drug conjugates.

In the present invention, the usual one- and three-letter codes for natural amino acids as well as the generally acceptable three-letter codes for other amino acids such as α-aminoisobutyric acid (Aib), N-methylglycine (Sar), etc. are used.

Amino acids abbreviated in the present invention are described as follows according to the IUPAC-IUB nomenclature: alanine: A, arginine: R, asparagine: N, aspartic acid: D, cysteine: C, glutamic acid: E, glutamine: Q, glycine: G, histidine: H, isoleucine: I, leucine: L, lysine: K, methionine: M, phenylalanine: F, proline: P, serine: S, threonine: T, tryptophan: W, tyrosine: Y and valine: V.

In the present invention, the position of a specific amino acid in the amino acid sequence refers to the position numbered according to the Kabat numbering system.

As used herein, the term "amino acid modification/variation" refers to substitution, insertion and/or deletion, preferably substitution of amino acids in a polypeptide sequence. The term "amino acid substitution" or "substitution" as used herein means that an amino acid at a specific position in the polypeptide sequence of the wild-type human antibody Fc domain is replaced with another amino acid. For example, a human antibody Fc domain variant including the L235P substitution means that leucine (L), the 235th amino acid residue in the amino acid sequence of the wild-type human antibody Fc domain, is replaced with proline (P).

In the human antibody Fc domain variant of the present invention, amino acid 235 (L) of the wild-type human antibody Fc domain may be substituted with proline (P).

In one embodiment, the human antibody Fc domain variant of the present invention may include one or more amino acid substitutions selected from the group consisting of L234A, D265N, D265M, D265L, P329V, P329L, P329G and P331L in addition to L235P.

In one embodiment, the human antibody Fc domain variant of the present invention may include amino acid substitutions of L235P and L234A of the wild-type human antibody Fc domain.

In one embodiment, the human antibody Fc domain variant of the present invention may include amino acid substitutions of D265N, D265M or D265L, in addition to L235P and L234A of the wild-type human antibody Fc domain.

In one embodiment, the human antibody Fc domain variant of the present invention may include amino acid substitutions of P329V, P329L or P329G, in addition to L235P and L234A of the wild-type human antibody Fc domain.

According to an embodiment, in the human antibody Fc domain variant of the present invention, amino acid 268 of the wild-type human antibody Fc domain may be further substituted with glutamine (Q), in addition to L235P and L234A thereof.

According to an embodiment, in the human antibody Fc domain variant of the present invention, amino acid 296 of the wild-type human antibody Fc domain may be further substituted with phenylalanine (F), in addition to L235P and L234A thereof.

According to an embodiment, in the human antibody Fc domain variant of the present invention, amino acid 300 of the wild-type human antibody Fc domain may be further substituted with phenylalanine (F), in addition to L235P and L234A thereof.

According to an embodiment, in the human antibody Fc domain variant of the present invention, amino acid 333 of the wild-type human antibody Fc domain may be further substituted with valine (V), in addition to L235P and L234A thereof.

In one embodiment, the human antibody Fc domain variant of the present invention may be a human antibody Fc domain variant LALP including amino acid substitutions of L234A and L235P. The human antibody Fc domain variant LALP may include the amino acid sequence of SEQ ID NO: 1, and may be encoded with a nucleic acid molecule including the nucleotide sequence of SEQ ID NO: 2.

In one embodiment, the human antibody Fc domain variant of the present invention may be the human antibody Fc domain variant LALP1 including amino acid substitutions of L234A, L235P and D265N. The human antibody Fc domain variant LALP1 may include the amino acid sequence of SEQ ID NO: 3, and may be encoded with a nucleic acid molecule including the nucleotide sequence of SEQ ID NO: 4.

In one embodiment, the human antibody Fc domain variant of the present invention may be a human antibody Fc domain variant LALP5 including amino acid substitutions of L234A, L235P and P329V. The human antibody Fc domain variant LALP5 may include the amino acid sequence of SEQ ID NO: 5, and may be encoded with a nucleic acid molecule including the nucleotide sequence of SEQ ID NO: 6.

In one embodiment, the human antibody Fc domain variant of the present invention may be a human antibody Fc domain variant LALP6 including amino acid substitutions of L234A, L235P, and P329L. The human antibody Fc domain variant LALP6 may include the amino acid sequence of SEQ ID NO: 7, and may be encoded with a nucleic acid molecule including the nucleotide sequence of SEQ ID NO: 8.

In one embodiment, the human antibody Fc domain variant of the present invention may be a human antibody Fc domain variant LALP7 including amino acid substitutions of L234A, L235P and D265M. The human antibody Fc domain variant LALP7 may include the amino acid sequence of SEQ ID NO: 9, and may be encoded with a nucleic acid molecule including the nucleotide sequence of SEQ ID NO: 10.

In one embodiment, the human antibody Fc domain variant of the present invention may be a human antibody Fc domain variant LALP8 including amino acid substitutions of L234A, L235P, D265L and P331L. The human antibody Fc domain variant LALP8 may include the amino acid sequence of SEQ ID NO: 11, and may be encoded with a nucleic acid molecule including the nucleotide sequence of SEQ ID NO: 12.

In one embodiment, the human antibody Fc domain variant of the present invention may be a human antibody Fc domain variant LALPPG including amino acid substitutions of L234A, L235P and P329G. The human antibody Fc domain variant LALPPG may include the amino acid sequence of SEQ ID NO: 13, and may be encoded with a nucleic acid molecule including the nucleotide sequence of SEQ ID NO: 14.

In one embodiment, the human antibody (immunoglobulin) may be IgA, IgM, IgE, IgD or IgG, or variants thereof. The human antibody may be IgG1, IgG2, IgG3 or IgG4, preferably IgG1, more preferably anti-HER2 antibody, and most preferably trastuzumab. Papain digestion of antibodies forms two Fab domains and one Fc domain; in human IgG molecules, the Fc region is generated by papain digestion of the N-terminus at Cys 226.

In one embodiment, the human antibody (immunoglobulin) may be IgG1 or a modification thereof, and the Fc domain of wild-type IgG1 may include the amino acid sequence of SEQ ID NO: 15, and may be encoded with a nucleic acid molecule including the nucleotide sequence of SEQ ID NO: 16.

In one embodiment, the human antibody Fc domain variant of the present invention may have reduced binding ability to Fc gamma receptors (FcyRs) compared to the wild-type human antibody Fc domain. Here, the Fc gamma receptor may be FcyRI, FcyRIIa, FcyRIIb or FcγRIIIa.

In one embodiment, the human antibody Fc domain variant of the present invention may have reduced binding ability to C1q, compared to the wild-type human antibody Fc domain.

In one embodiment, the human antibody Fc domain variant of the present invention may have reduced effector function, compared to the wild-type human antibody Fc domain.

In one embodiment, the effector functions may be Fc-mediated effector function selected from C1q-binding, complement activation, complement dependent cytotoxicity (CDC), antibody-dependent cellular cytotoxicity (ADCC), Fc-receptor binding including Fc-gamma receptor binding, protein A-binding, protein G-binding, antibody-dependent cellular phagocytosis (ADCP), complement dependent cellular cytotoxicity (CDCC), complement-enhanced cytotoxicity, opsonization, Fc-containing polypeptide internalization, target downregulation, ADC uptake, induction of apoptosis, cell death, cell cycle arrest, and any combination thereof.

In one embodiment, the human antibody Fc domain variant of the present invention has a pH-dependent FcRn binding affinity similar to that of the wild-type human antibody Fc domain, whereby it may have an *in vivo* half-life and thermal stability similar to those of the wild-type human antibody Fc domain.

In one embodiment, the human antibody Fc domain variants of the present invention may be used for the purpose of not killing cells to which they bind.

In one embodiment, the human antibody Fc domain variants of the present invention may be used as an immune checkpoint inhibitor antibody or an immune cell-inducing bispecific antibody (or bispecific immune cell engaging bispecific antibody, abbrev. to "bispecific antibody").

The human antibody Fc domain variants of the present invention may be prepared by any method known in the art. In one embodiment, the human antibody Fc domain variant according to the invention encodes a polypeptide sequence including specific amino acid modification and then, if desired, is used to form nucleic acids that are cloned into host cells, expressed and assayed. Various methods therefor have been described in Molecular Cloning - A Laboratory Manual, 3rd Ed., Maniatis, Cold Spring Harbor Laboratory Press, New York, 2001; Current Protocols in Molecular Biology, John Wiley & Sons.

Nucleic acids encoding Fc domain variants according to the invention may be inserted into expression vectors for protein expression. Expression vectors typically include proteins operably linked, i.e., placed in a functional relationship, with control or regulatory sequences, selectable markers, optional fusion partners, and/or additional elements. An Fc variant according to the present invention may be produced by a method of culturing a host cell transformed with a nucleic acid under appropriate conditions, preferably an expression vector containing a nucleic acid encoding the Fc domain variant according to the present invention, to induce protein expression. A variety of suitable host cells including mammalian cells, bacteria, insect cells and yeast may be used, but they are not limited thereto. Methods for introducing exogenous nucleic acids into host cells are known in the art and may vary depending on the host cells used. Preferably, the Fc domain variant according to the present invention may be produced using *E. coli,* which has high industrial value due to its low production costs, as the host cell.

The present invention provides a method for production of Fc domain variants, including the steps of: culturing a host cell into which a nucleic acid encoding an Fc domain variant is introduced under conditions suitable for protein expression; and purifying or isolating the Fc domain variant expressed from the host cell.

"FcRn" or "neonatal Fc receptor" refers to a protein that binds to the Fc region of an IgG antibody, which is encoded at least partially by the FcRn gene. FcRn may be derived from any organism including human, mouse, rat, rabbit and monkey, but it is not limited thereto.

As is known in the art, a functional FcRn protein includes two polypeptides, often referred to as light and heavy chains. The light chain is beta-2-microglobulin, and the heavy chain is encoded by the FcRn gene. Unless otherwise stated herein, FcRn or an FcRn protein refers to a complex of the FcRn heavy chain and beta-2-microglobulin.

The present invention provides an antibody including a human antibody Fc domain variant or a fragment thereof having immunological activity.

In one embodiment, an antibody or an immunologically active fragment thereof may loss its binding ability to Fc gamma receptors (FcyRs), thereby reducing its binding ability to FcyRs or C1q compared to the wild-type human antibody.

In one embodiment, the Fc gamma receptor may be FcyRI, FcyRIIa, FcyRIIb or FcγRIIIa.

In one embodiment, an antibody or an immunologically active fragment thereof may have reduced effector function compared to the wild-type human antibody.

In one embodiment, the antibody may be a polyclonal antibody, monoclonal antibody, minibody, domain antibody, bispecific antibody, antibody mimetic, chimeric antibody or antibody conjugate.

In one embodiment, human antibodies include humanized antibodies.

Fragments having immunological activity may include antibody Fab, Fd, Fab', dAb, F(ab'), F(ab')2, single chain fragment variable (scFv), Fv, single chain antibody, Fv dimer, and complementarity determining region or diabody.

Antibodies may be isolated or purified by various methods known in the art. Standard purification methods include chromatographic techniques, electrophoresis, immunology, precipitation, dialysis, filtration, concentration and chromatofocusing techniques. As is known in the art, a variety of natural proteins, such as bacterial proteins A, G and L, may bind to antibodies, and may be used for purification. Often, purification by specific fusion partners may be possible.

Antibodies include functional fragments of antibody molecules as well as whole antibody forms. A whole antibody has a structure of two full-length light chains and two full-length heavy chains, wherein each light chain is connected to the heavy chain by a disulfide bond. A functional fragment of an antibody molecule refers to a fragment that possesses an antigen-binding function. Examples of antibody fragments include: (i) Fab fragment consisting of a variable region (VL) of the light chain, a variable region (VH) of the heavy chain, a constant region (CL) of the light chain, and a first constant region (CH1) of the heavy chain; (ii) Fd fragment consisting of VH and CH1 domains; (iii) Fv fragment consisting of VL and VH domains of a single antibody; (iv) dAb fragment consisting of VH domain; (v) an isolated CDR region; (vi) F(ab')2 fragment which is a bivalent fragment including two linked Fab fragments; (vii) a single chain Fv molecule (scFv) in which the VH domain and the VL domain are bound by a peptide linker to form an antigen binding site; (viii) bispecific single-chain Fv dimer (PCT/US92/09965); and (ix) diabody (WO94/13804), which is a multivalent or multispecific fragment prepared by gene fusion.

The antibody or immunologically active fragment thereof of the present invention may be selected from the group consisting of animal-derived antibodies, chimeric antibodies, humanized antibodies, human antibodies, and immunologically active fragments thereof. Antibodies may be recombinantly or synthetically produced.

Antibodies or fragments thereof having immunological activity may be isolated from the living body (not present in the living body) or non-naturally occurring, for example, may be produced synthetically or recombinantly.

The "antibody" refers to a substance produced by stimulation of an antigen within the immune system, the type of which is not particularly limited, and may be obtained naturally or unnaturally (e.g., synthetically or recombinantly). The antibodies are very stable not only *in vitro* but also *in vivo,* and have a long half-life, making them advantageous for mass expression and production. Further, an antibody inherently has a dimeric structure and thus very high avidity. A complete antibody has a structure of two full-length light chains and two full-length heavy chains, wherein each light chain is connected to the heavy chain by a disulfide bond. The constant region of an antibody is divided into a heavy chain constant region and a light chain constant region, wherein the heavy chain constant region has gamma ($\gamma$), mu ($\mu$), alpha ($\alpha$), delta ($\delta$) and epsilon ($\varepsilon$) types, and is divided into subclasses such as gamma 1 ($\gamma$1), gamma 2 (y2), gamma 3 (y3), gamma 4 ($\gamma$4), alpha 1 ($\alpha$1) and alpha 2 ($\alpha$2). The constant region of the light chain has kappa ($\kappa$) and lambda ($\lambda$) types.

The "heavy chain" is interpreted to include both of: a full-length heavy chain, in which a variable region domain VH including an amino acid sequence having sufficient variable region sequence to impart specificity to an antigen, three constant region domains CH1, CH2 and CH3, and a hinge are included; and fragments thereof. Further, the "light chain" is interpreted to include both of: a full-length light chain, in which a variable region domain VL including an amino acid sequence having sufficient variable region sequence to impart specificity to an antigen and a constant region domain CL are included; and fragments thereof.

The "Fc domain," "Fc fragment" or "Fc region" forms an antibody together with the Fab domain/fragment, and the Fab domain/fragment consists of a variable region VL of the light chain and a variable region VH of the heavy chain, a constant region CL of the light chain, and a first constant region CH1 of the heavy chain while the Fc domain/fragment consists of a second constant region CH2 and a third constant region CH3 of the heavy chain.

In one aspect, the present invention relates to a nucleic acid molecule which encodes an Fc domain variant of the present invention, an antibody including the same, or a fragment thereof having immunological activity.

In one aspect, the present invention relates to a vector including a nucleic acid molecule and a host cell including the vector.

Nucleic acid molecules of the present invention may be isolated or recombined, and may include DNA and RNA in the form of single and double chains, as well as corresponding complementary sequences. In the case of a nucleic acid isolated from a naturally occurring source, the isolated nucleic acid is a nucleic acid which is isolated from the surrounding genetic sequence present in the genome of an individual from which the nucleic acid is isolated. In the case of nucleic acids synthesized enzymatically or chemically from a template, such as PCR products, cDNA molecules or oligonucleotides, the nucleic acids resulting from these procedures may be understood as isolated nucleic acid molecules. The isolated nucleic acid molecules refer to nucleic acid molecules either in the form of separate fragments or as components of larger nucleic acid constructs. A nucleic acid is operably linked when placed in a functional relationship with another nucleic acid sequence. For example, a DNA of the pre-sequence or secretion leader is operably linked to the DNA of a polypeptide when the polypeptide is expressed as a form of preprotein before secretion, and a promoter or enhancer is operably linked to a coding sequence when the promoter or enhancer affects transcription, or a ribosome binding site is operably linked to the coding sequence when it is arranged to facilitate translation. In general, the "operably linked" means that the DNA sequences to be linked are located adjacent to each other, and in the case of the secretory leaders, it means that they are adjacent and exist within the same reading frame. However, the enhancers do not need to be located adjacent to each other. Linking is accomplished by ligation at convenient restriction enzyme sites. If such sites do not exist, synthetic oligonucleotide adapters or linkers are used according to conventional methods.

In regard to the isolated nucleic acid molecule encoding the Fc domain variant of the present invention, an antibody including the same or a fragment thereof having immunological activity, due to codon degeneracy or in consideration of preferred codon in the organism to be expressed, various modifications may be made to the coding region within the range that does not change the amino acid sequence of the Fc domain variant expressed from the coding region, the antibody including the same or a fragment thereof having immunological activity. Further, those skilled in the art will understand that, even in the portion excluding the coding region, various alternations or modifications may be made within the range that does not affect the expression of the gene, and that such modified genes are also included within the scope of the present invention. That is, as long as the nucleic acid molecule of the present invention encodes a protein having equivalent activity, one or more nucleic acid bases may be mutated by substitution, deletion, insertion or a combination thereof, and these are also included within the scope of the present invention. The sequence of such nucleic acid molecule may be single or double chain, and may be DNA molecules or RNA (mRNA) molecules.

An isolated nucleic acid molecule encoding an Fc domain variant of the present invention, an antibody including the same or a fragment thereof having immunological activity according to the present invention may be inserted into an expression vector for protein expression. The expression vectors typically include proteins operably linked, i.e., placed in a functional relationship, with control or regulatory sequences, selectable markers, optional fusion partners, and/or additional elements. Under appropriate conditions, by a method of culturing a host cell transformed with a nucleic acid, preferably, an expression vector which includes an isolated nucleic acid molecule encoding the Fc domain variant of the present invention, an antibody including the same or a fragment thereof having immunological activity thus to induce protein expression, it is possible to produce the Fc domain variant of the present invention, an antibody including the same or a fragment thereof having immunological activity. A variety of suitable host cells including mammalian cells, bacteria, insect cells and yeast, but they are not limited thereto. Methods for introducing exogenous nucleic acids into the host cells are known in the art, and will vary depending on the host cells used. Preferably, E. *coli,* which has high industrial value due to its low production costs, may be used to produce the Fc domain variant as the host cell.

Vectors of the present invention include plasmid vectors, cosmid vectors, bacteriophage vectors and viral vectors, etc., but they are not limited thereto. Suitable vectors include expression control elements such as promoters, operators, start codons, stop codons, polyadenylation signals and enhancers, as well as signal sequences or leader sequences for membrane targeting or secretion, and may be prepared in various ways according to the purposes. The promoter of the vector may be constitutive or inducible. The signal sequences may use: PhoA signal sequence, OmpA signal sequence, etc. When the host is *Escherichia* sp; $\alpha$-amylase signal sequence, subtilisin signal sequence, etc. When the host is *Bacillus* sp.; MF$\alpha$ signal sequence, SUC2 signal sequence, etc. When the host is yeast; and an insulin signal sequence, $\alpha$-interferon signal sequence, antibody molecule signal sequence, etc. When the host is an animal cell, but they are not limited thereto. In addition, the vector may include a selection marker for selecting host cells containing the vector, and if it is a replicable expression vector, it will include an origin of replication.

In the present invention, the "vector" refers to a transporter capable of inserting a nucleic acid sequence for introduction into a cell capable of replicating the nucleic acid sequence. The nucleic acid sequences may be exogenous or heterologous. The vectors may include plasmids, cosmids, and viruses (e.g., bacteriophages), but they are not limited thereto. Those skilled in the art may construct vectors by standard recombination techniques.

In one embodiment, when constructing the vector, expression regulatory sequences such as a promoter, terminator, enhancer, etc., sequences for membrane targeting or secretion, or the like may be appropriately selected depending on the type of the host cells to produce the Fc domain variant, an antibody including the same or a fragment thereof having immunological activity, and may be combined in various ways according to the purposes.

The "expression vector" means a vector including a nucleic acid sequence that encodes at least a portion of the gene product to be transcribed. In some cases, the RNA molecule is then translated into a protein, polypeptide, or peptide. The expression vectors may include various regulatory sequences. In addition to the regulatory sequences that regulate transcription and translation, vectors and expression vectors may also include nucleic acid sequences that also provide other functions.

The "host cell" includes eukaryotes and prokaryotes, and means any transformable organism capable of replicating the vector or expressing the gene encoded by the vector. The host cell may be transfected or transformed by the vector, which refers to a process in which an exogenous nucleic acid molecule is transferred or introduced into the host cell.

In one embodiment, the host cells may be bacteria or animal cells, wherein the animal cell line may be CHO cells, HEK cells or NSO cells, and the bacteria may be *Escherichia coli.*

In one aspect, the present invention relates to a fusion protein in which the Fc domain variant of the present invention, an antibody including the same or a fragment thereof having immunological activity is linked to a cargo molecule.

In one embodiment, the cargo molecule may be a detection agent, therapeutic agent, drug, peptide, growth factor, cytokine, receptor trap, chemical compound, carbohydrate moiety, enzyme, antibody or fragment thereof, DNA-based molecule, viral vector, or cytotoxic agent; one or more liposomes or nanocarriers loaded with a detection agent, therapeutic agent, drug, peptide, enzyme, antibody or fragment thereof, DNA-based molecule, viral vector or cytotoxic agent; or one or more nanoparticles, nanowires, nanotubes or quantum dots.

In one embodiment, the fusion protein may be an agonist antibody, an antagonist antibody or a therapeutic antibody.

The present invention provides an antibody-drug conjugate in which the antibody of the present invention or a fragment thereof having immunological activity is conjugated to one or more drugs.

In one embodiment, the drug may be an immune checkpoint inhibitor or a bispecific immune cell engager, and may have reduced effector function.

In one embodiment, the drug may be chimeric antigen receptor (CAR) cell therapeutics, oncolytic drug, immunotherapy agent, cytotoxic agent, angiogenesis inhibitor, kinase inhibitor, costimulatory molecule blocker, adhesion molecule blocker, anti-cytokine agent, nucleic acid therapeutics, anti-CTLA-4 agent, anti-PD-1 agent, anti-PD-L1 agent, anti-PD-L2 agent, TNF-$\alpha$ cross-linking agent, TRAIL cross-linking agent, anti-CD27 agent, anti-CD30 agent, anti-CD40 agent, anti-4-1BB agent, anti-GITR agent, anti-OX40 agent, anti-TRAILR1 agent, anti-TRAILR2 agent, tagretin, interferon-alpha, clobetasol, peg interferon, prednisone, romidepsin, bexarotene, methotrexate, triamcinolone cream, anti-chemokine, vorinostat, gabapentin, cyclosporine, rapamycin, FK506, detectable marker or reporter, TNF antagonist, antirheumatic agent, muscle relaxants, narcotics, non-steroidal anti-inflammatory drugs (NSAIDs), analgesics, anesthetics, sedatives, local anesthetics, neuromuscular blocker, antibacterial agent, psoriasis therapeutics, corticosteroid, anabolic steroid, erythropoietin, immunizations, immunoglobulin, immunosuppressant, growth hormone, hormone replacement drug, radiopharmaceuticals, antidepressant, antipsychotics, stimulant, asthma drug, beta agonist, inhaled steroid, epinephrine or its analogues, cytokine, cytokine antagonist, PD-1 antagonist, adenosine A2AR antagonist, CD73 inhibitor, CTLA-4 inhibitor, TIM-3 inhibitor, LAG-3 inhibitor, anthracycline, antisense oligonucleotide (ASO), siRNA, aptamer, or a combination thereof.

In one aspect, the present invention provides a pharmaceutical composition for prevention or treatment of cancer, which includes a human antibody Fc domain variant, an antibody including the same, or a fragment thereof having immunological activity, or an antibody therapeutic agent including the same as an active ingredient.

In one embodiment, the cancer may be any one selected from the group consisting of: brain tumor, melanoma, myeloma, non-small cell lung cancer, oral cancer, liver cancer, stomach cancer, colon cancer, breast cancer, lung cancer, bone cancer, pancreatic cancer, skin cancer, head or neck cancer, cervical cancer, ovarian cancer, colon cancer, small intestine. Cancer, rectal cancer, fallopian tube carcinoma, anal cancer, endometrial carcinoma, vaginal carcinoma, vulvar carcinoma, Hodgkin's disease, esophageal cancer, lymph node cancer, bladder cancer, gallbladder cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, renal or ureteral cancer, renal cell carcinoma, renal pelvic carcinoma, central nervous system tumor, primary central nervous system lymphoma, spinal cord tumor, brainstem glioma, and pituitary gland adenoma.

In one embodiment, the composition of the present invention may further include an immunogenic apoptosis inducing agent, wherein the immunogenic apoptosis inducing agent may be one or more selected from the group consisting of an anthracycline-based anticancer agent, taxane-based anticancer agent, anti-EGFR antibody, BK channel agonist, bortezomib, cardiac glycoside, cyclophosmide anticancer agent, GADD34/PP1 inhibitor, LV-tSMAC, Measles virus, bleomycin, mitoxantrone or oxaliplatin; and wherein the anthracycline-based anticancer agent may be daunorubicin, doxorubicin, epirubicin, idarubicin, pixantrone, sabarubicin or valrubicine, and the taxane-based anticancer agent may be paclitaxel or docetaxel.

The pharmaceutical composition for treatment or prevention of cancer according to the present invention may increase cancer treatment effects of conventional anticancer agents based on cancer cell killing effects by administering the composition together with chemical anticancer drugs (anticancer agents). Such administration in combination may be done simultaneously or sequentially with the anticancer agents. Examples of the anticancer agents may include: DNA alkylating agents such as mechloethamine, chlorambucil, phenylalanine, mustard, cyclophosphamide, ifosfamide, carmustine (BCNU), lomustine (CCNU), streptozotocin, busulfan, thiotepa, cisplatin and carboplatin; anti-cancer antibiotics such as dactinomycin (actinomycin D), plicamycin and mitomycin C; and plant alkaloids such as vincristine, vinblastine, etoposide, teniposide, topotecan and iridotecan, but they are not limited thereto.

The "prevention" refers to all actions that inhibit or delay the occurrence, spread and recurrence of cancer by administering the pharmaceutical composition according to the present invention.

The "treatment" refers to all actions that kill cancer cells or improve or beneficially change symptoms of cancer by administering the composition of the present invention. Anyone with ordinary knowledge in the technical field to which the present invention pertains ("those skilled in the art") would know the exact criteria for diseases for which the composition of the present invention is effective, and determine the extent of improvement, enhancement and treatment thereof with reference to data presented by the Korean Medical Association.

The term "therapeutically effective amount" as used herein in combination with an active ingredient refers to an amount of a pharmaceutically acceptable salt of the composition effective in preventing or treating the target disease, and the therapeutically effective amount of the composition of the present invention may vary depending on several factors, for example, administration method, target site, and condition of a patient. Therefore, when used in the human body, the administration amount or dosage should be determined as a desirable amount by considering both safety and efficiency. It is also possible to estimate an amount used in humans from the effective amount determined through animal experiments. These considerations in determining the effective amount have been described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present invention is administered in a pharmaceutically effective amount. As used herein, the term "pharmaceutically effective amount" refers to an amount which is sufficient to treat a disease with a reasonable benefit/risk ratio applicable to medical treatment and does not cause side effects, and the effective dose level may be determined according to factors including health status of the patent, type and severity of cancer, activity of drug, sensitivity to the drug, administration method, administration time, administration route and excretion rate, treatment duration, drugs combined or used simultaneously, and other factors well known in the field of medicine. The composition of the present invention may be administered as an individual therapeutic agent or in combination with other therapeutic agents, may be administered sequentially or simultaneously with conventional therapeutic agents, and may be administered singly or multiple times. Considering all of the above factors, it is important to administer an amount that can achieve maximum effects with the minimum amount without side effects, and this may be easily determined by those skilled in the art.

The pharmaceutical composition of the present invention may further include pharmaceutically acceptable additives. In this case, as the pharmaceutically acceptable additives, starch, gelatinized starch, microcrystalline cellulose, lactose, povidone, colloidal silicon dioxide, calcium hydrogen phosphate, lactose, mannitol, taffy, gum arabic, pregelatinized starch, corn starch, powdered cellulose, hydroxypropyl cellulose, opadry, sodium starch glycolate, lead carnauba, synthetic aluminum silicate, stearic acid, magnesium stearate, aluminum stearate, calcium stearate, white sugar, dextrose, sorbitol, talc, or the like may be used. The pharmaceutically acceptable additive according to the present invention is preferably included in an amount of 0.1 to 90 parts by weight based on the composition, but it is not limited thereto.

The composition of the present invention may also include carriers, diluents, excipients or a combination of two or more thereof, which are commonly used in biological agents. The pharmaceutically acceptable carriers are not particularly limited as long as they are suitable for *in vivo* delivery of the composition, and may include, for example, the compounds described in Merck Index, 13th ed., Merck & Co. Inc., saline, sterilized water, Ringer's solution, buffered saline solution, dextrose solution, maltodextrin solution, glycerol, ethanol and one or more of these ingredients, which may be mixed and used. In addition, if necessary, other typical additives such as antioxidants, buffers, and bacteriostatic agents may be added. Further, diluents, dispersants, surfactants, binders and lubricants may be additionally added to formulate dosage forms such as aqueous solutions, suspensions, emulsions, etc., pills, capsules, granules or tablets. Furthermore, it is preferably formulated according to each disease or ingredient using an appropriate method in the art or a method disclosed in Remington's Pharmaceutical Science (Mack Publishing Company, Easton PA, 18th, 1990).

The composition of the present invention may be administered parenterally (e.g., applied intravenously, subcutaneously, intraperitoneally, or topically as an injection formulation) or orally depending on the desired method, and the dosage is determined in varied ranges by the patient's weight, age, gender, health condition, diet, administration time, administration method, excretion rate, and severity of disease. A daily dosage of the composition according to the present invention may range from 0.0001 to 10 mg/ml, preferably 0.0001 to 5 mg/ml, and more preferably administered once or several times a day.

Liquid formulations for oral administration of the composition of the present invention may include suspension, oral solution, emulsion, syrups, etc. Further, in addition to the commonly used simple diluents such as water and liquid paraffin, various excipients such as a wetting agent, sweetener, flavor, preservatives, etc. may also be included together. Formulations for parenteral administration include sterile aqueous solution, non-aqueous solvent, suspension, emulsion, lyophilized formulation, suppositories, etc.

The present invention provides a human antibody Fc domain variant; an antibody including the same or a fragment thereof having immunological activity; and an antibody-drug conjugate; alternatively, a method of treating cancer including the step of administering the above pharmaceutical composition to a subject.

In one aspect, the present invention provides a method for production of a human antibody Fc domain variant, which includes the steps of: a) culturing a host cell which includes a vector including a nucleic acid molecule encoding the human antibody Fc domain variant of the present invention; and b) recovering the polypeptide expressed by the host cell.

In one aspect, the present invention provides a method for production of an antibody with reduced effector function, which includes the steps of: a) culturing a host cell which includes a vector including a nucleic acid molecule encoding the antibody of the present invention or a fragment thereof having immunological activity; and b) purifying the antibody expressed from the host cell.

In one embodiment, purification of the antibody may include filtration, HPLC, anion exchange or cation exchange, high performance liquid chromatography (HPLC), affinity chromatography, or a combination thereof, and preferably, affinity chromatography using protein A is used.

The antibodies of the present invention or fragments thereof having immunological activity may be used for detection and imaging of targets.

The present invention provides a composition for target detection, including a conjugate in which an antibody or a fragment thereof having immunological activity is bound with a functional substance.

The targets may be antigens, antibodies, drugs, biomarkers, etc.

The condition or disease of an individual may be diagnosed through target detection and imaging.

The functional substance may be one or more selected from the group consisting of contrast agents, radioisotopes, fluorescent markers, dyes, enzymes, polypeptides, nucleic acids, carbohydrates and lipids.

The contrast agent may include lipiodol, ioxytalamic acid, iohexol, iopamidol, ioversol, iodixanol, gadobutrol, meglumine gadoterate, gadodiamide, gadobenate dimeglumine, barium sulfate, etc.

The radioisotopes may include $^{3}H$, $^{18}F$, $^{11}C$, $^{14}C$, $^{15}O$, $^{13}N$, $^{32}P$, $^{35}S$ , $^{36}Cl$, $^{51}Cr$, $^{57}Co$, $^{58}Co$, $^{59}Fe$, $^{90}Y$, $^{125}I$,$^{131}I$, $^{186}Re$, etc.

The fluorescent marker may include green fluorescent protein (GFP), yellow fluorescent protein (YFP), blue fluorescent protein (BFP), cyan fluorescent protein (CFP), etc.

The dyes may include acridine dyes, cyanine dyes, fluorine dyes, oxazine dyes, phenanthridine dyes, rhodamine dyes, etc.

The enzyme may include β-glucuronidase, β-D-glucosidase, urease, peroxidase, alkaline phosphatase, acetylcholinesterase, glycose oxidase, hexokinase, malate dehydrogenase, glucose-6- phosphodehydrogenase, invertase, etc.

In one embodiment, the presence and progression stage of an individual's disease may be diagnosed by detecting an antigen with the composition for target detection of the present invention.

In one embodiment, the therapeutic efficacy of a drug administered to an individual may be monitored by detecting an antigen with the composition for target detection of the present invention.

The composition for target detection of the present invention may be applied to various analysis methods using immune responses. The analysis method is, for example, an immunoassay or an immunodetection method. The immunoassay or immunodetection method may include radioimmunoassay (RIA), enzyme-linked immunosorbent assay (ELISA), fluorescence immunoassay (FIA), chemiluminescence immunoassay, western blotting, etc.

The composition for target detection of the present invention may be used for *in vivo* detection (imaging). *In vivo* detection (imaging) methods may include, for example, scintigraphy, positron emission tomography (PET), single photon emission tomography (SPECT), near-infrared (NIR) optical imaging, magnetic resonance imaging (MRI), etc.

Diagnosable diseases include, for example, cancer, cardiovascular disease, neurological disorder, diabetes, autoimmune disease, inflammatory disease, viral infection, allergy and the like.

The composition for target detection of the present invention may include a reagent for performing an antigen-antibody reaction or a reagent for detecting the reaction. The reagent for performing antigen-antibody reaction includes buffers, salts, etc. The reagent for detecting the reaction includes formulations commonly used in immunoassay or immunodetection methods, for example, monoclonal antibodies, fragments thereof, or a labeled secondary antibody for recognizing conjugate, and a substrate corresponding to the label.

Hereinafter, the present invention will be described in more detail through examples.

## EXAMPLE

### Example 1. Construction of mammalian cell display system for screening glycosylated Fc variants

An Fc library was prepared to discover new glycosylated Fc variants that can remove immune action mechanisms. Specifically, the FLP-FRT gene recombination system used in the production of stable cell lines was utilized to display and stably screen the glycosylated Fc domain on the surface of mammalian cells. Further, in order to display the glycosylated Fc domain on the cell membrane, a platelet-derived growth factor receptor (PDGFR) transmembrane domain was fused to C-terminus of Fc. The Fc-PDGFR gene was prepared by cloning into pcDNA5/FRT plasmid (Invitrogen, V601020) into which FRT site was inserted. The corresponding plasmid induces genetic recombination by co-transfecting CHO cells (Invitrogen, R75807) into which the FRT site is inserted with the pOG44 plasmid (Invitrogen, V600520) expressing FLP, which is an enzyme causing genetic recombination, whereby CHO cell line stably expressing the glycosylated Fc through integration of Fc-PDGFR DNA into chromosomal DNA of the CHO cell was prepared. At this time, in order to select only CHO cells in which gene integration occurred after transfection, the hygromycin-B resistance gene was integrated together, further, the glycosylated Fc stable expression CHO cell lines were selected and prepared by treatment with 500 μg/ml of hygromycin-B (Invitrogen, 10687010) (FIGS. 1A and 1B).

Example 2. FcyRs expression and purification

In order to conduct FACS screening of a cell line (stable cell line) stably expressing the glycosylated Fc library and select Fc variants, in which an antibody-mediated immune action mechanism was completely removed, using a CHO cell display system of the glycosylated Fc established in Example 1, Fc gamma receptors (FcyRs) were produced. Specifically, tetrameric FcγRIIIa-158V-streptavidin-His was prepared by fusing streptavidin to the C-terminus of each receptor so that efficient FACS screening would be possible by improving the visible binding affinity with Fc. Further, the tetrameric FcyRI-streptavidin-His, FcyRI-GST, FcγRIIa-131H-GST, FcγRIIa-131R-GST, FcyRIIb-GST, FcyRIIIa-158V-GST and FcyRIIIa-158F-GST, which are required to analyze the binding ability to FcyRs of the discovered glycosylated Fc variants by ELISA, were prepared and produced. Further, FcRn-GST, which is required to analyze the FcRn binding ability associated with the *in vivo* half-life of antibodies, was prepared and produced. In order to produce each receptor protein, each protein produced above was cloned into an animal cell expression vector, then transfected into Expi293F cells using PEI, followed by incubating for 7 days at 37 °C and 125 rpm under 8% $CO_2$ condition. After completion of the incubation, the supernatant was recovered, equilibrated with PBS, purified by Ni-NTA (Anti-His) or anti-GST affinity chromatography, and confirmed by SDS-PAGE gel. As a result, it was confirmed that high purity tetrameric FcyRI-streptavidin, tetrameric FcyRIIIa-158V-streptavidin, FcyRI-GST, FcγRIIa-131H-GST, FcγRIIa-131R-GST, FcyRIIb-GST, FcγRIIIa-158V-GST, FcyRIIIa-158F-GST and FcRn-GST were purified (FIGS. 2A to 2E).

Example 3. Labeling and validation of FcyRs

Among the produced proteins, tetrameric FcγRIIIa-streptavidin was labeled with Alexa647 (Invitrogen, A20173) fluorescent dye for FACS screening. In order to confirm whether to express or not and the expression level of each of the displayed Fc variants, Protein A (Amicogen, 1070020) whose binding sites do not overlap with FcyRs was prepared by conjugating the same with FITC (Invitrogen, F6434). At this time, conjugation of fluorescent dyes (Alexa647 and FITC) was performed according to the manual provided by each manufacturer. Through this, the fluorescence-labeled tetrameric FcγRIIIa-streptavidin-Alexa647 and Protein A-FITC were induced to bind to wild-type Fc displayed on the CHO cells, followed by confirming their activity. As a result, through fluorescence signals due to Protein A-FITC binding, it was confirmed that Protein A-FITC had normal activity and the wild-type Fc displayed on the CHO cells was stably expressed (FIG. 3A). Further, tetrameric FcyRIIIa-streptavidin-Alexa647 was also shown to normally bind to wild-type Fc (FIG. 3B), therefore, it has been verified that Alexa647 fluorescently labeled FcγRIIIa also had excellent activity, and Fcs displayed on the CHO cells were expressed normally and had their respective functions.

**Example 4. Preparation of glycosylated Fc variants with reference to goat IgG Fc sequence**

Among various animal-derived IgG antibodies, goat IgG has very low cross reactivity with human FcyRs (see S. T. Jung et al. (2010) "Aglycosylated IgG variants expressed in bacteria that selectively bind FcyRI potentiate tumor cell killing by monocyte-dendritic cells", Proceedings of the National Academy of Sciences USA (PNAS), 107:2, 604-609; S. T. Jung, et al. (2013) "Effective phagocytosis of low Her2 tumor cell lines with engineered, aglycosylated IgG displaying high FcyRIIa affinity and selectivity", ACS Chemical Biology, 8:2, 368-375; M. Jo et al. (2018) "Engineered aglycosylated full-length IgG Fc variants exhibiting improved FcγRIIIa binding and tumor cell clearance", mAbs, 2:10, 278-289; H. W. Yoon et al. (2019) "Optimal combination of beneficial mutations for improved ADCC effector function of aglycosylated antibodies," Molecular Immunology, 114, 62-71), the sequences of goat IgG Fc and human IgG Fc were compared, and mutations were introduced focusing on four regions of IgG antibody Fc (Lower hinge, B/C loop, C'/E loop and F/G loop) predicted to interact with human FcyRs, thereby preparing goat IgG Fc sequence-derived human glycosylated Fc variants (FIG. 4).

Example 5. Animal cell expression and purification of trastuzumab Fc variants including glycosylated Fc variants

The glycosylated Fc variants prepared in Example 4 were cloned into the model antibody, that is, trastuzumab heavy chain gene to prepare an expression vector, and the heavy chain gene and light chain gene of the variants were firstly mixed at a ratio of 1:1 in Freestyle 293 expression culture medium (Gibco, 12338-018), and then PEI (polyethylenimine, Polyscience, 23966) and the expression vector gene were mixed at a ratio of 4:1, followed by incubation for 20 minutes at room temperature. Then, the mixture was transfected into Expi293F animal cells cultured at a density of $2 \times 10^6$ cells/ml, and cultured for 7 days at 37°C and 125 rpm under 8% $CO_2$ condition, followed by centrifugation to recover only the supernatant. The supernatant was equilibrated with 25 × PBS and then filtered through a 0.2 μm syringe filter. Protein A resin was input into the filtered culture medium including the trastuzumab Fc variants, stirred at 4°C for 16 hours, spun down, followed by recovering the resin and washing the same with 2 ml of 1 × PBS. 300 μl of 100 mM glycine (pH 2.7) was added to the resin to elute the same, followed by neutralization using 100 μl of 1 M Tris-HCl (pH 8.0). In order to change the buffer, Amicon Ultra-4 centrifugal filter units 30K (Merck Millipore, UFC503096) were used, and highly purified glycosylated antibody trastuzumab Fc variants were ensured through SDS-PAGE analysis (FIGS. 5A and 5B).

**Example 6. Analysis of human FcγRs binding ability of glycosylated Fc variants**

ELISA assay was performed to confirm the FcyRs binding ability of the glycosylated trastuzumab Fc variants purified in Example 5 above. Specifically, 50 μl of each of FcyRs-GST (FcyRI-GST and FcγRIIIa-158V-GST) diluted with 4 μg/ml in 0.05 M $Na_2CO_3$ (pH 9.6) was input into a flat bottom polystyrene high bind 96 well microplate (Costar, 3590), and fixed at 4 °C for 16 hours. After blocking with 100 μl of 4% skim milk (GenomicBase, SKI400) at room temperature for 1 hour and washing 4 times with 180 μl of 0.05% PBST, 50 μl of each of the glycosylated trastuzumab Fc variants serially diluted with 1% skim milk was dispensed into each well and reacted at room temperature for 1 hour. After washing, 50 μl of HRP-Protein L (GenScript, M00098) was added, incubated at room temperature for 1 hour, and then washed. After adding 50 μl of 1-Step Ultra TMB-ELISA substrate solution (Thermo Fisher Scientific, 34028) to develop color, the reaction was terminated by adding 50 μl of 2 M $H_2SO_4$ at a time, and the absorbance was analyzed using an Epoch microplate spectrophotometer (BioTek).

As a result, it was confirmed that, among the glycosylated Fc variants, only the Fc variants into which L235P was introduced showed that the binding ability to FcyRI (FIG. 6A) was removed, and the binding ability to FcγRIIIa-158V was reduced compared to wild-type IgG1 (FIG. 6B).

**Example 7. Construction of glycosylated Fc variant library to remove FcγRs binding ability**

Based on the L235P variant prepared in Example 6, glycosylated Fc engineering was performed using the established CHO cell Fc display system to completely remove the binding ability to FcγRIIIa-158V. Specifically, in order to select the glycosylated Fc variants with removed FcyRs binding ability, a CHO cell-based glycosylated antibody Fc variant library was prepared, wherein mutation was introduced to four regions of Fc (Lower hinge, B/C loop, C'/E loop and F/G loop) having very important effects on FcyRs binding to human IgG1 Fc into which L235P was introduced (FIG. 7). The library gene was co-transfected with the FLP expression plasmid in the same manner as the cell display system construction method described in Example 1 above, and then subjected to a selection process using Hygromycin-B medium to prepare a glycosylated Fc variant library-stable expression CHO cell line.

**Example 8. Selection and production of Fc variants with removal of FcγRs binding ability using CHO cell display**

In order to select the glycosylated Fc with removed FcγRIIIa binding ability, binding of FcyRIIIa-Alexa647 and Protein A-FITC was induced in the CHO cell Fc display system and Fc library-stable expression CHO cell line established in the above example, and then the expression level by Protein A-FITC and FcγRIIIa binding were simultaneously monitored, whereby a population expected to show the removed FcγRIIIa binding ability was selected by gating through FACS screening (FIG. 8). After recovering the selected glycosylated Fc variant-expression CHO cells through genomic DNA prep, the base sequences of the selected engineered glycosylated Fc variants were confirmed (Table 1). For ELISA assay of FcyRs binding ability of the selected engineered glycosylated Fc variants (LP1, LP5, LP6, LP7 and LP8), high purity glycosylated antibody trastuzumab Fc variants were produced and purified by the method of Example 5 above (FIG. 9).

[TABLE 1]

| Name of variant | Introduced mutant |
|---|---|
| LP1 | L235P, D265N |
| LP5 | L235P, P329V |
| LP6 | L235P, P329L |
| LP7 | L235P, D265M |
| LP8 | L235P, D265L, P331L |

**Example 9. Analysis of human FcγRs binding ability of glycosylated trastuzumab Fc variants**

ELISA assay was performed to confirm the FcyRs binding ability of the glycosylated trastuzumab Fc variants (LP1, LP5, LP6, LP7 and LP8) purified in Example 8 above. Specifically, 50 μl of each of FcyRs-GST (FcyRI-GST, FcγRIIa-131H-GST, FcγRIIa-131R-GST, FcyRIIb-GST, FcγRIIIa-158V-GST and FcyIIIa-158F-GST) diluted with 4 μg/ml in 0.05 M $Na_2CO_3$ (pH 9.6) was input into a flat bottom polystyrene high bind 96 well microplate (Costar, 3590), and fixed for 16 hours at 4 °C, washed, followed by adding 100 μl of 4% skim milk (GenomicBase, SKI400) thereto and blocking for 1 hour at room temperature. The washing was performed 4 times with 180 μl of 0.05% PBST. After washing 4 times, 50 μl of each of the glycosylated trastuzumab Fc variants (LP1, LP5, LP6, LP7 and LP8) serially diluted with 1% skim milk was dispensed into each well and reacted at room temperature for 1 hour. After washing it, antibody reaction was performed using 50 μl of HRP-Protein L (GenScript, M00098) at room temperature for 1 hour, followed by washing again. After color development by adding 50 μl of 1-Step Ultra TMB-ELISA substrate solution (Thermo Fisher Scientific, 34028), the reaction was terminated by adding 50 μl of 2 M $H_2SO_4$, and then the absorbance was measured and analyzed using an Epoch microplate spectrophotometer (BioTek).

As a result, the glycosylated trastuzumab Fc variants discovered in the above example showed that, unlike the LALA (L234A/L235A) Fc variant, the binding ability to FcyRI and FcγRIIIa-158V was significantly removed. It was confirmed that the glycosylated trastuzumab Fc variants in the above examples had reduced binding ability to FcγRIIa-131H and FcγRIIa-131R than the LALA (L234A/L235A) Fc variant (FIGS. 10A to 10C).

**Example 10. Preparation of glycosylated Fc variants with reduced FcγRs binding ability**

In the above example, since the L235P variant still has binding ability to FcγRIIIa-158V, an additional mutation was introduced to reduce binding to all FcyRs. To this end, the L234A variant was combined with L235P to prepare glycosylated Fc variant LALP (L234A and L235P) (Table 2).

[TABLE 2]

| Name of variant | Introduced mutant |
|---|---|
| LALP | L234A, L235P |

**Example 11. Production of glycosylated Fc variants with reduced FcγRs binding ability and analysis of human FcγRs binding ability**

For ELISA assay of the binding ability of the glycosylated Fc variants LALP (L234A/L235P) and LALA (L234A/L235A) Fc variants prepared in Example 10 above to FcyRs, high purity glycosylated antibody trastuzumab Fc variant was produced and purified by the method of Example 5 above (FIG. 11). To confirm FcyRs binding ability of the purified glycosylated trastuzumab Fc variants, 50 μl of each of FcyRs-GST (FcyRI-GST, FcγRIIa-131H-GST, FcγRIIa-131R-GST, FcyRIIb-GST, FcyRIIIa-158V-GST and FcyIIIa-158F-GST) diluted with 4 μg/ml in 0.05 M $Na_2CO_3$ (pH 9.6) was imput into a flat bottom polystyrene high bind 96 well microplate (Costar, 3590), and fixed for 16 hours at 4 °C. After blocking with 100 μl of 4% skim milk (GenomicBase, SKI400) at room temperature for 1 hour and washing 4 times with 180 μl of 0.05% PBST, 50 μl of each of the glycosylated trastuzumab Fc variants serially diluted with 1% skim milk was dispensed into each well and reacted at room temperature for 1 hour. After washing, 50 μl of HRP-Protein L (GenScript, M00098) was added, incubated at room temperature for 1 hour, and then washed. After adding 50 μl of 1-Step Ultra TMB-ELISA substrate solution (Thermo Fisher Scientific, 34028) to develop color, the reaction was terminated by adding 50 μl of 2 M $H_2SO_4$ at a time, and the absorbance was analyzed using an Epoch microplate spectrophotometer (BioTek).

As a result, the FcyRs binding ability of the LALP (L234A/L235P) Fc variant was significantly reduced compared to that of the wild-type IgG1 Fc, and it was found that the binding ability to FcγRIIa-131H, FcγRIIa-131R and FcyRIIIa-158V was more reduced than the LALA (L234A/L235A) Fc variant, which is currently being widely used in clinical practice to reduce side effects of immune cell destruction (FIGS. 12A to 12C).

**Example 12. Construction of glycosylated Fc variant library to completely remove FcγRs binding ability**

The glycosylated Fc variant LALP (L234A and L235P) prepared in Example 10 above has reduced binding ability to FcγRIIIa-158V compared to the LALA (L234A/L235A) Fc variant. However, since some binding ability still remains, glycosylated Fc was engineered through the established CHO cell Fc display system to completely remove binding ability. Specifically, in order to select the glycosylated Fc variants with removed FcyRs binding ability, a CHO cell-based glycosylated antibody Fc variant library was prepared, wherein mutation was introduced to four regions of Fc (Lower hinge, B/C loop, C'/E loop and F/G loop) having very important effects on FcyRs binding to human IgG1 Fc into which LALP (L234A/L235P) was introduced (FIG. 7). The library gene was co-transfected with the FLP expression plasmid in the same manner as the cell display system construction method described in Example 1 above, and then subjected to a selection process using Hygromycin-B medium to prepare a glycosylated Fc variant library-stable expression CHO cell line.

**Example 13. Selection and production of Fc variants with complete removal of FcγRs binding ability using CHO cell display**

In order to select the glycosylated Fc with removed FcγRIIIa binding ability, binding of FcyRIIIa-Alexa647 and Protein A-FITC was induced in the CHO cell Fc display system and Fc library-stable expression CHO cell line established in the above example, and then the expression level by Protein A-FITC and FcγRIIIa binding were simultaneously monitored, whereby a population expected to show the removed FcγRIIIa binding ability was selected by gating through FACS screening (FIG. 8). After recovering the selected glycosylated Fc variant-expression CHO cells through genomic DNA prep, the base sequences of the selected engineered glycosylated Fc variants were confirmed (Table 3). For ELISA assay of the FcyRs binding ability of the selected engineered glycosylated Fc variants (LALP1, LALP5, LALP6, LALP7 and LALP8), high purity glycosylated antibody trastuzumab Fc variants were produced and purified by the method of Example 5 above (FIG. 13).

[TABLE 3]

| Name of variant | Introduced mutant |
|---|---|
| LALP1 | L234A, L235P, D265N |
| LALP5 | L234A, L235P, P329V |

(continued)

| Name of variant | Introduced mutant |
|---|---|
| LALP6 | L234A, L235P, P329L |
| LALP7 | L234A, L235P, D265M |
| LALP8 | L234A, L235P, D265L, P331L |

**Example 14. Analysis of human FcγRs binding ability of glycosylated trastuzumab Fc variants with complete removal of FcγRs binding ability**

ELISA assay was performed to confirm the FcyRs binding ability of the glycosylated trastuzumab Fc variants (LALP1, LALP5, LALP6, LALP7 and LALP8) purified in Example 13. Specifically, 50 μl of each of FcγRs-GST (FcyRI-GST, FcyRIIa-131H-GST, FcγRIIa-131R-GST, FcyRIIb-GST, FcyRIIIa-158V-GST and FcyIIIa-158F-GST) diluted with 4 μg/ml in 0.05 M $Na_2CO_3$ (pH 9.6) was input into a flat bottom polystyrene high bind 96 well microplate (Costar, 3590), and fixed for 16 hours under condition of 4 °C, washed, followed by adding 100 μl of 4% skim milk (GenomicBase, SKI400) thereto and blocking for 1 hour at room temperature. The washing was performed 4 times with 180 μl of 0.05% PBST. After washing 4 times, 50 μl of each of the glycosylated trastuzumab Fc variants (LALP1, LALP5, LALP6, LALP7 and LALP8) serially diluted with 1% skim milk was dispensed into each well and reacted at room temperature for 1 hour. After washing it, antibody reaction was performed using 50 μl of HRP-Protein L (GenScript, M00098) at room temperature for 1 hour, followed by washing again. After color development by adding 50 μl of 1-Step Ultra TMB-ELISA substrate solution (Thermo Fisher Scientific, 34028), the reaction was terminated by adding 50 μl of 2 M $H_2SO_4$, and then the absorbance was measured and analyzed using an Epoch microplate spectrophotometer (BioTek).

As a result, it was confirmed that the glycosylated trastuzumab Fc variants discovered in the above example had completely lost their binding ability to all FcyRs compared to wild-type IgG1 and the previously known variant LALA (L234A/L235A) (FIGS. 14A to 14C).

**Example 15. Thermal stability analysis of glycosylated trastuzumab Fc variants with complete removal of FcγRs binding ability**

To determine the temperature-dependent thermostability of glycosylated trastuzumab Fc variants (LALA, LP1, LP5, LP6, LP7, LP8, LALP, LALP1, LALP5, LALP6, LALP7 and LALP8), differential scanning fluorimetry (DSF) analysis was performed. Specifically, 45 μl of glycosylated trastuzumab Fc variant diluted with 5 μM in 1 × PBS and 5 μl of SYPRO Orange (Invitrogen, S6651) dye diluted to 200× were admixed and dispensed on a PCR plate (Thermo Scientific, AB0900W). Further, as a control, 1 × PBS was prepared in the same manner (all samples were performed in triplicate). An optically clear sealing film (Thermo Scientific, AB1170) was attached to the plate containing the sample, followed by measuring fluorescence intensity while increasing the temperature by 0.03 °C per second from 25°C to 99.9 °C using the QuantStudio 3 Real-Time PCR System (Applied Biosystems, A28567). A fluorescence value at each temperature was fitted with the Boltzmann model using OriginPro software, and then the midpoint of the sigmoidal transition curve was obtained.

As a result, the discovered glycosylated trastuzumab Fc variants were found to have a temperature similar to the decomposition temperature of wild-type IgG1, except for the LP8 (L235P/D265L/P331L) Fc variants and the LALP8 (L234A/L235P/D265L/P331L) Fc variants (FIGS. 15 and 16).

**Example 16. Analysis of human FcRn binding ability of glycosylated trastuzumab Fc variants with complete removal of FcγRs binding ability**

To confirm the FcRn binding ability associated with the *in vivo* half-life of the glycosylated trastuzumab Fc variants, ELISA assay according to pH was performed. Specifically, 50 μl of each of the glycosylated trastuzumab Fc variants diluted with 4 μg/ml in 0.05 M $Na_2CO_3$ (pH 9.6) was input into a flat bottom polystyrene high bind 96 well microplate (Costar, 3590), and fixed for 16 hours at 4 °C, followed by adding 100 μl of 4% skim milk (GenomicBase, SKI400) thereto and blocking for 1 hour at room temperature. After washing 4 times with 180 μl of 0.05% PBST (pH 6.0/pH 7.4), 50 μl of FcRn-GST serially diluted with 1% skim milk (pH 6.0/pH 7.4) was dispensed into each well and reacted at room temperature for 1 hour. After washing, the cells were incubated for 1 hour at room temperature using 50 μl of Anti-GST-HRP Conjugate (GE Healthcare, RPN1236V) and washed again. After color development by adding 50 μl of 1-Step Ultra TMB-ELISA substrate solution (Thermo Fisher Scientific, 34028), the reaction was terminated by adding 50 μl of 2 M $H_2SO_4$, and then the absorbance was measured and analyzed using an Epoch microplate spectrophotometer (BioTek).

As a result, the glycosylated trastuzumab Fc variants LALP, LALP1, LALP5 and LALP6 of the present invention were found to maintain the FcRn binding properties of wild-type IgG1 (FIG. 17).

In addition, the glycosylated trastuzumab Fc variants LP1, LP5, LP6 and LP7 of the present invention were shown to maintain the FcRn binding properties of wild-type IgG1 (FIGS. 18A and 18B).

**Example 17. Analysis of human C1q binding ability of glycosylated trastuzumab Fc variants with complete removal of FcγRs binding ability**

ELISA assay was performed to confirm the C1q binding ability of the trastuzumab Fc variants of the present invention. Specifically, 50 μl of each of the glycosylated trastuzumab Fc variants diluted with 4 μg/ml in 0.05 M $Na_2CO_3$ (pH 9.6) was input into a flat bottom polystyrene high bind 96 well microplate (Costar, 3590), and fixed for 16 hours at 4 °C, followed by blocking with 100 μl of 4% skim milk (GenomicBase, SKI400) for 1 hour at room temperature. After washing 4 times with 180 μl of 0.05% PBST, 50 μl of Clq (Quidel, A400) protein serially diluted with 1% skim milk was dispensed into each well and reacted at room temperature for 1 hour. After washing the plate, 50 μl of anti-Clq-HRP (Invitrogen, PA1-84324) was added to each well, incubated at room temperature for 1 hour, and washed again. After color development by adding 50 μl of 1-Step Ultra TMB-ELISA substrate solution (Thermo Fisher Scientific, 34028), the reaction was terminated by adding 50 μl of 2 M $H_2SO_4$, and then the absorbance was measured and analyzed using an Epoch microplate spectrophotometer (BioTek).

As a result, all of the discovered glycosylated trastuzumab Fc variants (LALP, LALP1, LALP5, LALP6 and LALP7) were found to have their binding ability to C1q effectively removed (FIG. 19).

In addition, all of LP1, LP5, LP6, LP7 and LP8 were confirmed to have their binding ability to C1q effectively removed (FIG. 20).

**Example 18. Analysis of human FcγRs binding ability of glycosylated trastuzumab Fc variants with complete removal of FcγRs binding ability**

18-1. Preparation and production of control glycosylated trastuzumab Fc variants

LALAPG (L234A/L235A/P329G) (Reference: WO2012130831A1) Fc variants currently in clinical development were prepared to compare the binding ability to FcyRs with the glycosylated Fc variants of the present invention, and these LALAPG (L234A/L235A/P329G) Fc variants were produced and purified by the method of Example 5 above (FIG. 21A).

18-2. Analysis of human FcγRI binding ability based on HER2-expressing target cells

In order to confirm and compare FcyRI binding affinities between the glycosylated trastuzumab Fc variants (LP1, LP5, LP6 and LP7) and LALA prepared in Example 18-1 above by reproducing the *in vivo* mechanism of action, binding ability analysis was performed based on SKOV-3 cells that express HER2 as the target antigen of the trastuzumab Fc variants. Specifically, $4 \times 10^6$ SKOV-3 cells were treated with 100 nM each of trastuzumab WT, trastuzumab LALA variant, and the glycosylated trastuzumab Fc variants (LP1, LP5, LP6 and LP7) of the present invention, the binding between 50 nM of tetrameric FcyRI-Alexa647 and Protein A-FITC at a ratio of 1:1000 or the binding between 100 nM of tetrameric FcyRI-Alexa647 and Protein A-FITC at a ratio of 1:1000 was induced, followed by incubation for 1 hour. After washing with 1 × PBS/EDTA, the expression level by Protein A-FITC and the removed FcyRI binding ability were analyzed using FACS.

As a result, it was confirmed that the glycosylated trastuzumab Fc variants of the present invention not only have a lower FcyRI binding ability than the conventional Fc variant LALA (L234A/L235A), but also have the binding intensity similar to that of human serum IgG used as a negative control (FIG. 21B).

18-3. Analysis of human FcγRs binding ability using octet

In order to confirm and compare FcyRs binding affinities of the glycosylated trastuzumab Fc variants (LALP1, LALP5 and LALP6) and LALAPG prepared in Example 18-1 above by reproducing the *in vivo* mechanism of action, analysis was performed using octet.

Octet is a bio-layer interferometry (BLI) system that measures bonds between biological molecules by applying the interference phenomenon of light, and compares a difference in interference patterns of white light reflected from the surface in which a ligand is bound to the surface of the biocenter tip, and the surface in which the ligand is not bound to the same. In other words, the binding or dissociation phenomenon of two molecules was measured and the binding difference between the molecules was analyzed.

Specifically, an Fc variant-introduced trastuzumab model antibody diluted in 1 × kinetic buffer or 1 × PBS was loaded onto the FAB2G sensor (a sensor chip that binds to the CH1 domain of human IgG). Then, monomeric FcyRs diluted in 1 × kinetic buffer or 1 × PBS were flowed at different concentrations to measure the affinity between FcyR and

antibodies. When analyzing FcγRI, the antibody and FcγRI were diluted in 1 × kinetic buffer, wherein FcγRI was diluted two-fold from 500 nM and analyzed to a concentration of 62.5 nM. When analyzing other FcγRs, both antibodies and FcγRs were diluted in 1 × PBS, wherein FcγRs were diluted two-fold from 5000 nM and analyzed to a concentration of 625 nM.

The analysis results were confirmed through the difference in the interference patterns of white light when $R_{eq}$ (the calculated response at equilibrium) signal was present. $R_{eq}$ is a signal when rates of binding and dissociation of two molecules are balanced, and the difference in the interference patterns of white light when the $R_{eq}$ signal is present was indicated as Binding (nm) on the Y axis.

As a result, the trastuzumab Fc variants of the present invention not only had lower FcγRI binding ability than the conventional Fc variant LALA (L234A/L235A), but also had lower FcγRI binding ability than LALAPG (L234A/L235A/P329G). It was confirmed that the inventive variants exhibit excellent effects of completely removed binding ability to hFcγRI (FIG. 22).

Further, it was confirmed that the trastuzumab Fc variants of the present invention have significantly reduced binding ability to FcγRIIIa-158F, whereas LALA (L234A/L235A) and LALAPG (L234A/L235A/P329G) still have strong binding ability to the same (FIG. 23).

The trastuzumab Fc variants of the present invention have binding ability to FcγRI, FcγRIIa-131H, FcγRIIa-131R, FcγRIIb, FcγRIIIa-158V and FcγRIIIa-158F completely removed. In particular, even with regard to FcγRI and FcγRIIIa-158F, to which the LALAPG variant has the binding ability, the trastuzumab Fc variants of the present invention were confirmed to have the binding ability completely removed (Table 4).

[TABLE 4]

| | FcγRI | | | FcγRIIa-131H | | | FcγRIIa-131R | | | FcγRIIb | | | FcγRIIIa-158V | | | FcγRIIIa-158F | | |
|---|---|---|---|---|---|---|---|---|---|---|---|---|---|---|---|---|---|---|
| | $K_D$ (n M) | $k_{on}$ ($10^4$ /Ms | $k_{dis}$ ($10^{-3}/_s$) | $K_D$ (n M) | $k_{on}$ ($10^4$ /Ms ) | $k_{dis}$ ($10^{-3}$/s) | $K_D$ (n M) | $k_{on}$ ($10^4$ /Ms ) | $k_{dis}$ ($10^{-3}/_s$) | $K_D$ (n M) | $k_{on}$ ($10^4$ /Ms ) | $k_{dis}$ ($10^{-3}/_s$) | $K_D$ (n M) | $k_{on}$ ($10^4$ /Ms ) | $k_{dis}$ ($10^{-3}/_s$) | $K_D$ (n M) | $k_{on}$ ($10^4$ /Ms ) | $k_{dis}$ ($10^{-3}/_s$) |
| Trastuzumab WT | 1.4 1± 0.0 4 | 8.9 6± 0.0 927 | 0.1 26± 0.0 033 | 610 42 | Steady-state | | 790 22 | Steady-state | | 360 0± 66 | Steady-state | | 340 18 | 11. 3± 0.3 71 | 38. 3± 1.5 6 | 679 54. 6 | 9.3 0.5 78 | 63. 2 ±3 .22 |
| Trastuzumab-LALA | 136 5.1 9 | 14. 9± 0.5 08 | 20. 3± 0.3 5 | 380 00 ± 460 00 | Steady-state | | 220 00 ± 530 0 | Steady-state | | 390 000 | Steady-state | | 119 0± 89. 4 | 7.0 2± 0.4 45 | 83. 3± 3.3 9 | 136 0± 16. 7 | 2.4 3± 0.1 92 | 33± 3.1 1 |
| Trastuzumab-LALAPG | w.b | w.b | w.b | n.b | n.b | | n.b | n.b | | n.b | n.b | | n.b | n.b | n.b | w.b | w.b | w.b |
| Trastuzumab-LALP1 | n.b | n.b | n.b | n.b | n.b | | n.b | n.b | | n.b | n.b | | n.b | n.b | n.b | n.b | n.b | n.b |
| Trastuzumab-LALP5 | n.b | n.b | n.b | n.b | n.b | | n.b | n.b | | n.b | n.b | | n.b | n.b | n.b | n.b | n.b | n.b |
| Trastuzumab-LALP6 | n.b | n.b | n.b | n.b | n.b | | n.b | n.b | | n.b | n.b | | n.b | n.b | n.b | n.b | n.b | n.b |

## Example 19. Production of combinatorial glycosylated Fc variants and analysis of human FcyRs binding ability

To analyze whether combining the known P329G mutation with the LALP (L234A/L235P) mutation of the present invention has effects on reduction in FcyRs binding, LALPPG (L234A/L235P/P329G) Fc variant was prepared and produced (Table 5). Afterwards, the trastuzumab LALPPG variant was produced and purified by the method of Example 5 above (FIG. 24). ELISA assay was performed to compare FcyRs binding abilities of the purified trastuzumab LALPPG variant and trastuzumab LALP variant with the conventional trastuzumab LALA variant and trastuzumab LALAPG variant.

Specifically, 50 μl of each of FcyRs-GST (FcyRI-GST, FcγRIIa-131H-GST, FcγRIIa-131R-GST, FcyRIIb-GST, FcyRIIIa-158V-GST and IIIa-158F-GST) diluted with 4 μg/ml in 0.05 M $Na_2CO_3$ (pH 9.6) was input into a flat bottom polystyrene high bind 96 well microplate (Costar, 3590), and fixed for 16 hours under condition of 4 °C, followed by adding 100 μl of 4% skim milk (GenomicBase, SKI400) thereto and blocking for 1 hour at room temperature. The washing was performed 4 times with 180 μl of 0.05% PBST. After washing 4 times, 50 μl of each of the glycosylated trastuzumab Fc variants serially diluted with 1% skim milk was dispensed into each well and reacted at room temperature for 1 hour. After washing it, incubation was performed by adding 50 μl of HRP-Protein L (GenScript, M00098) at room temperature for 1 hour, followed by washing again. After color development by adding 50 μl of 1-Step Ultra TMB-ELISA substrate solution (Thermo Fisher Scientific, 34028), the reaction was terminated by adding 50 μl of 2 M $H_2SO_4$, and then the absorbance was measured and analyzed using an Epoch microplate spectrophotometer (BioTek).

[TABLE 5]

| Name of variant | Introduced mutant |
|---|---|
| LALPPG | L234A, L235P, P329G |

As a result, it was shown that the LALPPG (L234A/L235P/P329G) Fc variant did not bind to all FcyRs (FIGS 25A to 25C). Through this, it was confirmed that the LALP (L234A/L235P) Fc variants may have FcyRs binding ability effectively removed even in combination with the known P329G variant.

## Example 20. Confirmation of PBMC-mediated ADCC removal of glycosylated trastuzumab Fc variants

The reduced effector function of the glycosylated Fc variants of the present invention was confirmed. Real-time cell analysis was used to analyze human peripheral blood mononuclear cell (hPBMC)-mediated antibody-dependent cellular cytotoxicity (ADCC) of the glycosylated Fc variants. Electric current flows through a cell culture vessel with a gold sensor attached to the bottom surface, and if attached cells cover the surface of the sensor, a resistance is generated and less current is generated. However, if there are no cells or the cells have been dead, the resistance decreases while the current is generated frequently. Utilizing this fact, cell death was determined. Based on SKBR-3 cells that express HER2, the target antigen of trastuzumab Fc variants, cell death analysis was performed. 10,000 cells/well of SKBR-3 in RPMI (Gibco, 11875093) containing 10% FBS (Gibco, 16000044) were seeded on E-Plate (Agilent, 300600900) and then input into xCELLigence RTCA SP (Agilent, 300600900), followed by growing for cell adhesion and proliferation. Thereafter, PBMCs were isolated from whole blood obtained from a healthy donor using Histopaque 1077 (Sigma Aldrich, 10771), washed with 1 × PBS, and then diluted with RPMI medium mixed with 10% FBS. PBMC and antibodies were placed in the E-plate, and were left at room temperature for 30 minutes to allow the cells to settle. The E-plate was placed in the RTCA equipment and monitored in real time through RTCA Software 1.2 (Agilent) over time at 37 °C and 5% $CO_2$, and cytolysis was analyzed according to the time point. Cells killed through hPBMC and antibodies were analyzed for a decrease in resistance value, and the results were expressed as cytolysis (%) using the equation below.

Cytolysis (%) = (Resistance value of well administered only target cells - Resistance value of sample well) / Resistance value of well administered only target cells × 100

As a result, the glycosylated trastuzumab Fc variants of the present invention showed a significantly lower cytolysis rate than the conventional Fc variant LALA (L234A/L235A), thereby indicating that ADCC activity was exceptionally reduced. It was confirmed that ADCC was removed more effectively than the LALAPG (L234A/L235A/P329G) Fc variants (FIG. 26).

Further, cell death caused by ADCC was confirmed through real-time cell imaging. SKBR-3 was mixed with 2 μM Red CMTPX Dye (Invitrogen, C34552) diluted in no phenol red RPMI-1640 (Gibco, 11835030) medium and incubated for 30 minutes at 37 °C and 5% $CO_2$. After labeling, cells were washed with 1 × PBS, and then 1 × $10^4$ cells/well of SKBR-3 were placed on a black plate (Greiner, 655090), followed by overnight incubation using no phenol red RPMI media containing 10% FBS and 1 × Antibiotic-Antimycotic (Gibco, 15240062) in 5% $CO_2$ incubator. The next day, PBMC were isolated from whole blood obtained from a healthy donor using Histopaque 1077. PBMCs were labeled with 0.5 μg/ml Hoechst 33342 (Invitrogen, H3570) diluted in 1 × PBS for 5 minutes at room temperature, and washed three times with 1 × PBS. After treating each well with PBMC 1 × $10^5$ cells/well, antibodies and Caspase-3/7 Detection Reagents (Invitrogen, C10423), the plate was placed in LionheartFX (Agilent) equipment, and cell death was imaged under a fluorescence microscope over time at 37 °C and 5% $CO_2$.

When apoptosis of cancer cells occurs through human peripheral blood mononuclear cells (hPBMCs), such apoptosis was imaged as green dots, and the activity of ADCC is confirmed by counting the green dots (FIGS. 27A to 27C).

As a result, the glycosylated trastuzumab Fc variants of the present invention killed significantly fewer cells than the conventional Fc variant LALA (L234A/L235A). It was confirmed that the glycosylated trastuzumab Fc variants of the present invention killed a smaller number of cells, even compared to LALAPG (L234A/L235A/P329G) Fc variants, thereby resulting in reduction of ADCC activity (FIG. 27D).

**Example 21. Confirmation of NK92-mediated ADCC removal of glycosylated rituximab Fc variants**

The glycosylated Fc variants of the present invention were introduced into a rituximab model antibody and NK92-mediated antibody-dependent cellular cytotoxicity (ADCC) was analyzed. Ramos, a cancer cell line expressing the target antigen of rituximab, and NK92 cell line (ATCC, PTA8836) expressing GFP-FcγRIIIa were recovered and then cell downed at 300 × g for 3 minutes and 125 × g for 10 minutes, respectively, followed by resuspension with PBS and washing. The prepared target cells and effector cells were input into a V-bottom 96-well plate (Corning, 3894) in amounts of 20,000 cells/well of Ramos, 200,000 cells/well (E:T=10:1) of GFP-FcγRIIIa-NK92, together with antibody samples, and then were incubated in 5% $CO_2$ incubator for 3 hours at 37 °C to induce ADCC reaction. After incubation, 5 nM of Sytox Red (Invitrogen, S34859) dye was added, followed by incubation at room temperature for 15 minutes and staining dead cells. After the reaction was completed, the plate was spun down at 200 × g for 5 minutes to recover pellets, the pellets were washed twice with PBS and analyzed using BD FACS Lyric (BioTek) equipment.

After staining the dead cells using NK92, the number of dead cells was analyzed by FACS.

The results were expressed as cytotoxicity (%) using the equation below. Cytotoxicity (%) = (Number of dead cells / Number of total cells) × 100 - Background

As a result, the glycosylated rituximab Fc variants of the present invention showed significantly lower cytotoxicity values than the conventional Fc variant LALA (L234A/L235A), thereby indicating that ADCC activity was significantly reduced. It was confirmed that the cytotoxicity value was lower than that of the LALAPG (L234A/L235A/P329G) Fc variants or human serum IgG used as a negative control (FIG. 28), thereby indicating effective removal of ADCC (FIG. 28).

**Example 22. Confirmation of ADCP reduction of glycosylated trastuzumab Fc variants**

To confirm the reduced effector function of the glycosylated Fc variants of the present invention, antibody-dependent cell-mediated phagocytosis (ADCP) was analyzed. PBMCs were isolated from whole blood obtained from a healthy donor using Histopaque 1077, and monocytes were isolated by MACS (Miltenyi, 130-118-906) CD14 positive sorting. The isolated monocytes were treated with 50 ng/ml GM-CSF (PeproTech, 300-03) in RPMI medium containing 15% FBS and differentiated into macrophages for one week at 37 °C and 5% $CO_2$. SKBR-3 labeled with 2 μM PKH67 (Sigma Aldrich, MIDI67-1KT) was placed in a V-bottom plate at 2 × $10^4$ cells/well in RPMI medium diluted with 10% human serum, while differentiated macrophages were placed in the same plate at 1 × $10^5$ cells/well after recovering through Accutase (Innovative Cell Technologies, AT104), followed by incubation with antibody in each well for 4 hours at 37 °C and 5% $CO_2$. Afterwards, it was recovered through Accutase, washed with RPMI medium diluted with 10% human serum (Sigma Alrich, H6914-20ML), followed by fixation with 1% formaldehyde in a dark environment at room temperature for 10 minutes. Then, the cells were washed with 1 × PBS and labeled with APC-anti-human CD14 antibody (BioLegend, 301807) and APC-CD11b (BioLegend, 301309) in a dark environment and on ice for 30 minutes. After washing, the fluorescence value was measured using BD FACS Lyric equipment, and phagocytosis (%) was expressed using the equation below.

$$\text{Phagocytosis (\%)} = \text{Double positive} / (\text{Target cell} + \text{Double positive}) \times 100$$

As a result, the glycosylated trastuzumab Fc variants of the present invention showed a lower phagocytosis rate than the wild-type trastuzumab Fc, thereby confirming that ADCP was removed (FIG. 29).

**Example 23. Confirmation of CDC reduction of glycosylated rituximab Fc variants**

To confirm the reduced effector function of the glycosylated Fc variants of the present invention, complement-dependent cytotoxicity (CDC) was analyzed using Daudi, a cancer cell line expressing the target antigen of rituximab. In a V-bottom 96-well plate, $5 \times 10^5$ cell/well of Daudi and each antibody were input into no phenol red RPMI-1640 (Gibco, 11835030) medium containing 20% human complement serum (Sigma-Aldrich, S1764), and then incubated for 1 hour at 37 °C under 5% $CO_2$. After incubation, lysed cells were labeled for 20 minutes at room temperature with FITC Annexin V Apoptosis Detection Kit with 7-AAD (Biolegend, 640922) prepared in Annexin V binding buffer (Biolegend, 422201) in a dark environment. Afterwards, it was washed with Annexin V binding buffer, fixed with 3% formaldehyde at room temperature for 10 minutes, and then washed in the same manner. The diluted samples were measured for fluorescence value using BD FACS Lyric equipment and analyzed for cytotoxicity (%) using the equation below.

Cytotoxicity (%) = (Pro-apoptotic cell + Dead cell)/(Total cell) $\times$ 100 - Background

As a result, the glycosylated rituximab Fc variants of the present invention showed significantly lower cytotoxicity values than the conventional Fc variant LALA (L234A/L235A) and LALAPG (L234A/L235A/P329G) Fc variants. Further, it was confirmed that there was an excellent effect in reducing CDC (FIG. 30).

**Example 24. Activity analysis of glycosylated trastuzumab Fc variants in mice**

To confirm FcRn binding ability of the glycosylated trastuzumab Fc variants of the present invention, which is associated with the *in vivo* half-life in mice, ELISA assay was performed using mouse FcRn-GST by the method of Example 16 above.

As a result, the trastuzumab Fc variants of the present invention were shown to maintain the FcRn binding properties of wild-type IgG1 (FIG. 31A).

Further, *in vivo* PK was analyzed to understand the interaction of the glycosylated trastuzumab Fc variants of the present invention in an *in vivo* system. An antibody was intravenously injected into C57BL/6 mice at 2 mg/kg using 5 mice per variant. 1, 24, 168, 312, 504, 672, 840, 1,008, 1,176 and 1,344 hours after injection, blood was collected from each mouse followed by centrifugation at 1,000 $\times$ g for 15 minutes thus to ensure a serum from the supernatant. The serum was stored at -80 °C and then analyzed through ELISA to measure an antibody concentration in serum. After coating a high binding plated with HER2 diluted with 4 $\mu$g/ml in 0.05 M $Na_2CO_3$ (pH 9.6), it was fixed 4°C for 16 hours. After blocking with 100 $\mu$l of 4% skim milk (GenomicBase, SKI400) at room temperature for 1 hour and washing 4 times with 180 $\mu$l of 0.05% PBST, standards and serum serially diluted with 1% skim milk were dispensed into each well and reacted at room temperature for 1 hour. After washing, 50 $\mu$l of goat human IgG H+L (Jackson Immunoresearch, 109-036-003) was added, followed by incubation at room temperature for 1 hour, and then washing. Subsequently, after color development by adding 50 $\mu$l of 1-Step Ultra TMB-ELISA substrate solution (Thermo Fisher Scientific, 34028), 50 $\mu$l of 2 M $H_2SO_4$ was added at a time to terminate the reaction, and then the absorbance at 450 nm was measured using Epoch microplate spectrophotometer (BioTek) equipment. The antibody concentration was analyzed from a standard curve to determine each concentration of trastuzumab and the glycosylated trastuzumab Fc variants over time. As a result, it was confirmed that the glycosylated trastuzumab Fc variants of the present invention may maintain a concentration similar to wild-type trastuzumab and have *in vivo* half-life (FIG. 31B).

**Example 25. Analysis of mouse FcγRs binding ability of glycosylated trastuzumab Fc variants**

The use of the glycosylated trastuzumab Fc variant of the present invention was observed in animal experiments. ELISA assay was performed to confirm the binding ability of the glycosylated trastuzumab Fc variant to mouse FcyRs using mouse FcyRs-GST according to the method in Example 11 above.

As a result, it was confirmed that the binding ability of each of the glycosylated trastuzumab Fc variants (LALP1, LALP5 and LALP6) of the present invention to mFcyRI, mFcyRIIb and mFcyRIII was completely removed. Further, even with regard to mFcyRIV, to which the LALA mutant (L234A/L235A) has strong binding ability, the inventive variants showed the binding ability completely removed (FIGS. 32A and 32B).

**Example 26. Analysis of cynomolgus monkey FcγRs binding ability of glycosylated trastuzumab Fc variants**

The use of the glycosylated trastuzumab Fc variant of the present invention was observed in animal experiments. ELISA assay was performed to confirm the binding ability of the glycosylated trastuzumab Fc variant to cynomolgus monkey FcyRs using the cynomolgus monkey FcyRs-GST according to the method in Example 11 above.

As a result, it was confirmed that the binding ability of the glycosylated trastuzumab Fc variants (LALP1, LALP5 and LALP6) of the present invention to cFcyRIIa, cFcyRIIb and cFcyRIII was completely removed. Further, even with regard to cFcyRI and cFcyRIII, to which the LALA mutant (L234A/L235A) has strong binding ability, the inventive variants showed the binding ability completely removed (FIGS. 33A and 33B).

Binding affinities to human FcyRs, mouse FcyRs, cynomolgus monkey FcyRs and human C1q, and FcRn; thermal stability (Tm), ADCC activity and CDC activity were compared between the glycosylated trastuzumab Fc variants (LALP1, LALP5 and LALAP6) of the present invention and the IgG1 wild-type, LALA variant and LALAPG variant (Tables 6 and 7).

[TABLE 6]

| | | hFcγ RI | hFcγ RIIa (H) | hFcγ RIIa (R) | hFcγ RIIb | hFcγ RIII a(V) | hFcγ RIII a(F) | hC1 q | FcR n | Tm(°C ) | AD CC | AD CP | CDC |
|---|---|---|---|---|---|---|---|---|---|---|---|---|---|
| Control | IgG1 WT | ++++ | +++ | +++ | ++ | +++ | +++ | +++ | +++ | 69.54 | +++ | ++ | +++ |
| | LALA | ++ | + | + | + | ++ | ++ | - | +++ | 69.83 | ++ | - | + |
| | LALALP G | + | - | - | - | - | + | + | +++ | 67.35 | + | - | + |
| Variant | LALP1 | - | - | - | - | - | - | - | +++ | 67.04 | - | - | - |
| | LALP5 | - | - | - | - | - | - | - | +++ | 68.93 | - | - | - |
| | LALP6 | - | - | - | - | - | - | - | +++ | 68.50 | - | - | - |

[TABLE 7]

| | | mFcγRI | mFcγRI Ib | mFcγRI II | mFcγRI V | cFcγRI | cFcγRII a | cFcγRII b | cFcγRII I |
|---|---|---|---|---|---|---|---|---|---|
| Control | IgG1 WT | +++ | ++ | ++ | +++ | +++ | ++ | ++ | ++ |
| Control | LALA | - | + | - | ++ | ++ | - | - | + |
| Control | LALAL PG | - | - | - | - | - | - | - | - |
| Variant | LALP1 | - | - | - | - | - | - | - | - |
| Variant | LALP5 | - | - | - | - | - | - | - | - |
| Variant | LALP6 | - | - | - | - | - | - | - | - |

## Claims

1. A human antibody Fc domain variant in which amino acid 235 of the human antibody Fc domain is replaced with proline (P).

2. The human antibody Fc domain variant according to claim 1, wherein the human antibody Fc domain comprising an amino acid sequence of SEQ ID NO: 15.

3. The human antibody Fc domain variant according to claim 1, wherein amino acid 234 of the human antibody Fc domain is further substituted with alanine (A).

4. The human antibody Fc domain variant according to claim 1, wherein amino acid 265 of the human antibody Fc domain is further substituted with leucine (L), methionine (M) or asparagine (N).

5. The human antibody Fc domain variant according to claim 1, wherein amino acid 329 of the human antibody Fc domain is further substituted with valine (V), leucine (L), or glycine (G).

6. The human antibody Fc domain variant according to claim 1, wherein amino acid 268 of the human antibody Fc domain is further substituted with glutamine (Q).

7. The human antibody Fc domain variant according to claim 1, wherein amino acid 296 of the human antibody Fc domain is further substituted with phenylalanine (F).

8. The human antibody Fc domain variant according to claim 1, wherein amino acid 300 of the human antibody Fc domain is further substituted with phenylalanine (F).

9. The human antibody Fc domain variant according to claim 1, wherein amino acid 333 of the human antibody Fc domain is further substituted with valine (V).

10. The human antibody Fc domain variant according to claim 1, wherein the human antibody is IgG1.

11. An antibody comprising the human antibody Fc domain variant according to any one of claims 1 to 10, or a fragment thereof having immunological activity.

12. An antibody-drug conjugate in which the antibody according to claim 11 or a fragment thereof having immunological activity is conjugated to one or more drugs.

13. The antibody-drug conjugate according to claim 12, wherein the drug is any one selected from the group consisting of: chimeric antigen receptor (CAR) cell therapeutics, oncolytic drug, immunotherapy agent, cytotoxic agent, angiogenesis inhibitor, kinase inhibitor, costimulatory molecule blocker, adhesion molecule blocker, anti-cytokine agent, nucleic acid therapeutics, anti-CTLA-4 agent, anti-PD-1 agent, anti-PD-L1 agent, anti-PD-L2 agent, TNF-$\alpha$ cross-linking agent, TRAIL cross-linking agent, anti-CD27 agent, anti-CD30 agent, anti-CD40 agent, anti-4-1BB agent, anti-GITR agent, anti-OX40 agent, anti-TRAILR1 agent, anti-TRAILR2 agent, tagretin, interferon-alpha, clobetasol, peg interferon, prednisone, romidepsin, bexarotene, methotrexate, triamcinolone cream, anti-chemokine, vorinostat, gabapentin, cyclosporine, rapamycin, FK506, detectable marker or reporter, TNF antagonist, antirheumatic agent, muscle relaxants, narcotics, non-steroidal anti-inflammatory drugs (NSAIDs), analgesics, anesthetics, sedatives, local anesthetics, neuromuscular blocker, antibacterial agent, psoriasis therapeutics, corticosteroid, anabolic steroid, erythropoietin, immunizations, immunoglobulin, immunosuppressant, growth hormone, hormone replacement drug, radiopharmaceuticals, antidepressant, antipsychotics, stimulant, asthma drug, beta agonist, inhaled steroid, epinephrine or its analogues, cytokine, cytokine antagonist, PD-1 antagonist, adenosine A2AR antagonist, CD73 inhibitor, CTLA-4 inhibitor, TIM-3 inhibitor, LAG-3 inhibitor, anthracycline, antisense oligonucleotide (ASO), siRNA and aptamer.

14. A pharmaceutical composition for treatment or prevention of cancer, comprising: the human antibody Fc domain variant according to any one of claims 1 to 10; an antibody including the same or a fragment thereof having immunological activity; or the antibody-drug according to claim 12 or 13.

15. The pharmaceutical composition according to claim 14, wherein the cancer is selected from the group consisting of: brain tumor, melanoma, myeloma, non-small cell lung cancer, oral cancer, liver cancer, stomach cancer, colon cancer, breast cancer, lung cancer, bone cancer, pancreatic cancer, skin cancer, head or neck cancer, cervical cancer, ovarian cancer, colon cancer, small intestine cancer, rectal cancer, fallopian tube carcinoma, anal cancer, endometrial carcinoma, vaginal carcinoma, vulvar carcinoma, Hodgkin's disease, esophageal cancer, lymph node cancer, bladder cancer, gallbladder cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, renal or ureteral cancer, renal cell carcinoma, renal pelvic carcinoma, central nervous system tumor, primary central nervous system lymphoma, spinal cord tumor, brain stem glioma and pituitary adenoma.

16. A method for treatment of cancer, comprising: administering the human antibody Fc domain variant according to any one of claims 1 to 10, an antibody including the same or a fragment thereof having immunological activity; or the antibody-drug conjugate according to claim 12 or 13, to a subject.

17. The method according to claim 16, wherein the cancer is selected from the group consisting of: brain tumor, melanoma, myeloma, non-small cell lung cancer, oral cancer, liver cancer, stomach cancer, colon cancer, breast cancer, lung cancer, bone cancer, pancreatic cancer, skin cancer, head or neck cancer, cervical cancer, ovarian cancer, colon cancer, small intestine cancer, rectal cancer, fallopian tube carcinoma, anal cancer, endometrial carcinoma, vaginal carcinoma, vulvar carcinoma, Hodgkin's disease, esophageal cancer, lymph node cancer, bladder cancer, gallbladder cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, renal or ureteral cancer, renal cell carcinoma, renal pelvic carcinoma, central nervous system tumor, primary central nervous system lymphoma, spinal cord tumor, brain stem glioma and pituitary adenoma.

**18.** A composition for target detection, comprising a conjugate in which the antibody according to claim 11 or a fragment thereof having immunological activity and a functional substance are bound.

**19.** The composition for target detection according to claim 18, wherein the functional material is one or more selected from the group consisting of contrast agents, radioisotopes, inorganic particles, fluorescent markers, dyes, enzymes, polypeptides, nucleic acids, carbohydrates and lipids.

**20.** A method for diagnosing a disease, comprising administering the composition for target detection according to claim 18 to an individual.

**21.** The method according to claim 20, wherein the disease is any one selected from the group consisting of cancer, cardiovascular disease, neurological disorder, diabetes, autoimmune disease, inflammatory disease, viral infectious disease and allergy.

[FIG. 1A]

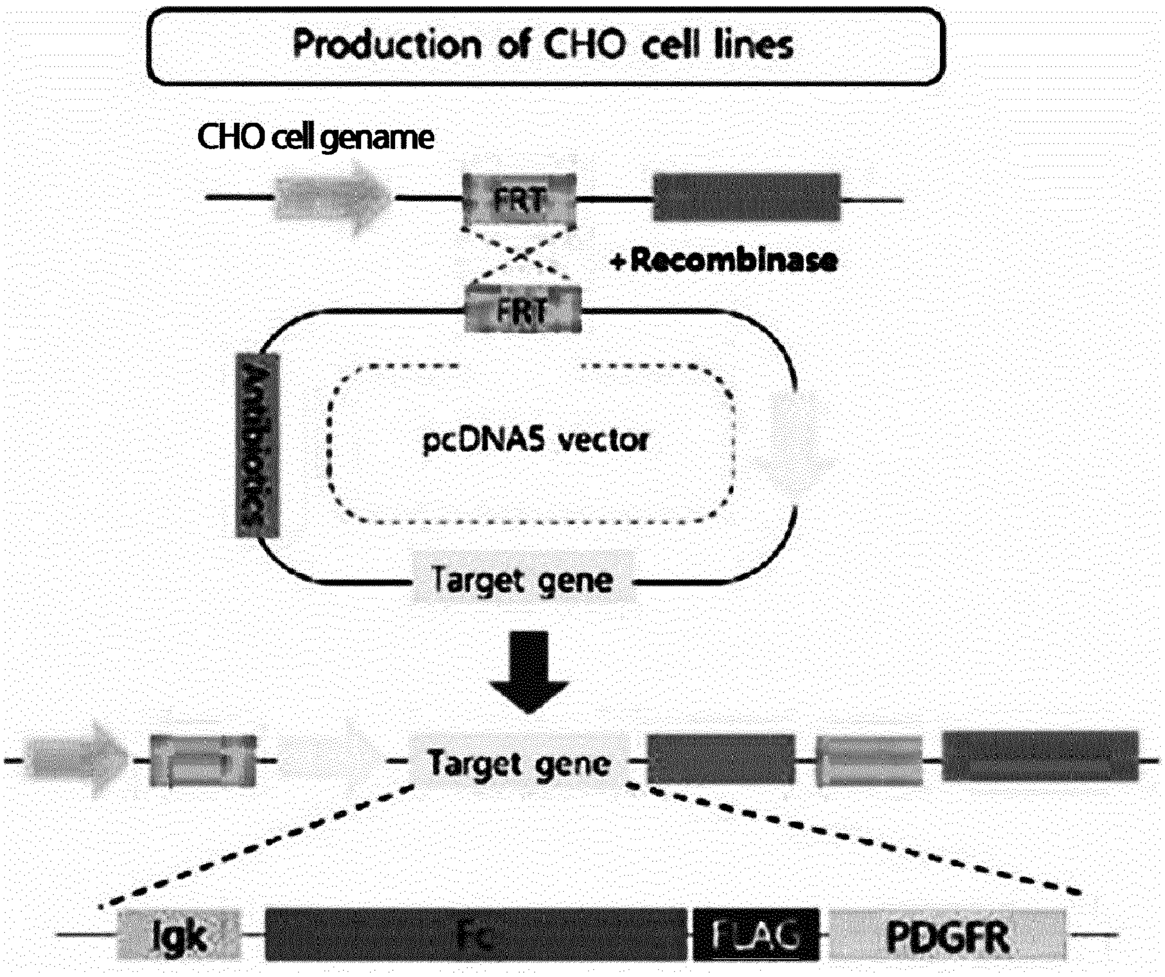
Production of CHO cell lines
CHO cell genome
FRT
+Recombinase
FRT
Antibiotics
pcDNA5 vector
Target gene
Target gene
Igk
Fc
FLAG
PDGFR

[FIG. 1B]

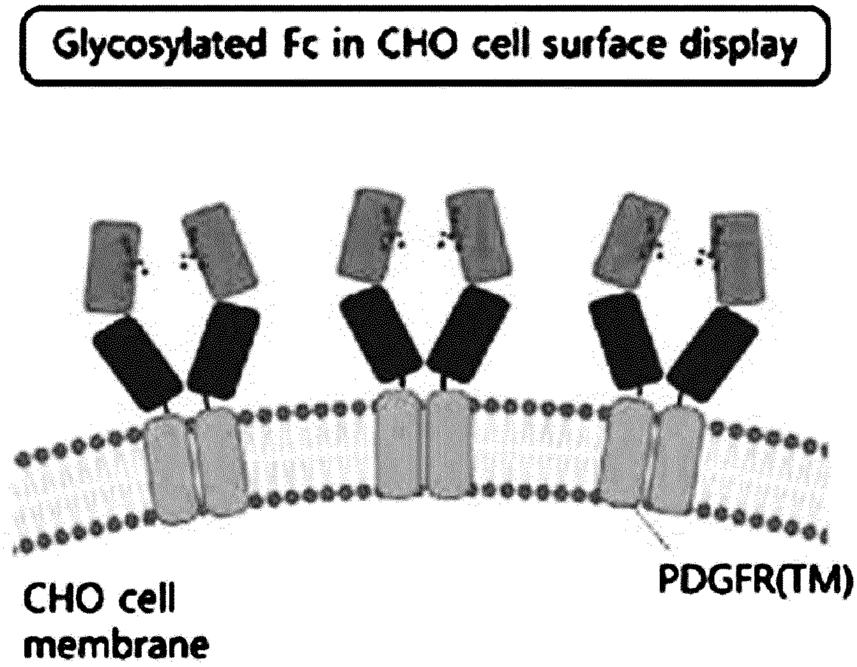
Glycosylated Fc in CHO cell surface display
CHO cell membrane
PDGFR(TM)

[FIG. 2A]

**1: Tetrameric FcγRI-streptavidin**
**2: Tetrameric FcγRIIIa-158V-streptavidin**

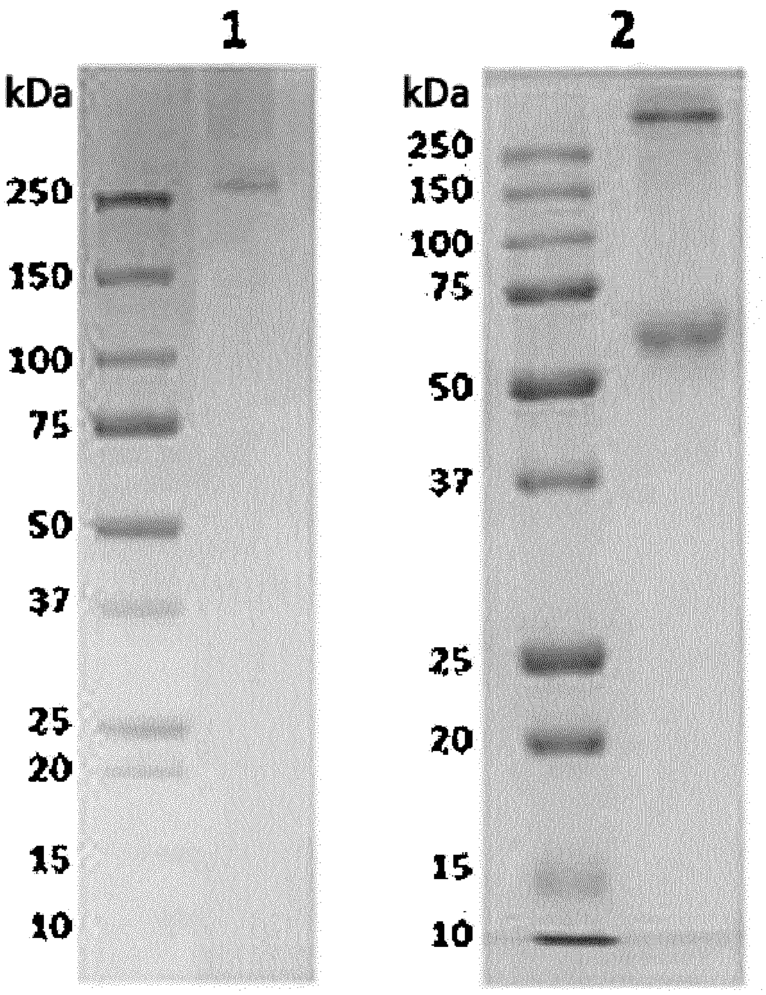

[FIG. 2B]

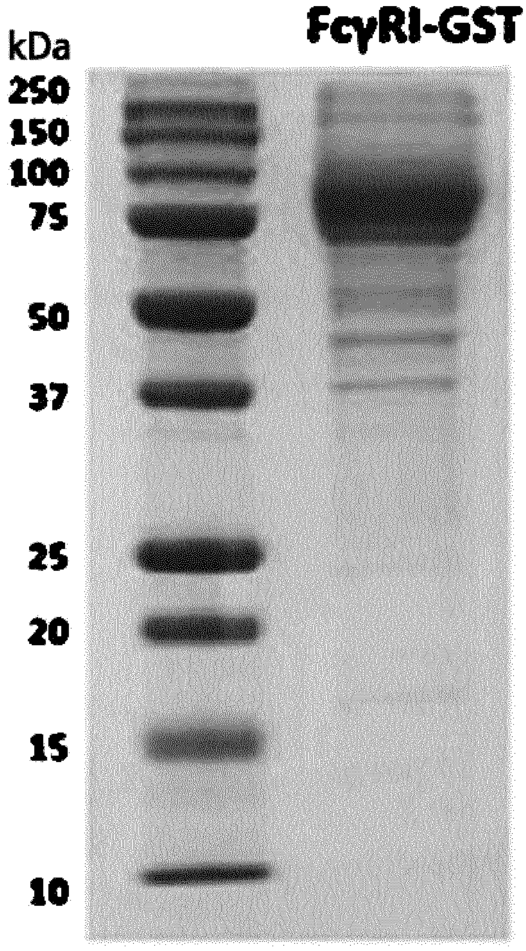

[FIG. 2C]

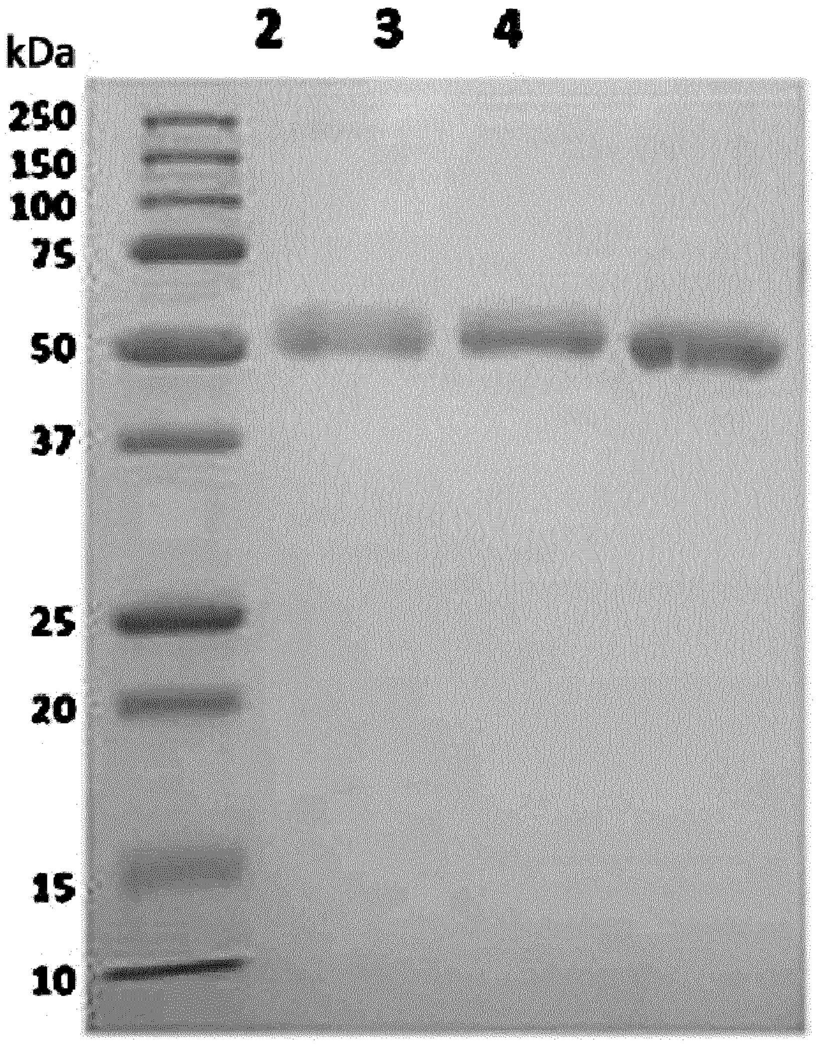


2: FcγRIIa-131H-GST
3: FcγRIIa-131R-GST
4: FcγRIIb-GST

[FIG. 2D]

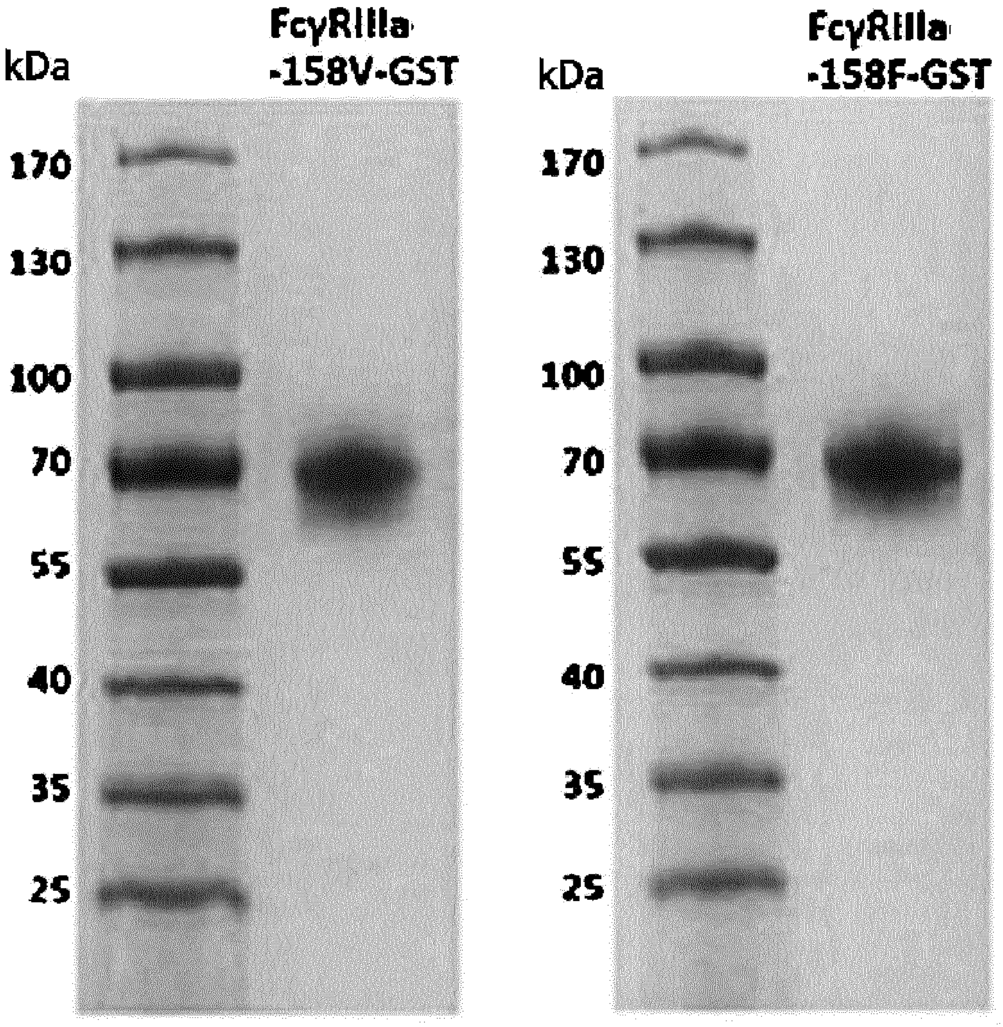
kDa
FcγRIIIa
-158V-GST
170
130
100
70
55
40
35
25
kDa
FcγRIIIa
-158F-GST
170
130
100
70
55
40
35
25

[FIG. 2E]

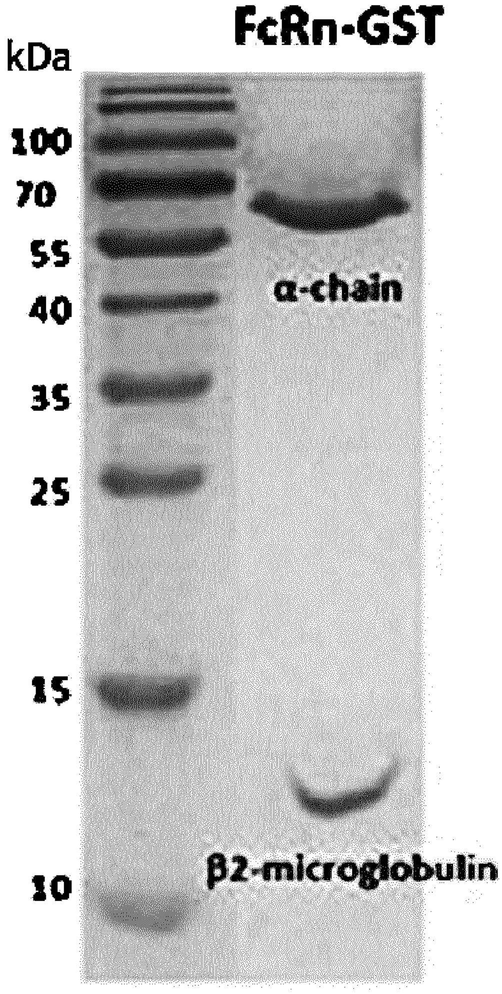

[FIG. 3A]

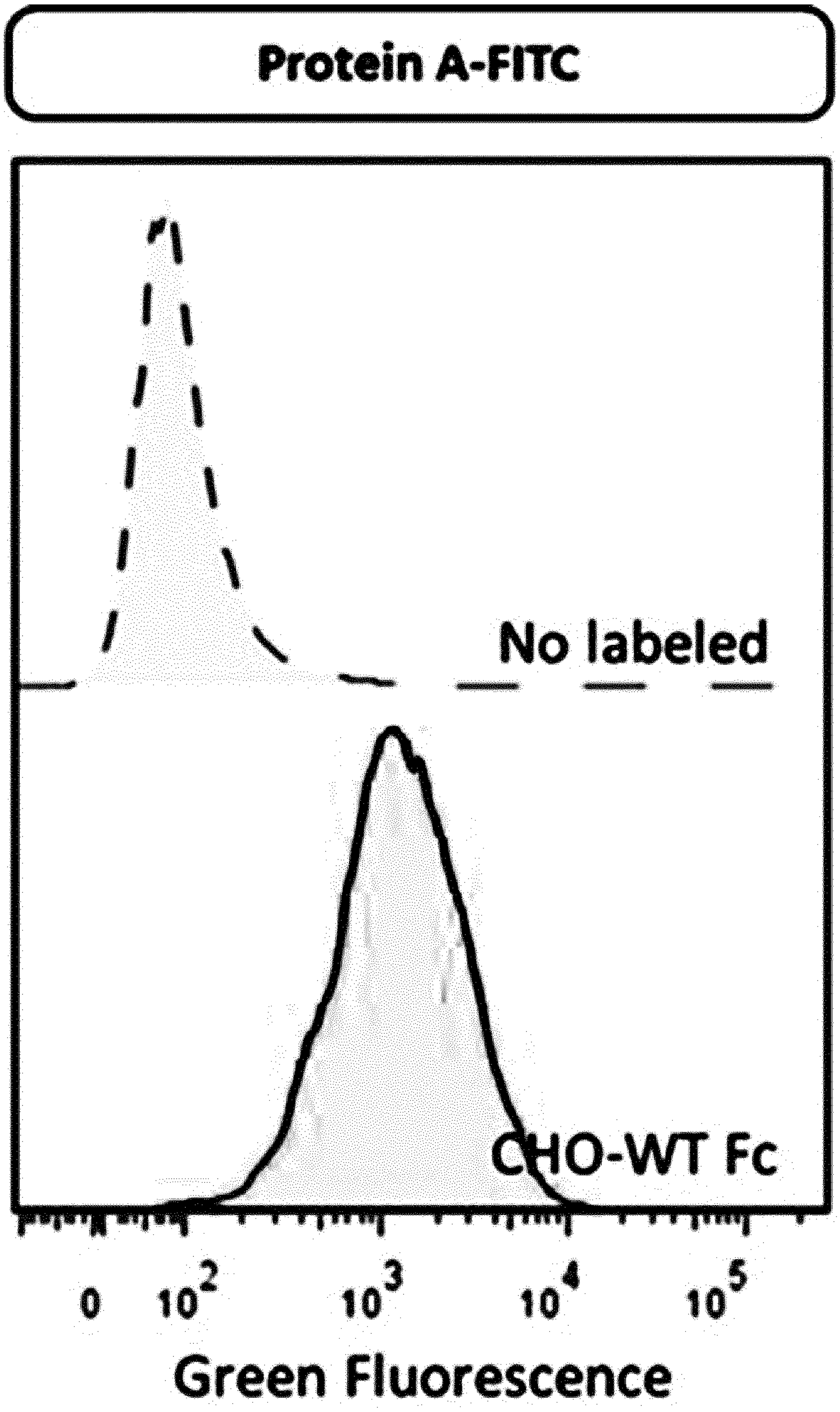
Protein A-FITC
No labeled
CHO-WT Fc
0
$10^2$
$10^3$
$10^4$
$10^5$
Green Fluorescence

[FIG. 3B]

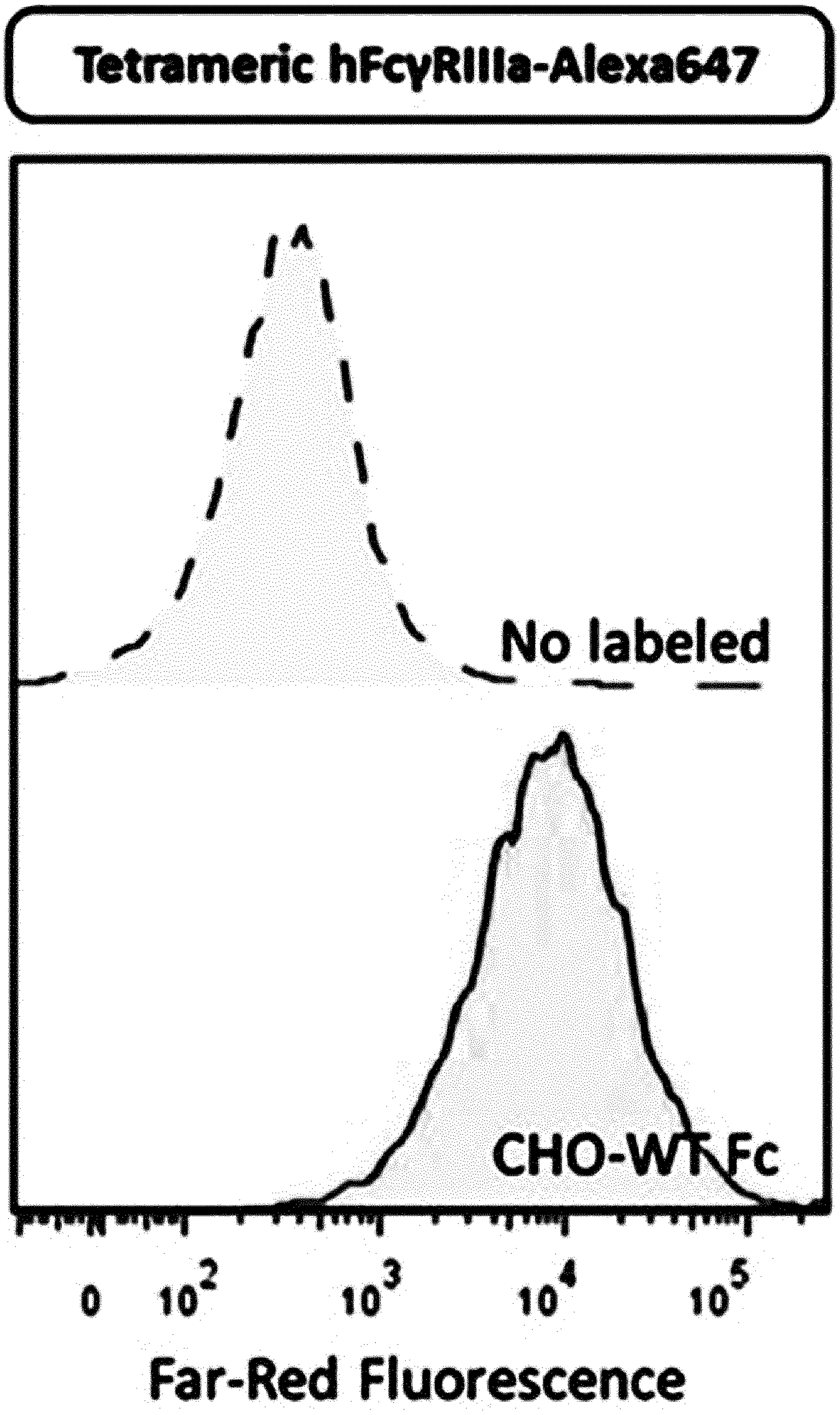
Tetrameric hFcγRIIIa-Alexa647
No labeled
CHO-WT Fc
0
10^2
10^3
10^4
10^5
Far-Red Fluorescence

[FIG. 4]

```
                      10        20        30        40        50
                  ....|....|....|....|....|....|....|....|....|....|
Human IgG1  DKTHTCPPCPAPELLGGPSVFLFPPKPKDTLMISRTPEVTCVVVDVSHED
Goat IgG1   .PCKH.RCP.-...P.....I.........T..GK..........GQD.

                      60        70        80        90       100
                  ....|....|....|....|....|....|....|....|....|....|
Human IgG1  PEVKFNWYVDGVEVHNAKTKPREEQYNSTYRVVSVLTVLHQDWLNGKEYK
Goat IgG1   ...Q.S.F..N....T.R.......F...F....A.PIQ....TG...F.

                     110       120       130       140       150
                  ....|....|....|....|....|....|....|....|....|....|
Human IgG1  CKVSNKALPAPIEKTISKAKGQPREPQVYTLPPSRDELTKNQVSLTCLVK
Goat IgG1   ...H.EG.....VR...RT...A......V.A.PQE..S.STL.V....T

                     160       170       180       190       200
                  ....|....|....|....|....|....|....|....|....|....|
Human IgG1  GFYPSDIAVEWESNGQPENNYK--TTPPVLDSDGSFFLYSKLTVDKSRWQ
Goat IgG1   ....DY.....QR.....SED.YG..TSQ..A...Y....R.R.N..S..

                     210       220
                  ....|....|....|....|....|....
Human IgG1  QGNVFSCSVMHEALHNHYTQKSLSLSPGK
Goat IgG1   E.DTYA.V..............I.KP...
```

[FIG. 5A]

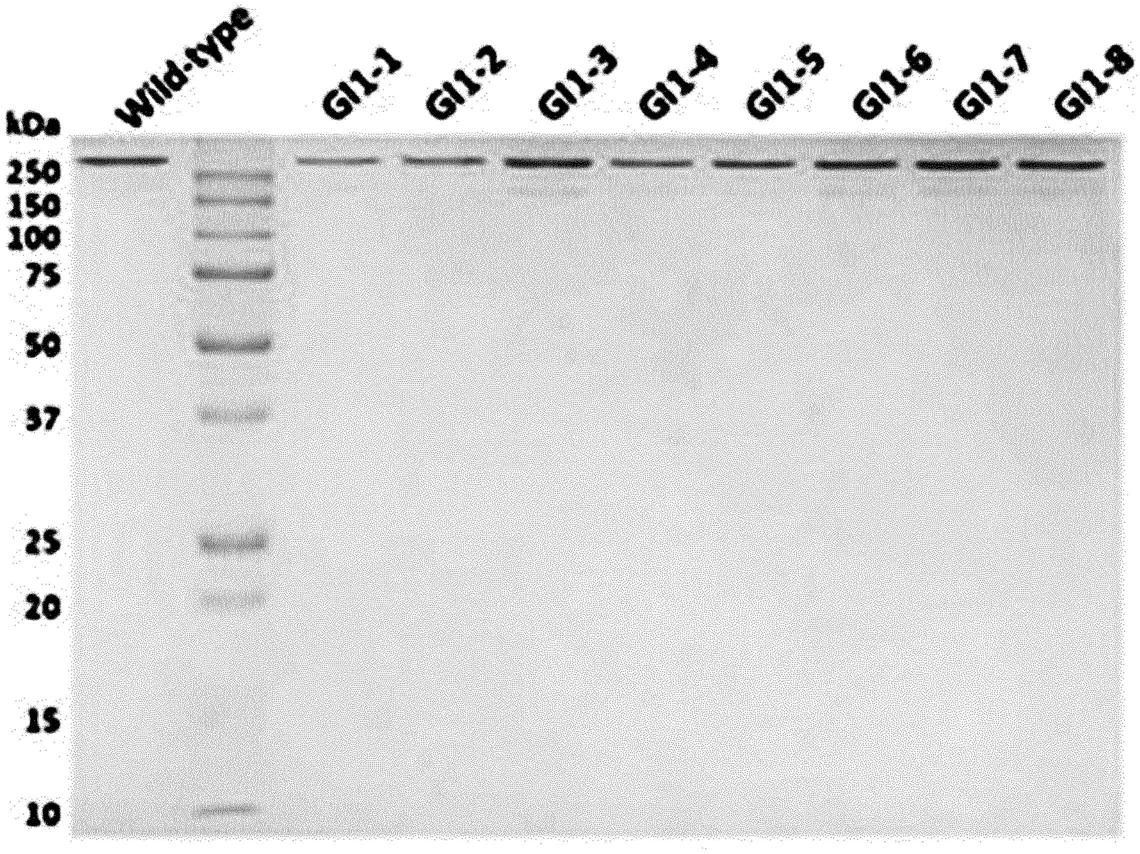


| Fc variants | Mutations |
|---|---|
| Wild-type | - |
| GI 1-1 | L235P |
| GI 1-2 | H268Q |
| GI 1-3 | Y296F/Y300F |
| GI 1-4 | E333V |
| GI 1-5 | L235P/H268Q |
| GI 1-6 | L235P/Y296F/Y300F |
| GI 1-7 | L235P/E333V |
| GI 1-8 | H268Q/Y296F/Y300F |

[FIG. 5B]

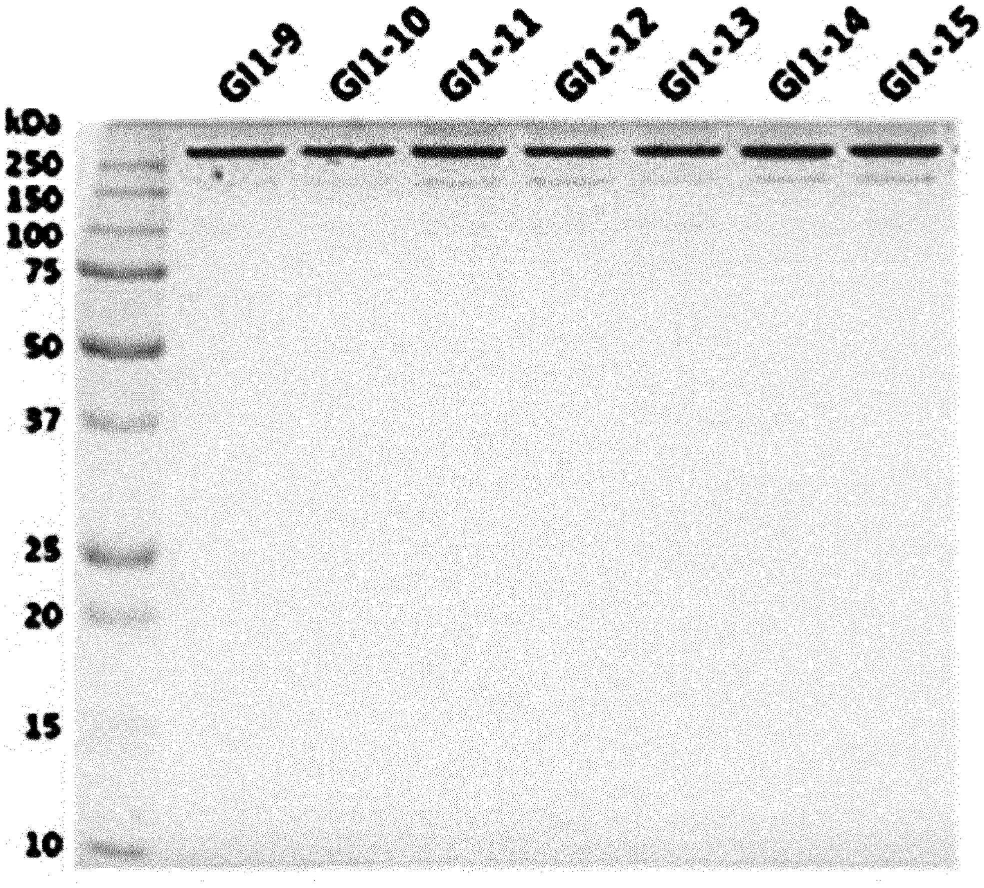


| Fc variants | Mutations |
|---|---|
| GI 1-9 | H268Q/E333V |
| GI 1-10 | Y296F/Y300F/E333V |
| GI 1-11 | L235P/H268Q/Y296F/Y300F |
| GI 1-12 | L235P/H268Q/E333V |
| GI 1-13 | L235P/Y296F/Y300F/E333V |
| GI 1-14 | H268Q/Y296F/Y300F/E333V |
| GI 1-15 | L235P/H268Q/Y296F/Y300F/E333V |

[FIG. 6A]

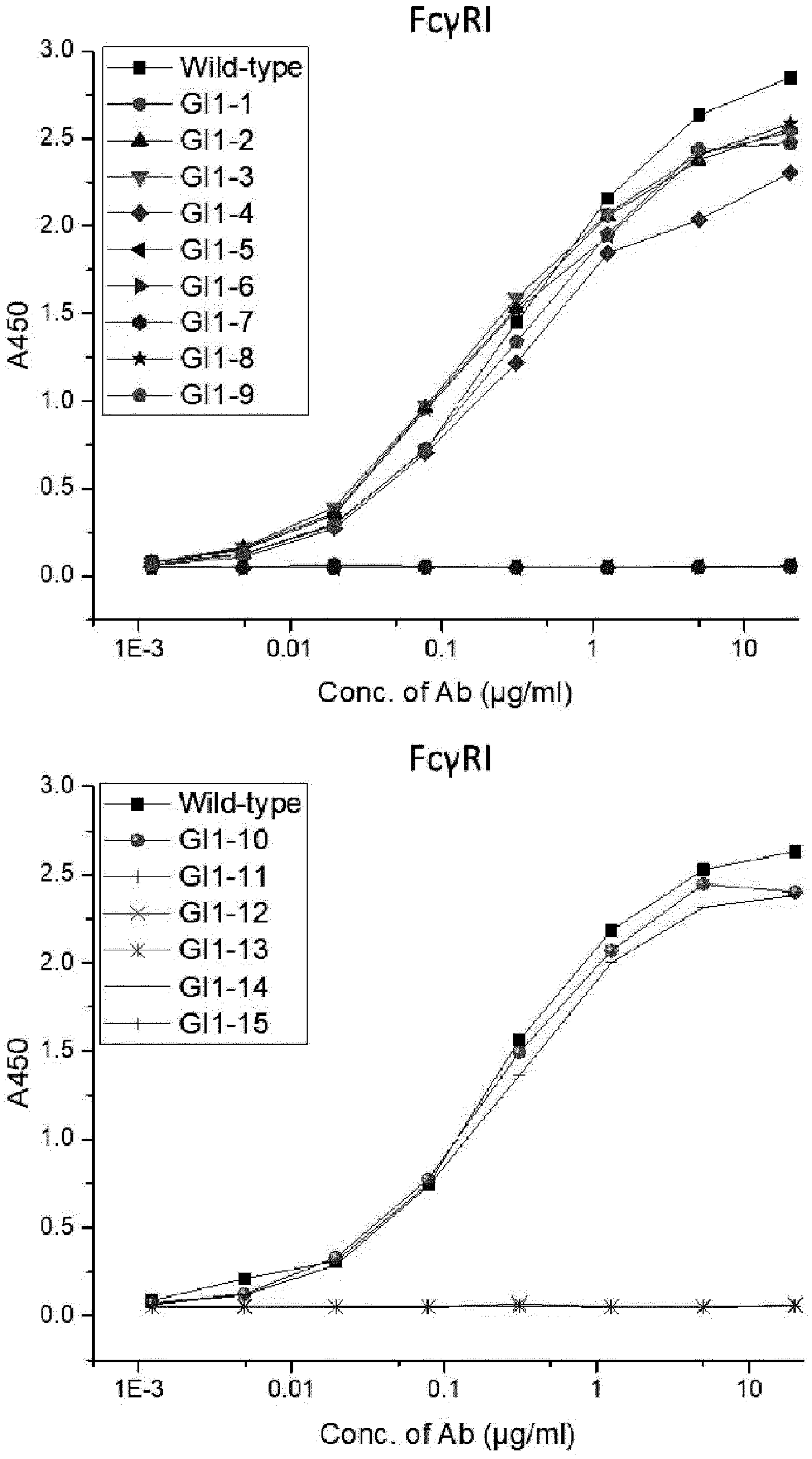
FcγRI
Wild-type
GI1-1
GI1-2
GI1-3
GI1-4
GI1-5
GI1-6
GI1-7
GI1-8
GI1-9
A450
Conc. of Ab (μg/ml)
FcγRI
Wild-type
GI1-10
GI1-11
GI1-12
GI1-13
GI1-14
GI1-15
A450
Conc. of Ab (μg/ml)

[FIG. 6B]

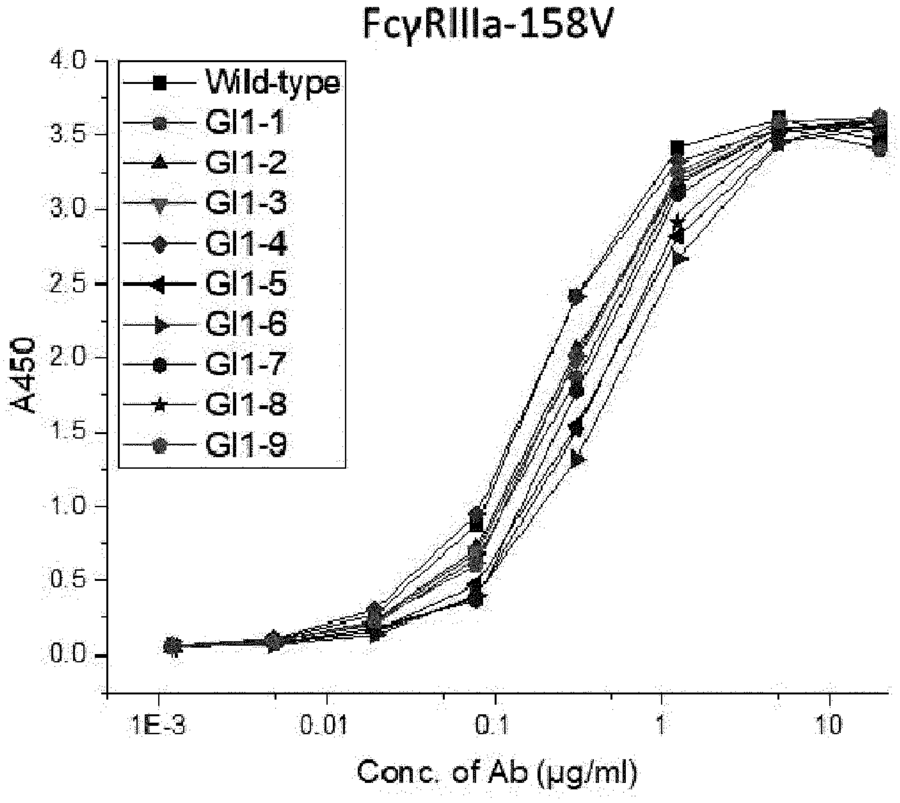
FcγRIIIa-158V
Wild-type
GI1-1
GI1-2
GI1-3
GI1-4
GI1-5
GI1-6
GI1-7
GI1-8
GI1-9
A450
Conc. of Ab (μg/ml)

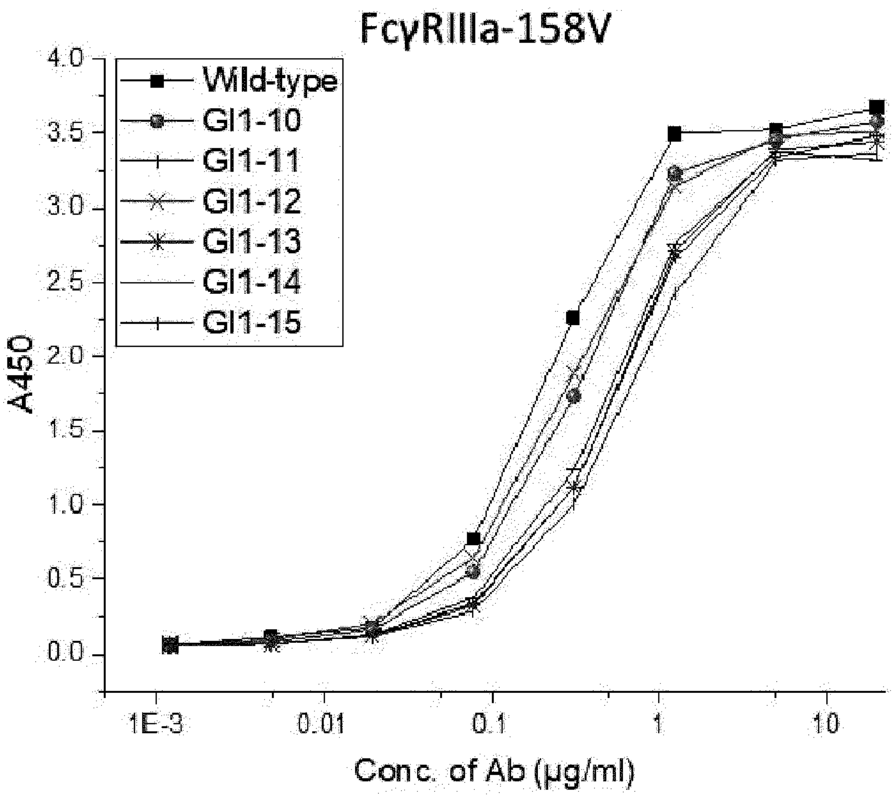
FcγRIIIa-158V
Wild-type
GI1-10
GI1-11
GI1-12
GI1-13
GI1-14
GI1-15
A450
Conc. of Ab (μg/ml)

[FIG. 7]

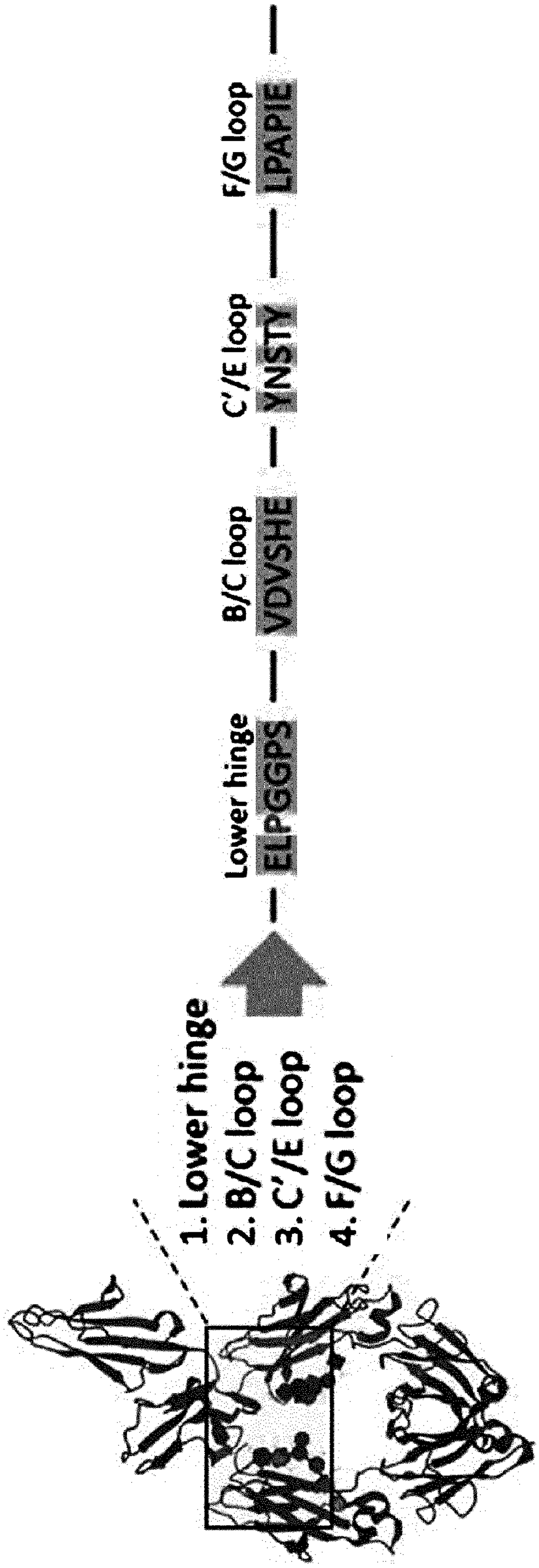
1. Lower hinge
2. B/C loop
3. C'/E loop
4. F/G loop
Lower hinge
ELPGGPS
B/C loop
VDVSHE
C'/E loop
YNSTY
F/G loop
LPAPIE

[FIG. 8]

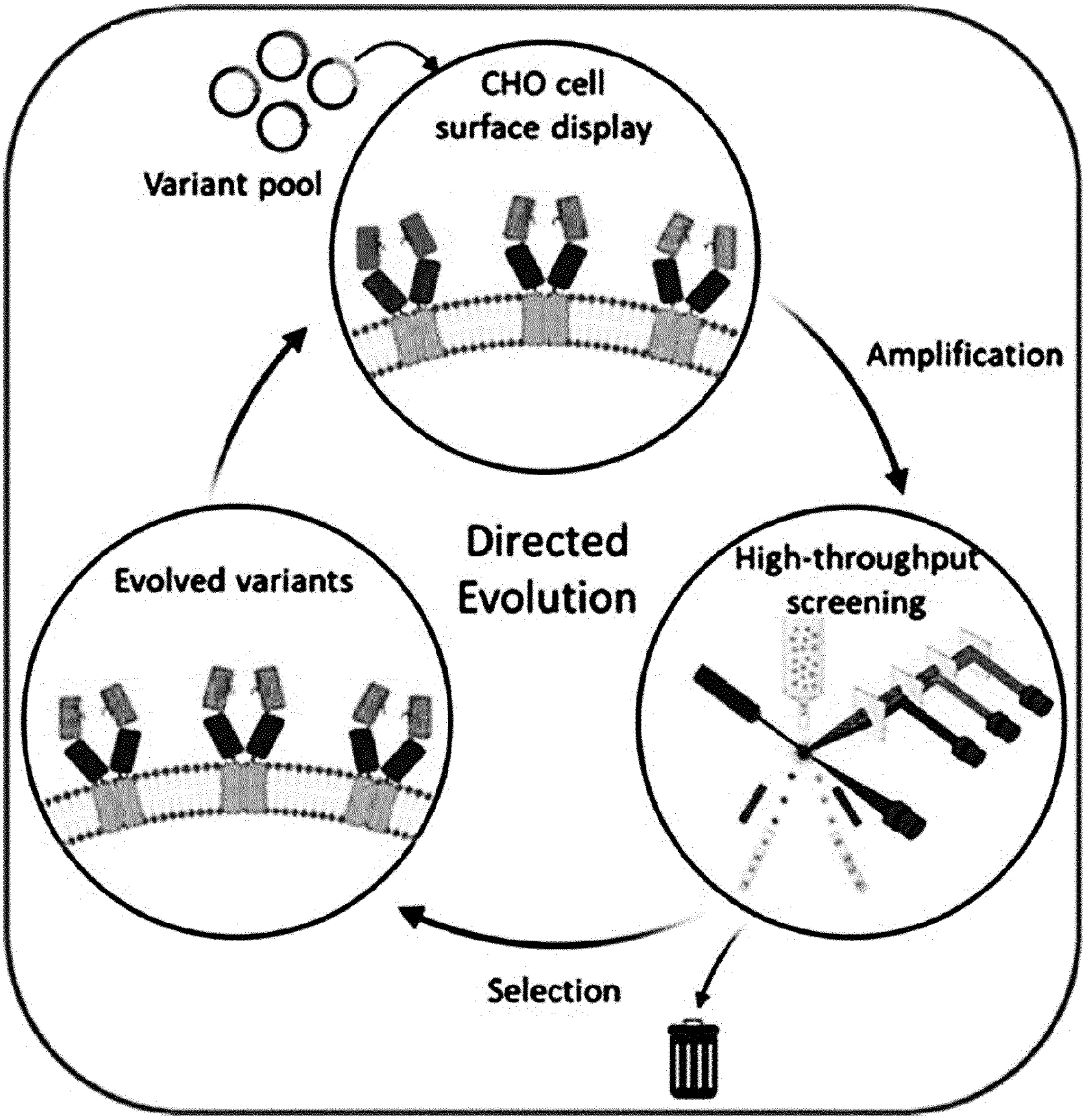
CHO cell
surface display
Variant pool
Amplification
Directed
Evolution
High-throughput
screening
Evolved variants
Selection

[FIG. 9]

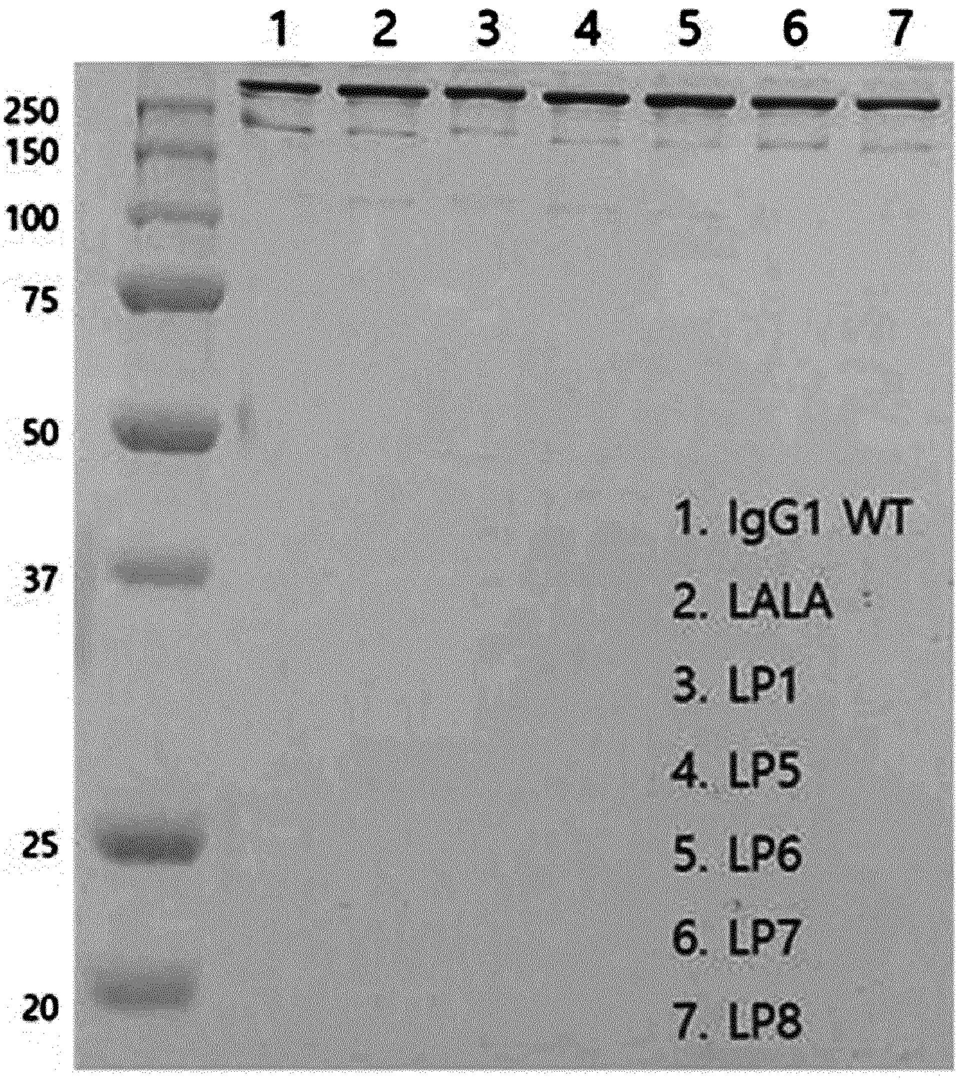
1
2
3
4
5
6
7
250
150
100
75
50
37
25
20
1. IgG1 WT
2. LALA
3. LP1
4. LP5
5. LP6
6. LP7
7. LP8

[FIG. 10A]

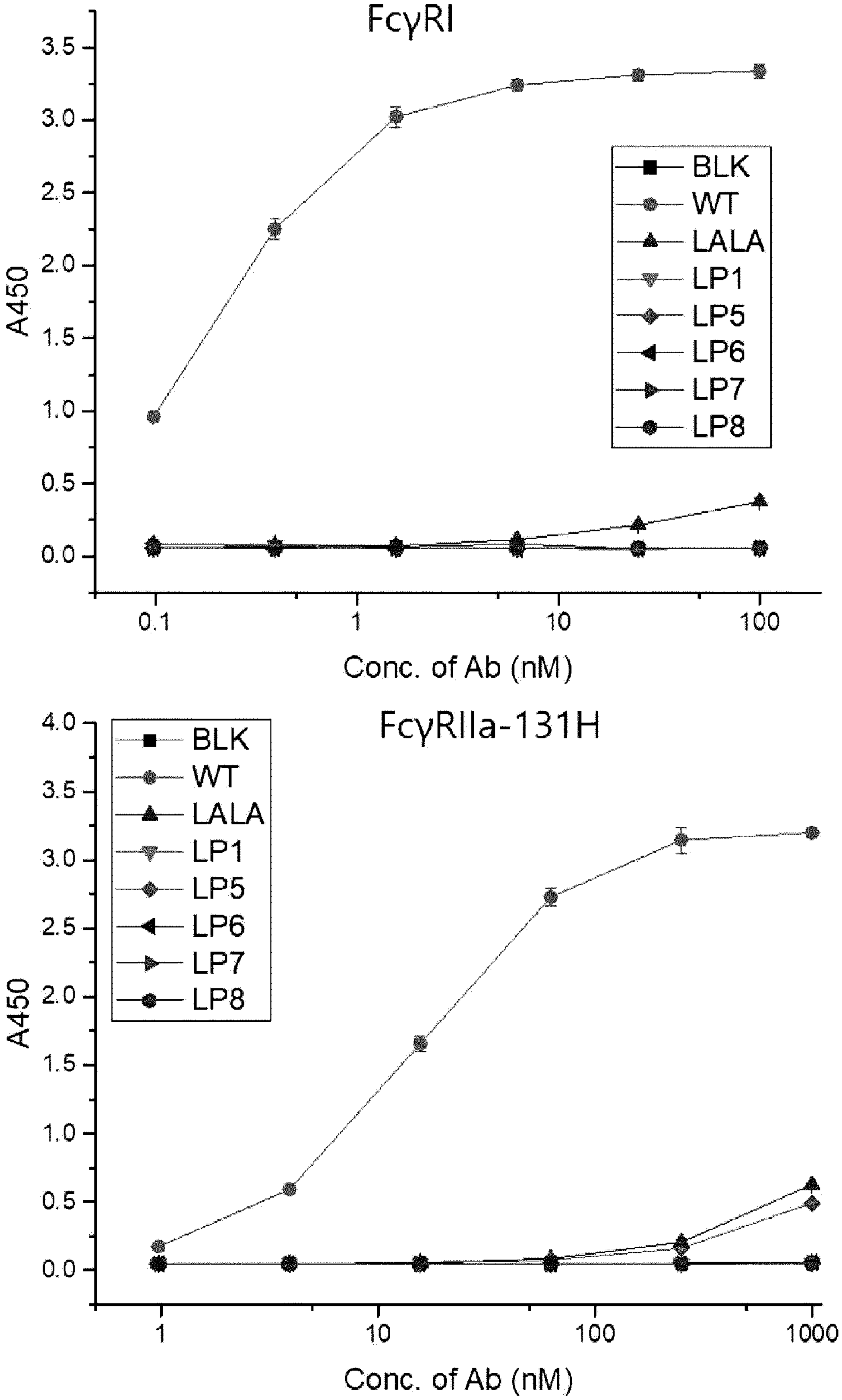
FcγRI
3.5
3.0
2.5
2.0
1.5
1.0
0.5
0.0
A450
BLK
WT
LALA
LP1
LP5
LP6
LP7
LP8
0.1
1
10
100
Conc. of Ab (nM)
FcγRIIa-131H
4.0
3.5
3.0
2.5
2.0
1.5
1.0
0.5
0.0
A450
BLK
WT
LALA
LP1
LP5
LP6
LP7
LP8
1
10
100
1000
Conc. of Ab (nM)

[FIG. 10B]

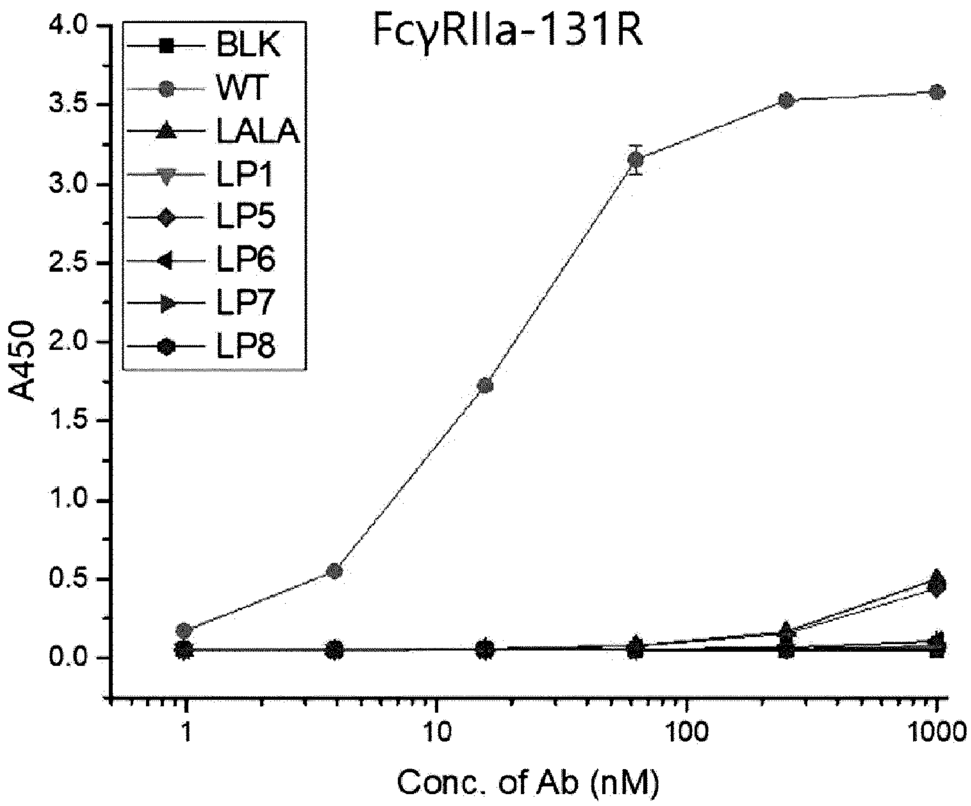
FcγRIIa-131R
BLK
WT
LALA
LP1
LP5
LP6
LP7
LP8
A450
4.0
3.5
3.0
2.5
2.0
1.5
1.0
0.5
0.0
1
10
100
1000
Conc. of Ab (nM)

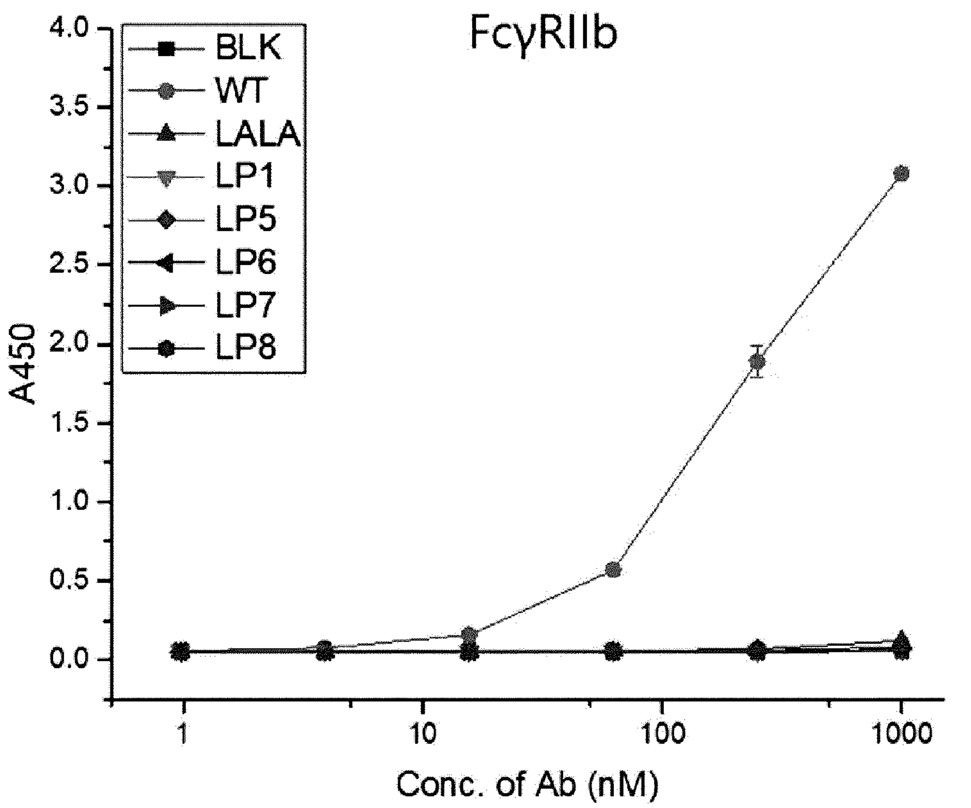
FcγRIIb
BLK
WT
LALA
LP1
LP5
LP6
LP7
LP8
A450
4.0
3.5
3.0
2.5
2.0
1.5
1.0
0.5
0.0
1
10
100
1000
Conc. of Ab (nM)

[FIG. 10C]

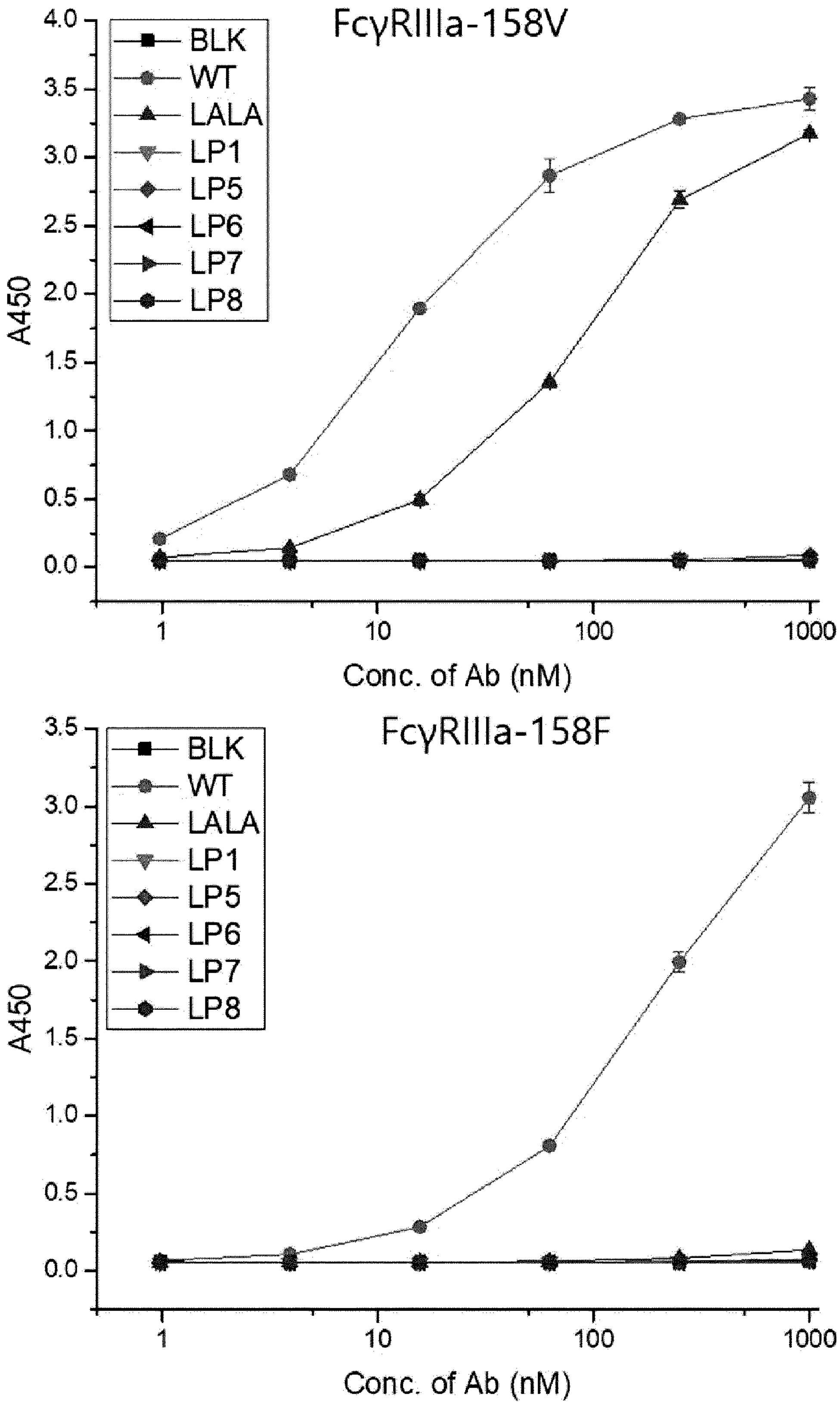
FcγRIIIa-158V
BLK
WT
LALA
LP1
LP5
LP6
LP7
LP8
A450
4.0
3.5
3.0
2.5
2.0
1.5
1.0
0.5
0.0
1
10
100
1000
Conc. of Ab (nM)
FcγRIIIa-158F
BLK
WT
LALA
LP1
LP5
LP6
LP7
LP8
A450
3.5
3.0
2.5
2.0
1.5
1.0
0.5
0.0
1
10
100
1000
Conc. of Ab (nM)

[FIG. 11]

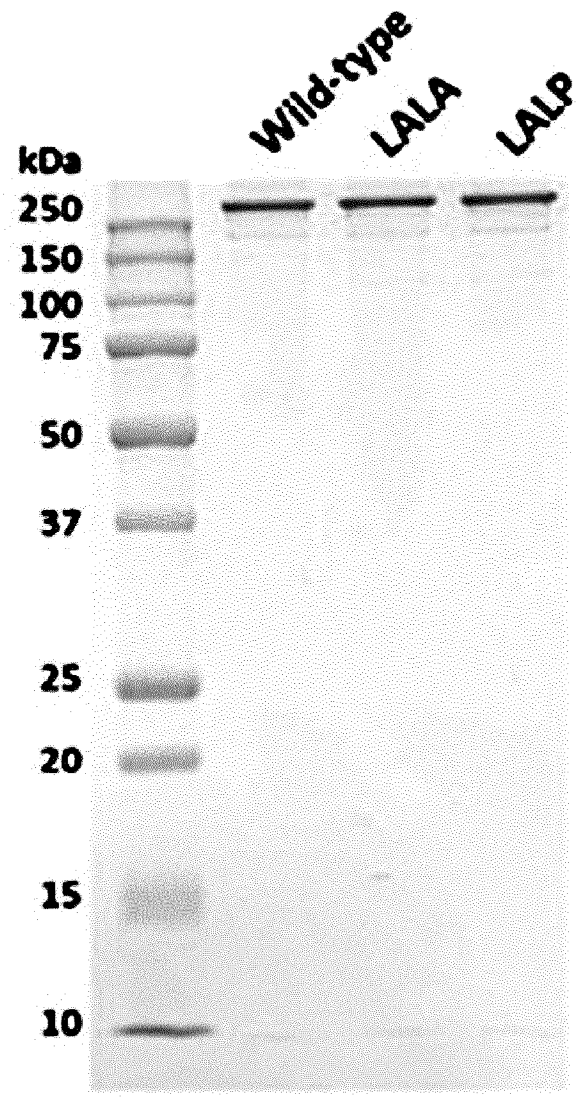


| Fc variants | Mutations |
|---|---|
| Wild-type | - |
| LALA | L234A/L235A |
| LALP | L234A/L235P |

[FIG. 12A]

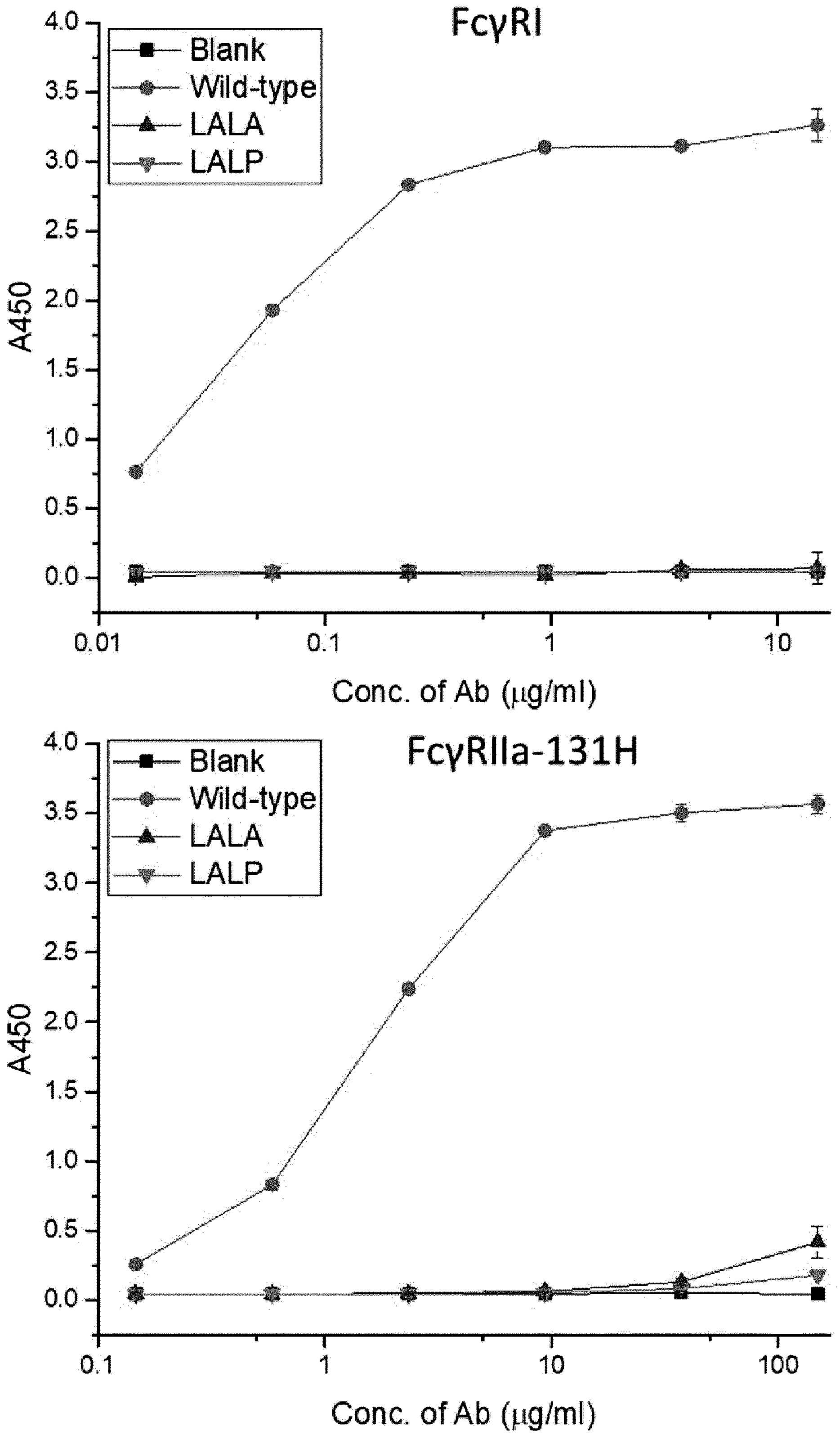
FcγRI
Blank
Wild-type
LALA
LALP
A450
4.0
3.5
3.0
2.5
2.0
1.5
1.0
0.5
0.0
0.01
0.1
1
10
Conc. of Ab (μg/ml)
FcγRIIa-131H
Blank
Wild-type
LALA
LALP
A450
4.0
3.5
3.0
2.5
2.0
1.5
1.0
0.5
0.0
0.1
1
10
100
Conc. of Ab (μg/ml)

[FIG. 12B]

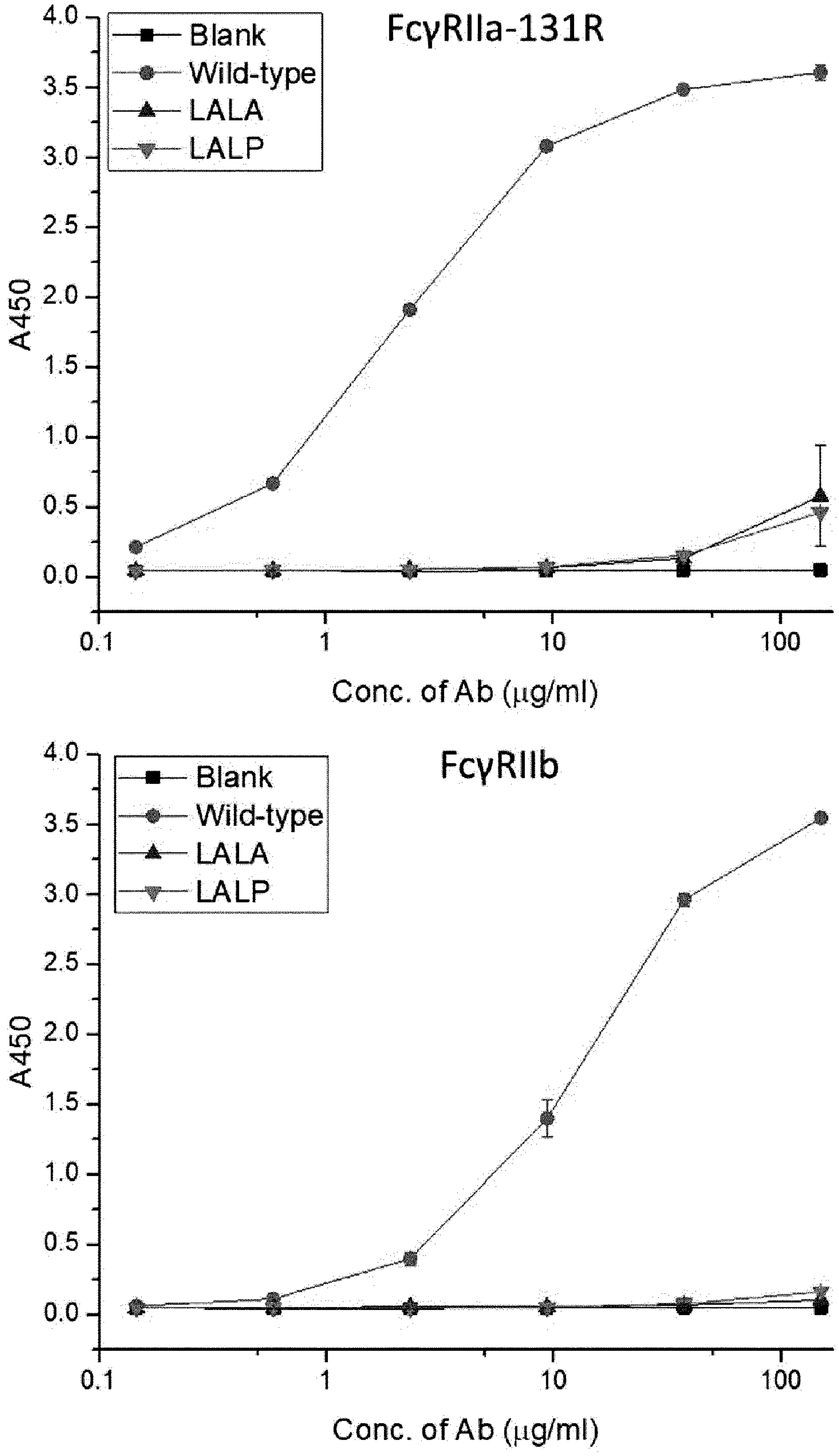
FcγRIIa-131R
Blank
Wild-type
LALA
LALP
A450
4.0
3.5
3.0
2.5
2.0
1.5
1.0
0.5
0.0
0.1
1
10
100
Conc. of Ab (μg/ml)
FcγRIIb
Blank
Wild-type
LALA
LALP
A450
4.0
3.5
3.0
2.5
2.0
1.5
1.0
0.5
0.0
0.1
1
10
100
Conc. of Ab (μg/ml)

[FIG. 12C]

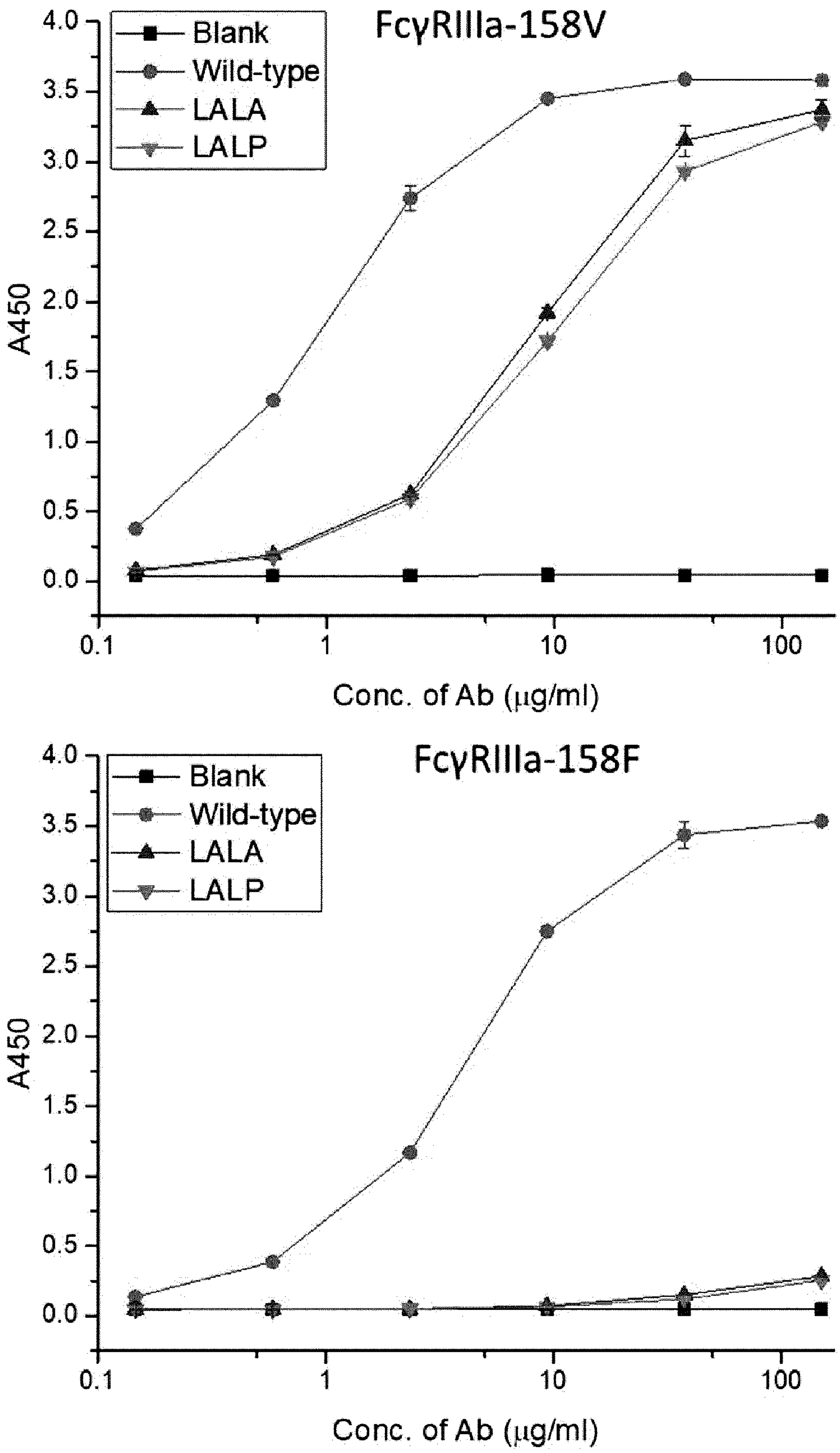
FcγRIIIa-158V
Blank
Wild-type
LALA
LALP
A450
4.0
3.5
3.0
2.5
2.0
1.5
1.0
0.5
0.0
0.1
1
10
100
Conc. of Ab (μg/ml)
FcγRIIIa-158F
Blank
Wild-type
LALA
LALP
A450
4.0
3.5
3.0
2.5
2.0
1.5
1.0
0.5
0.0
0.1
1
10
100
Conc. of Ab (μg/ml)

[FIG. 13]

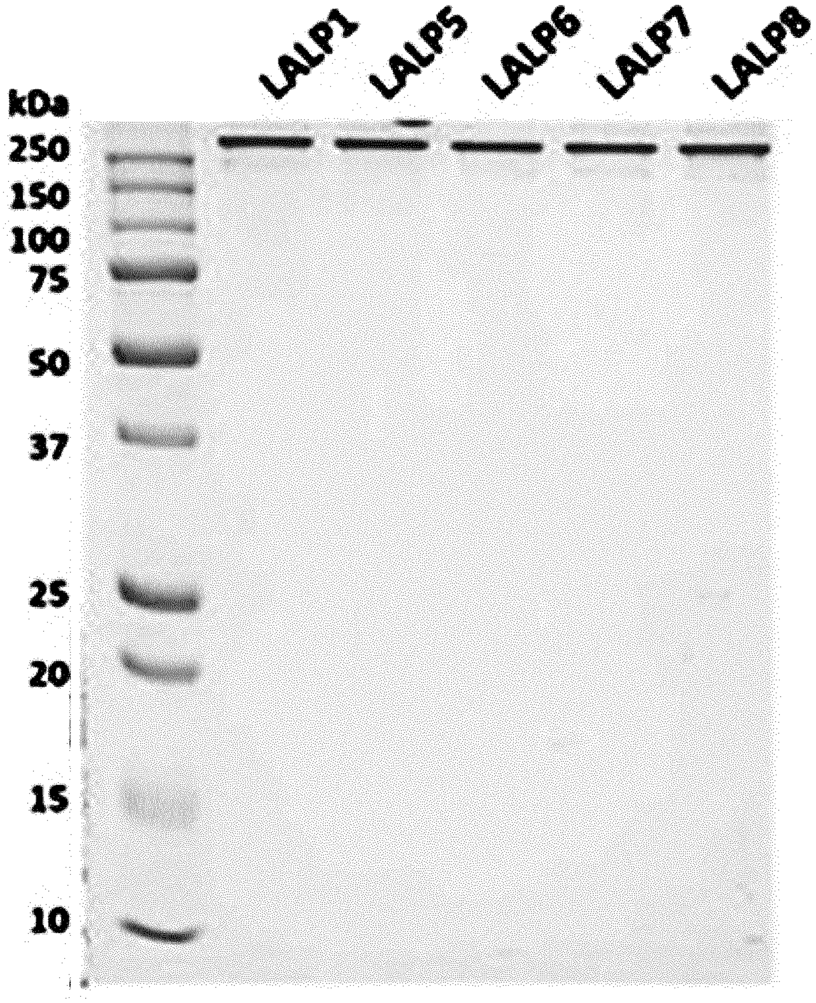


| Fc variants | Mutations |
|---|---|
| LALP1 | L234A/L235P/D265N |
| LALP5 | L234A/L235P/P329V |
| LALP6 | L234A/L235P/P329L |
| LALP7 | L234A/L235P/D265M |
| LALP8 | L234A/L235P/D265L/P331L |

[FIG. 14A]

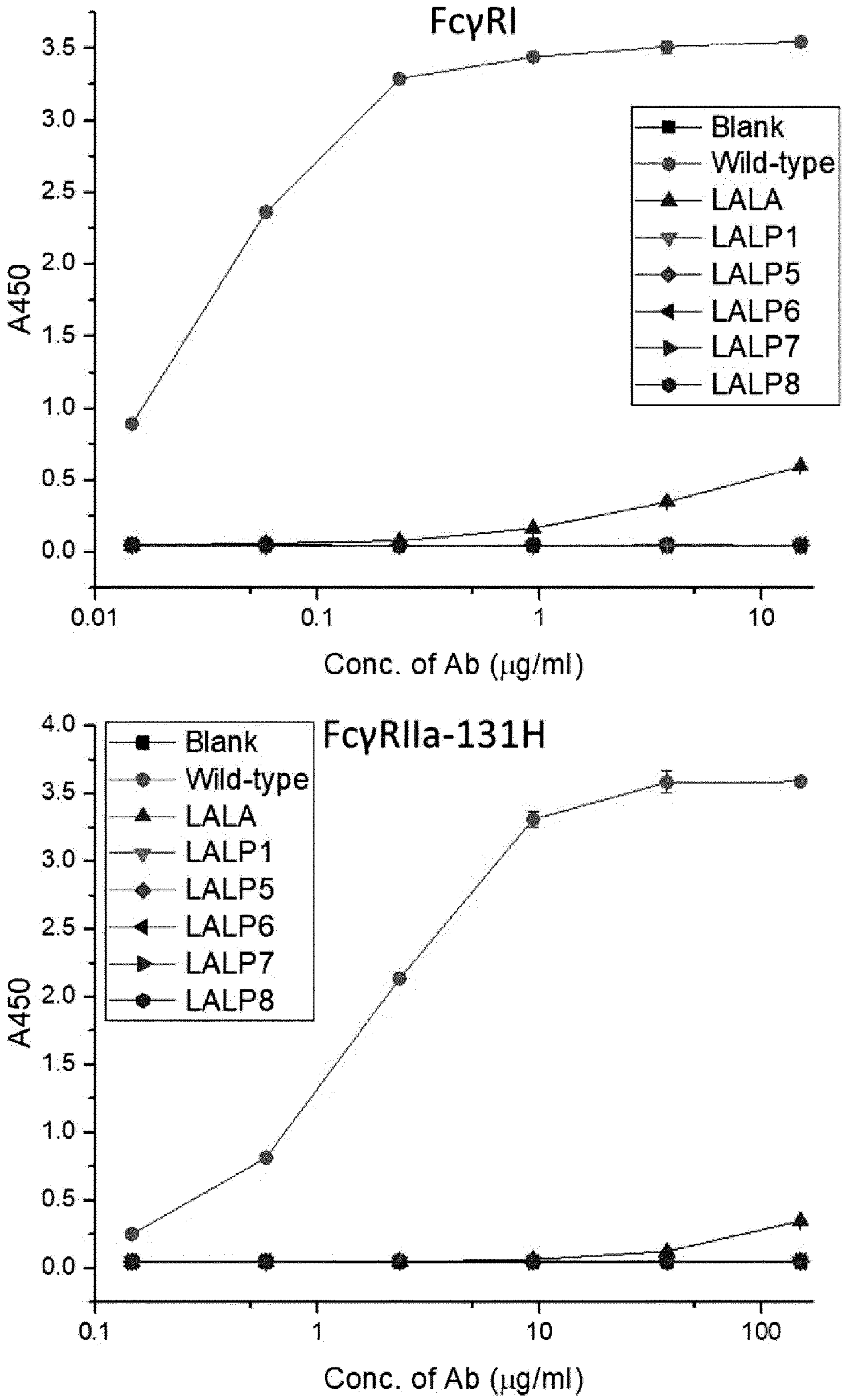
FcγRI
A450
Conc. of Ab (μg/ml)
Blank
Wild-type
LALA
LALP1
LALP5
LALP6
LALP7
LALP8
FcγRIIa-131H
A450
Conc. of Ab (μg/ml)
Blank
Wild-type
LALA
LALP1
LALP5
LALP6
LALP7
LALP8

[FIG. 14B]

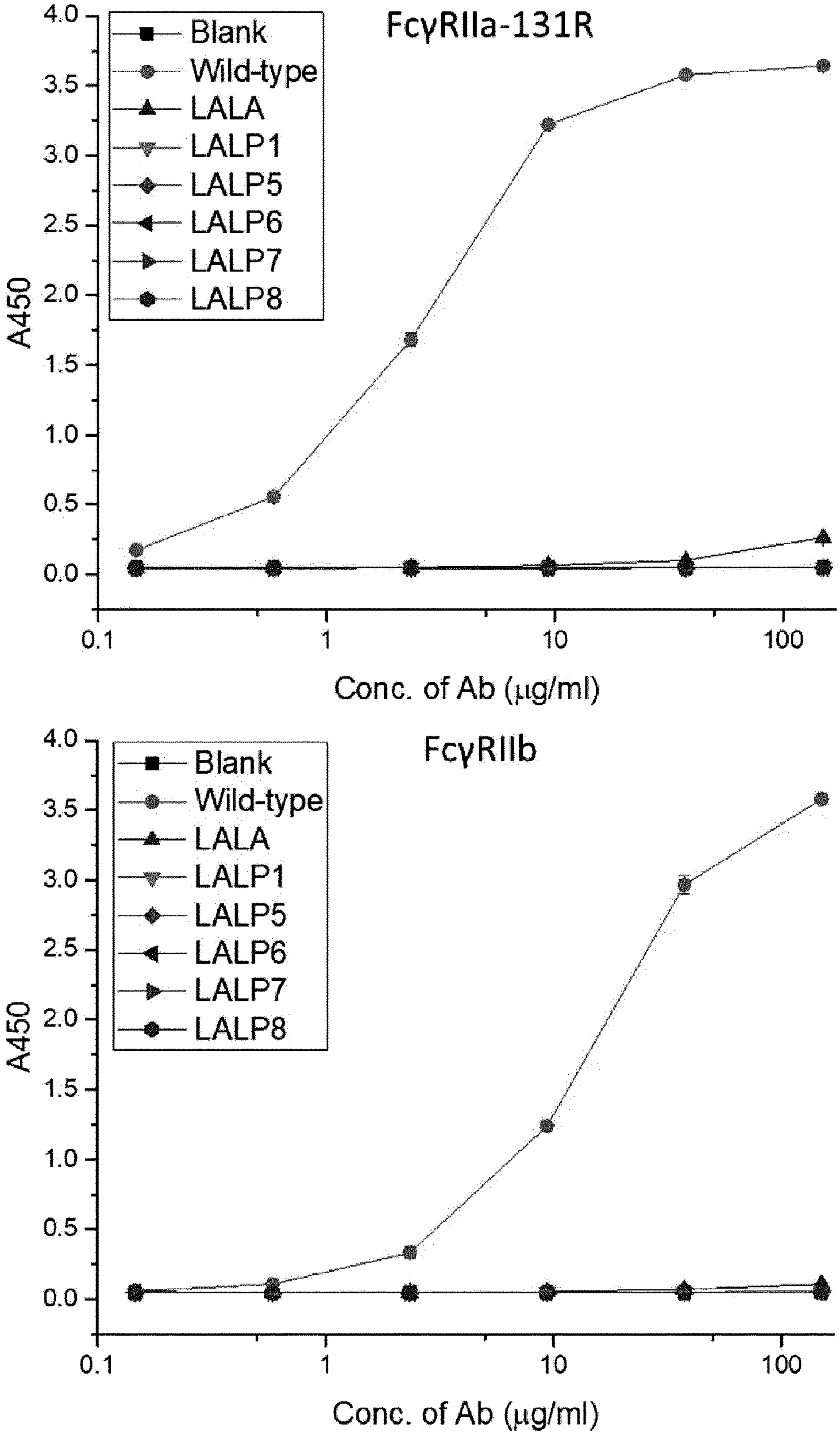
FcγRIIa-131R
Blank
Wild-type
LALA
LALP1
LALP5
LALP6
LALP7
LALP8
A450
4.0
3.5
3.0
2.5
2.0
1.5
1.0
0.5
0.0
0.1
1
10
100
Conc. of Ab (μg/ml)
FcγRIIb
Blank
Wild-type
LALA
LALP1
LALP5
LALP6
LALP7
LALP8
A450
4.0
3.5
3.0
2.5
2.0
1.5
1.0
0.5
0.0
0.1
1
10
100
Conc. of Ab (μg/ml)

[FIG. 14C]

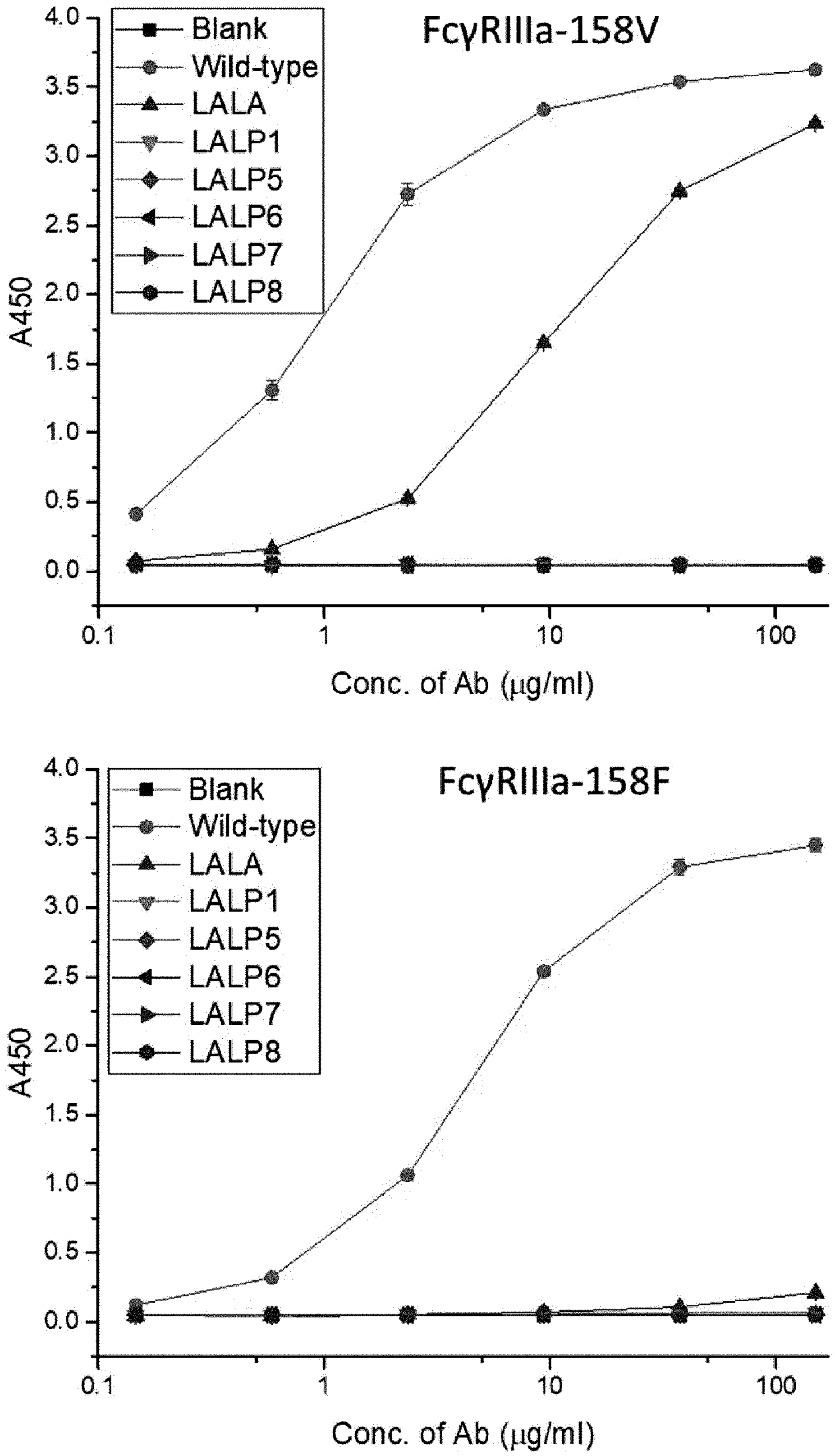
FcγRIIIa-158V
Blank
Wild-type
LALA
LALP1
LALP5
LALP6
LALP7
LALP8
A450
4.0
3.5
3.0
2.5
2.0
1.5
1.0
0.5
0.0
0.1
1
10
100
Conc. of Ab (μg/ml)
FcγRIIIa-158F
Blank
Wild-type
LALA
LALP1
LALP5
LALP6
LALP7
LALP8
A450
Conc. of Ab (μg/ml)

[FIG. 15]

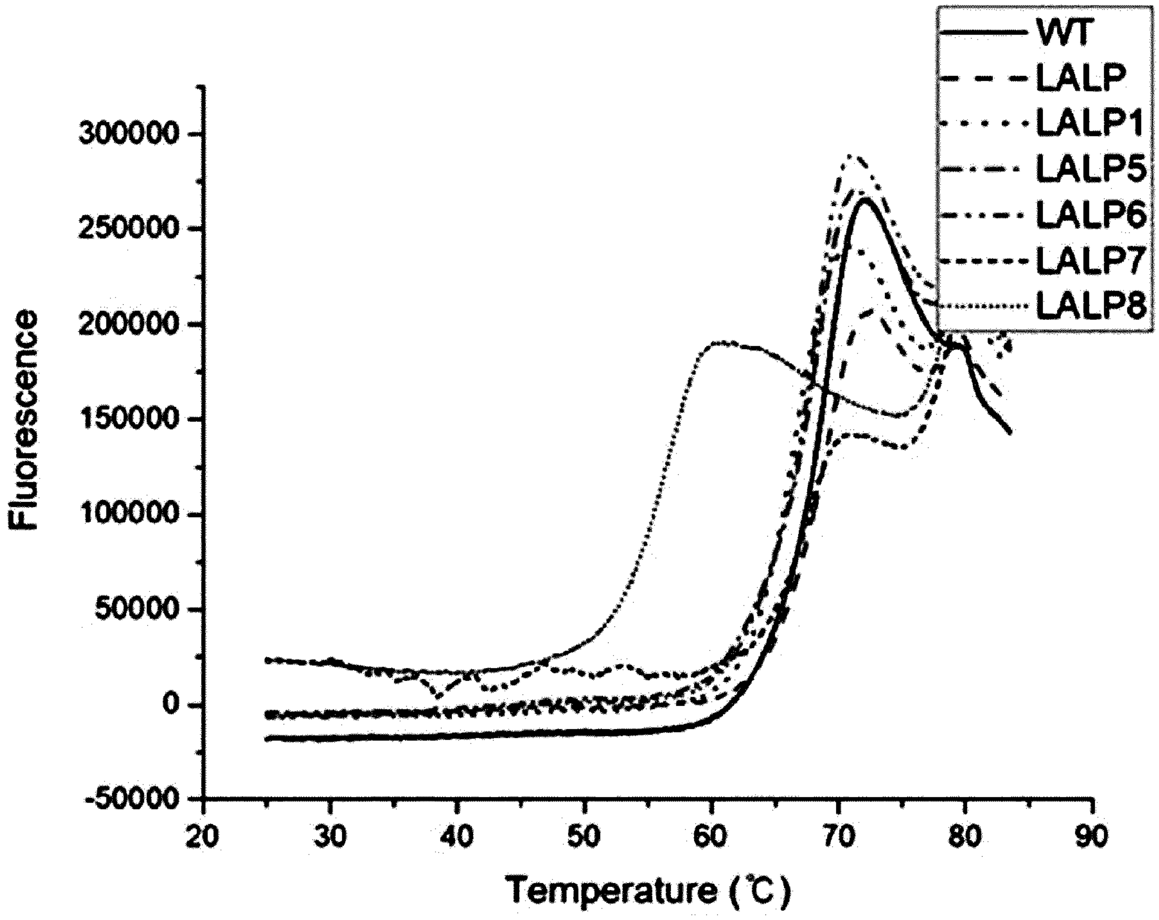


| Fc variants | Tm (℃) |
|---|---|
| Wild-type | 69.54494±0.106712 |
| LALP | 69.40118±0.376443 |
| LALP1 | 67.03947±0.361002 |
| LALP5 | 68.92884±0.222138 |
| LALP6 | 68.49757±0.320135 |
| LALP7 | 67.49127±0.585565 |
| LALP8 | 57.07921±0.617121 |

[FIG. 16]

| | Tm(°C) |
|---|---|
| WT | 68.97±0.06 |
| LP1(L235P/D265N) | 66.72±0.3 |
| LP5(L235P/P329V) | 68.07±0.07 |
| LP6(L235P/P329L) | 68.07±0.07 |
| LP7(L235P/D265M) | 67.15±0.26 |
| LP8(L235P/D265L/P331L) | 57.86±0.22 |

[FIG. 17]

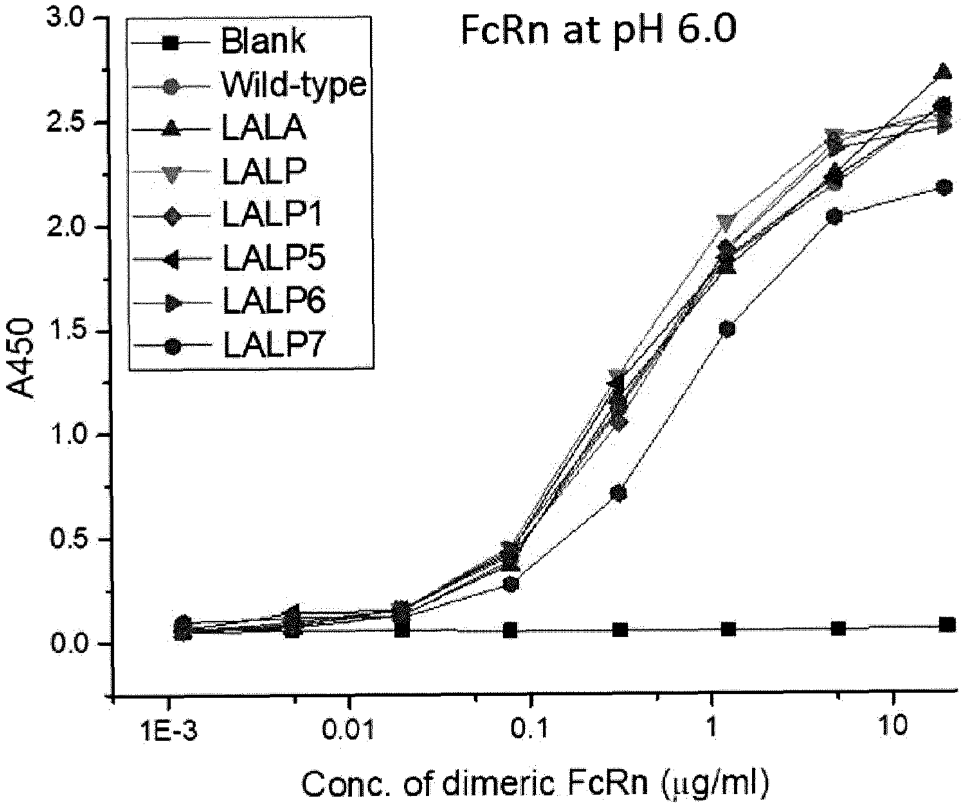
FcRn at pH 6.0
A450
3.0
2.5
2.0
1.5
1.0
0.5
0.0
Blank
Wild-type
LALA
LALP
LALP1
LALP5
LALP6
LALP7
1E-3
0.01
0.1
1
10
Conc. of dimeric FcRn (μg/ml)

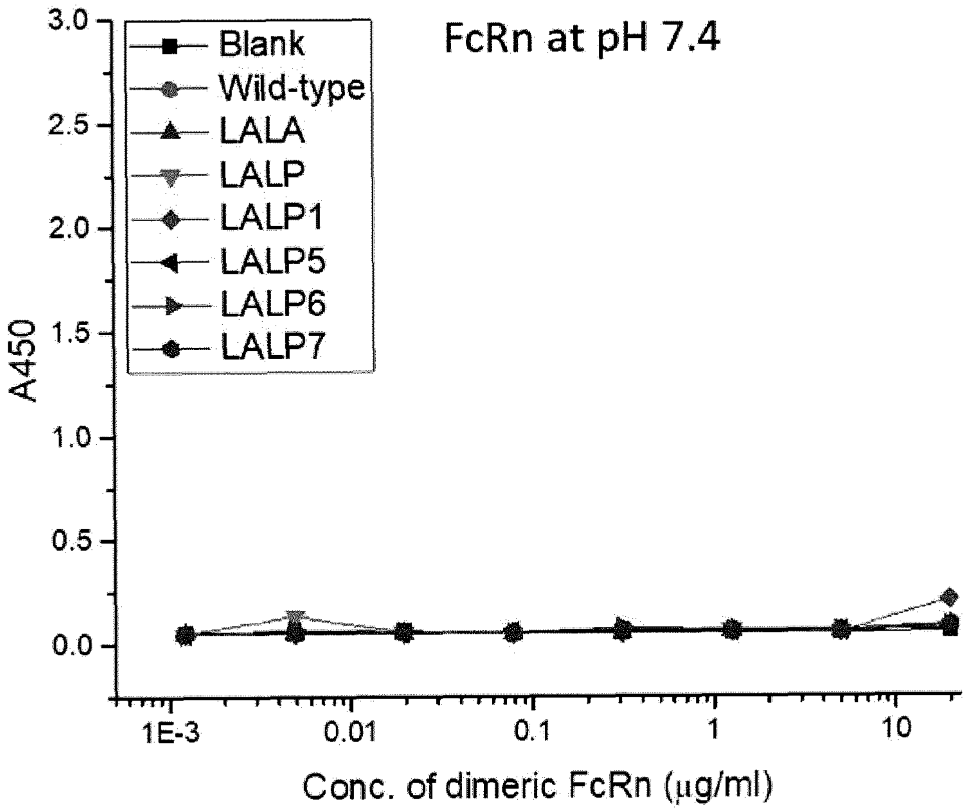
FcRn at pH 7.4
A450
3.0
2.5
2.0
1.5
1.0
0.5
0.0
Blank
Wild-type
LALA
LALP
LALP1
LALP5
LALP6
LALP7
1E-3
0.01
0.1
1
10
Conc. of dimeric FcRn (μg/ml)

[FIG. 18A]

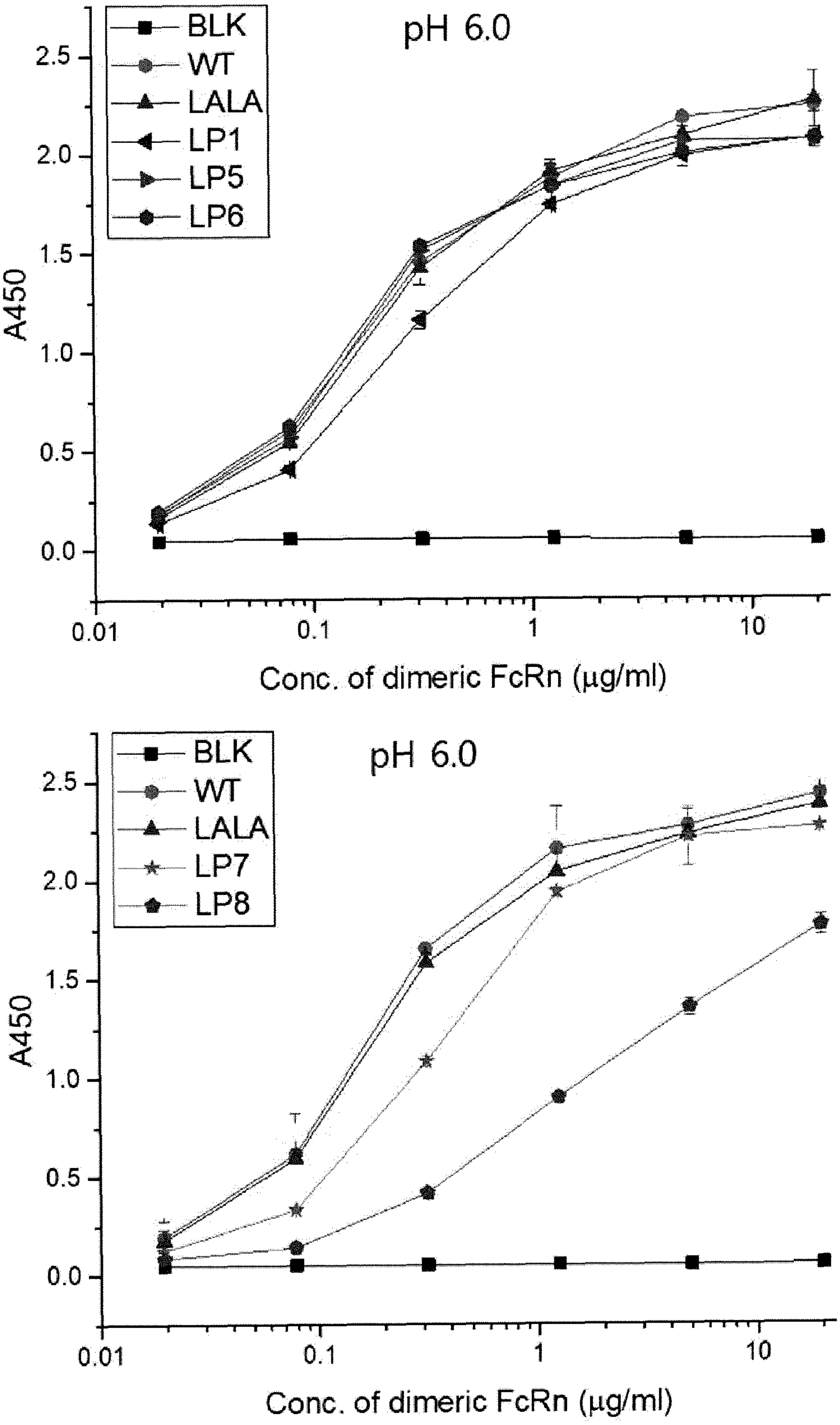
pH 6.0
BLK
WT
LALA
LP1
LP5
LP6
A450
Conc. of dimeric FcRn (μg/ml)
pH 6.0
BLK
WT
LALA
LP7
LP8
A450
Conc. of dimeric FcRn (μg/ml)

[FIG. 18B]

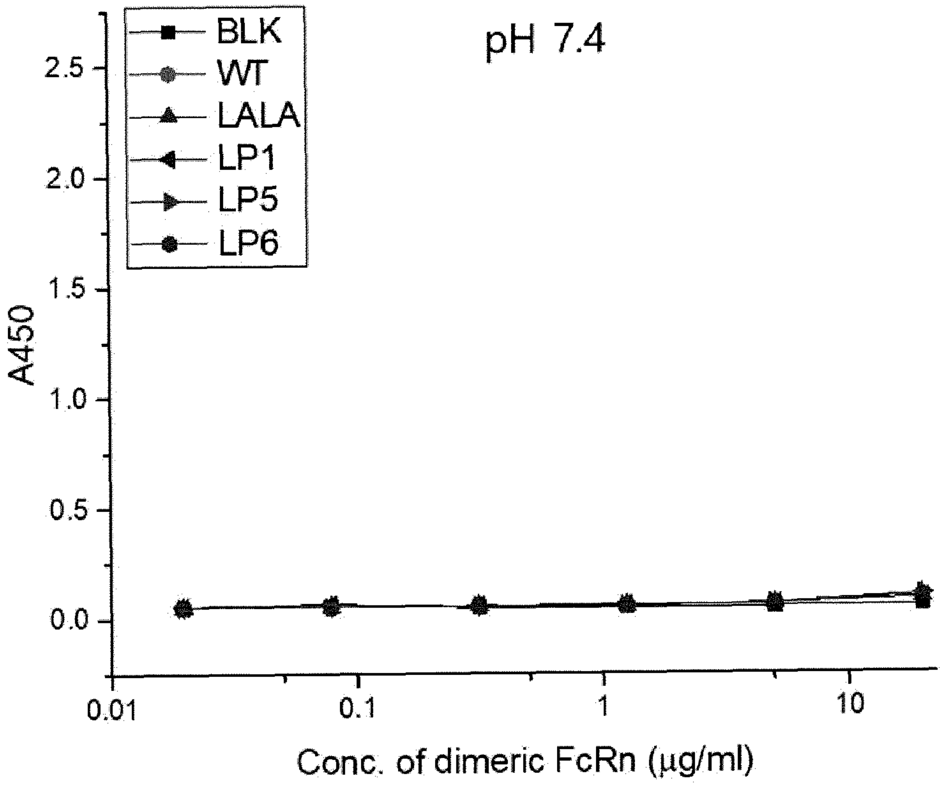
pH 7.4
BLK
WT
LALA
LP1
LP5
LP6
A450
2.5
2.0
1.5
1.0
0.5
0.0
0.01
0.1
1
10
Conc. of dimeric FcRn (μg/ml)

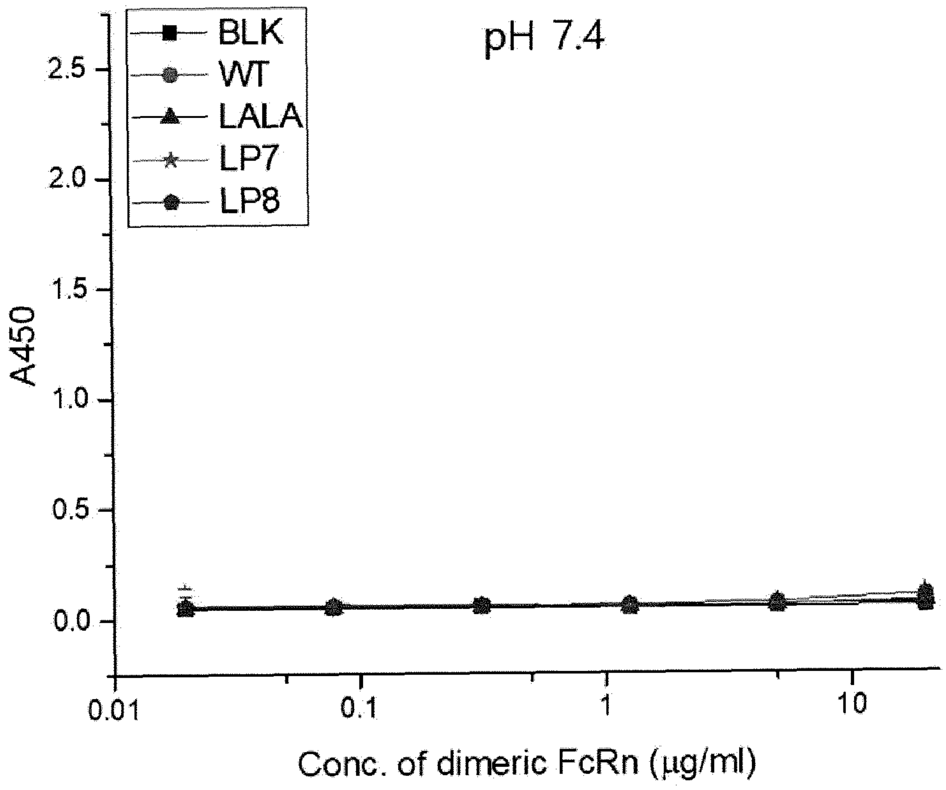
pH 7.4
BLK
WT
LALA
LP7
LP8
A450
2.5
2.0
1.5
1.0
0.5
0.0
0.01
0.1
1
10
Conc. of dimeric FcRn (μg/ml)

[FIG. 19]

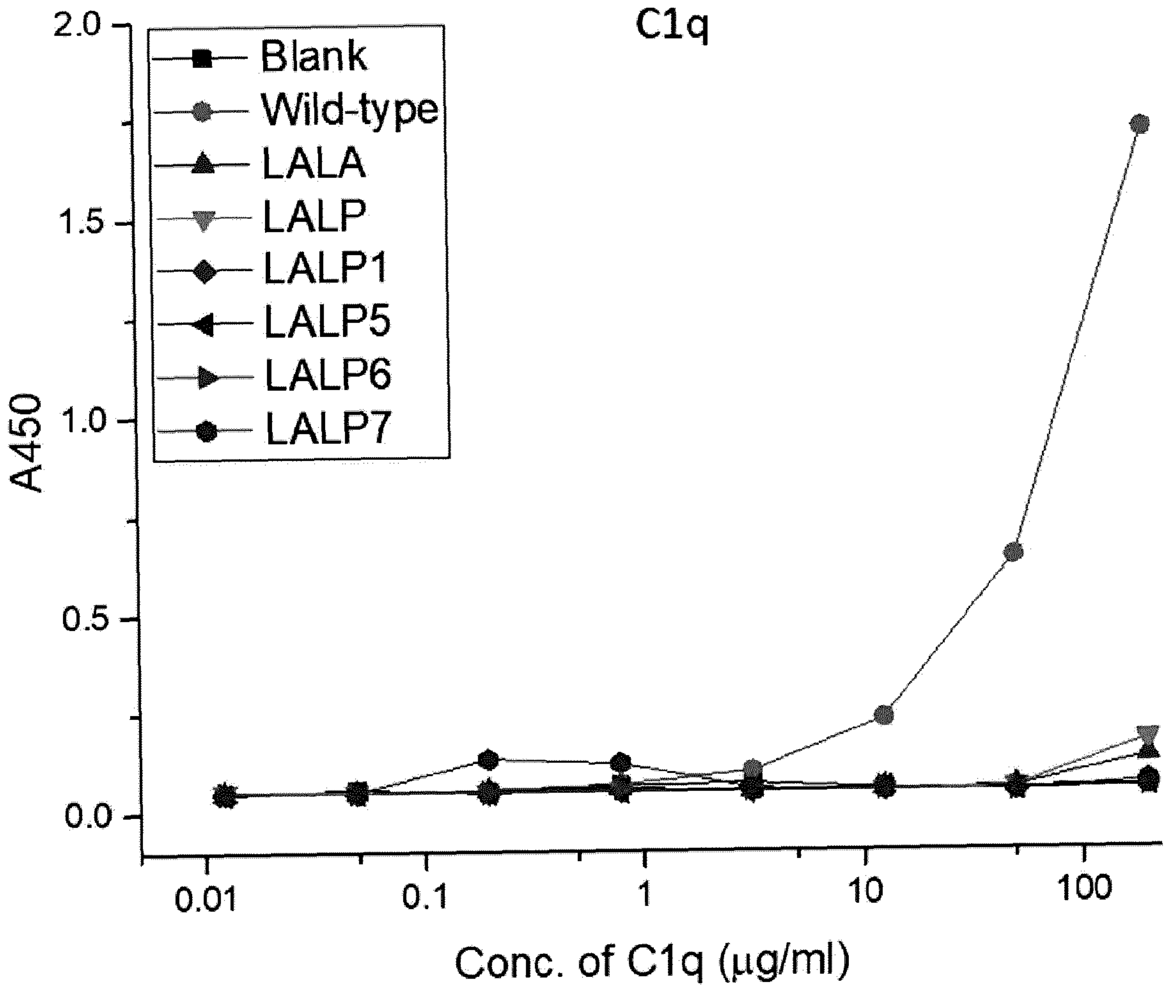
C1q
Blank
Wild-type
LALA
LALP
LALP1
LALP5
LALP6
LALP7
A450
2.0
1.5
1.0
0.5
0.0
0.01
0.1
1
10
100
Conc. of C1q (μg/ml)

[FIG. 20]

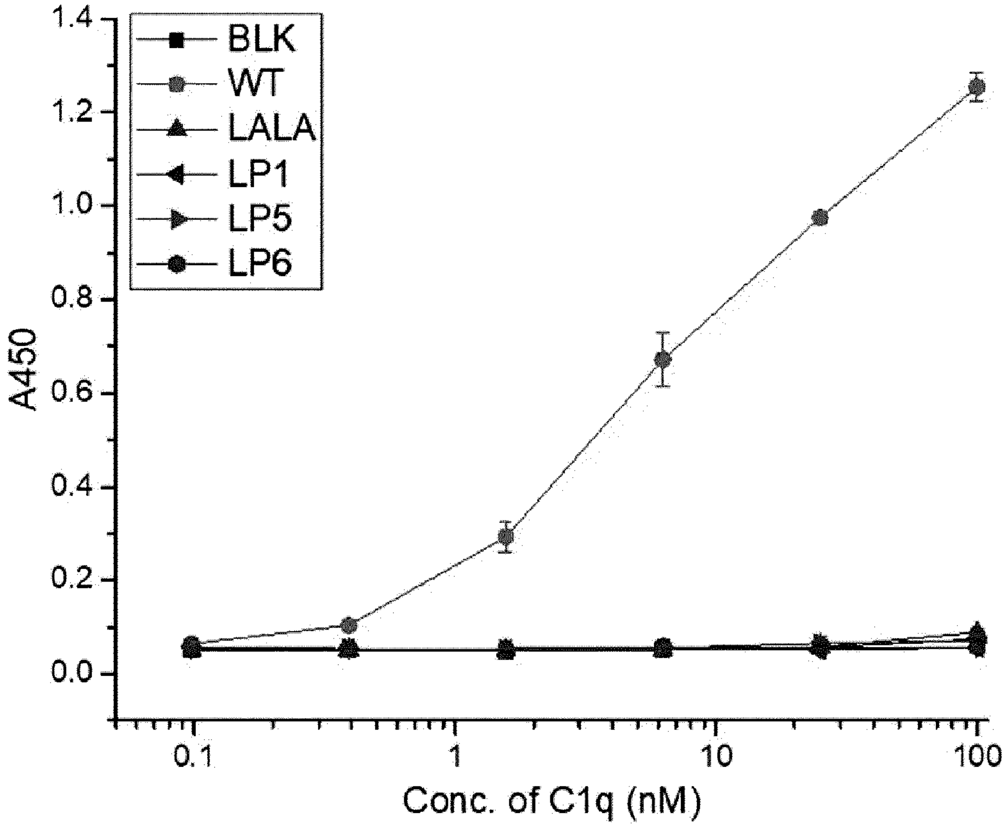
1.4
1.2
1.0
0.8
0.6
0.4
0.2
0.0
A450
BLK
WT
LALA
LP1
LP5
LP6
0.1
1
10
100
Conc. of C1q (nM)

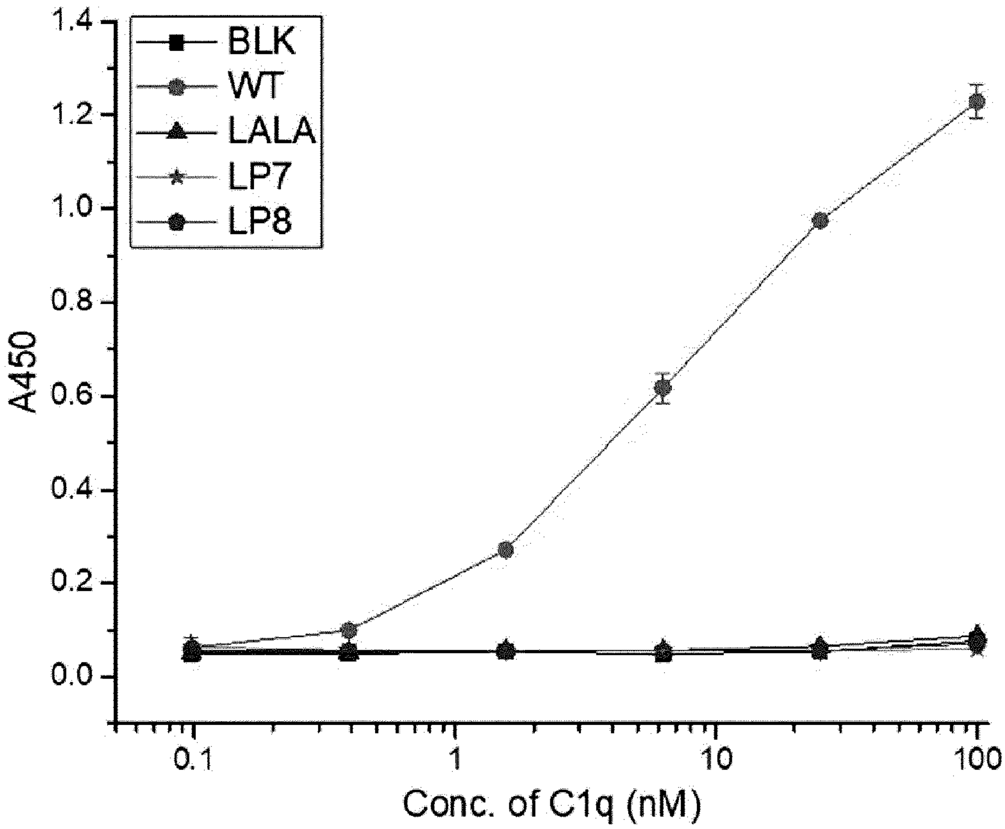
1.4
1.2
1.0
0.8
0.6
0.4
0.2
0.0
A450
BLK
WT
LALA
LP7
LP8
0.1
1
10
100
Conc. of C1q (nM)

[FIG. 21A]

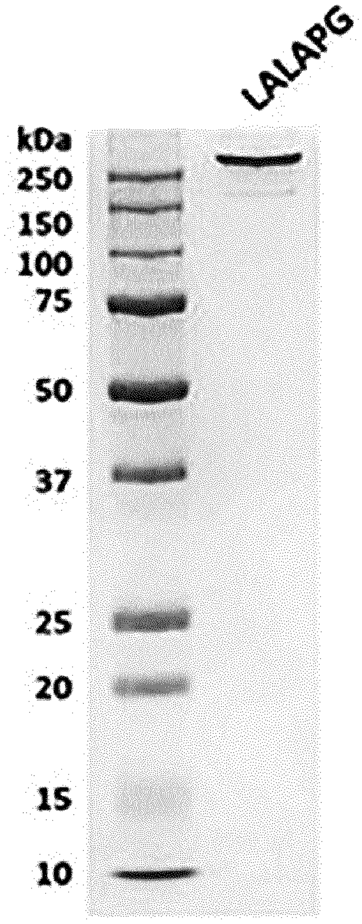


| Fc variants | Mutations |
|---|---|
| LALAPG | L234A/L235A/P329G |

[FIG. 21B]

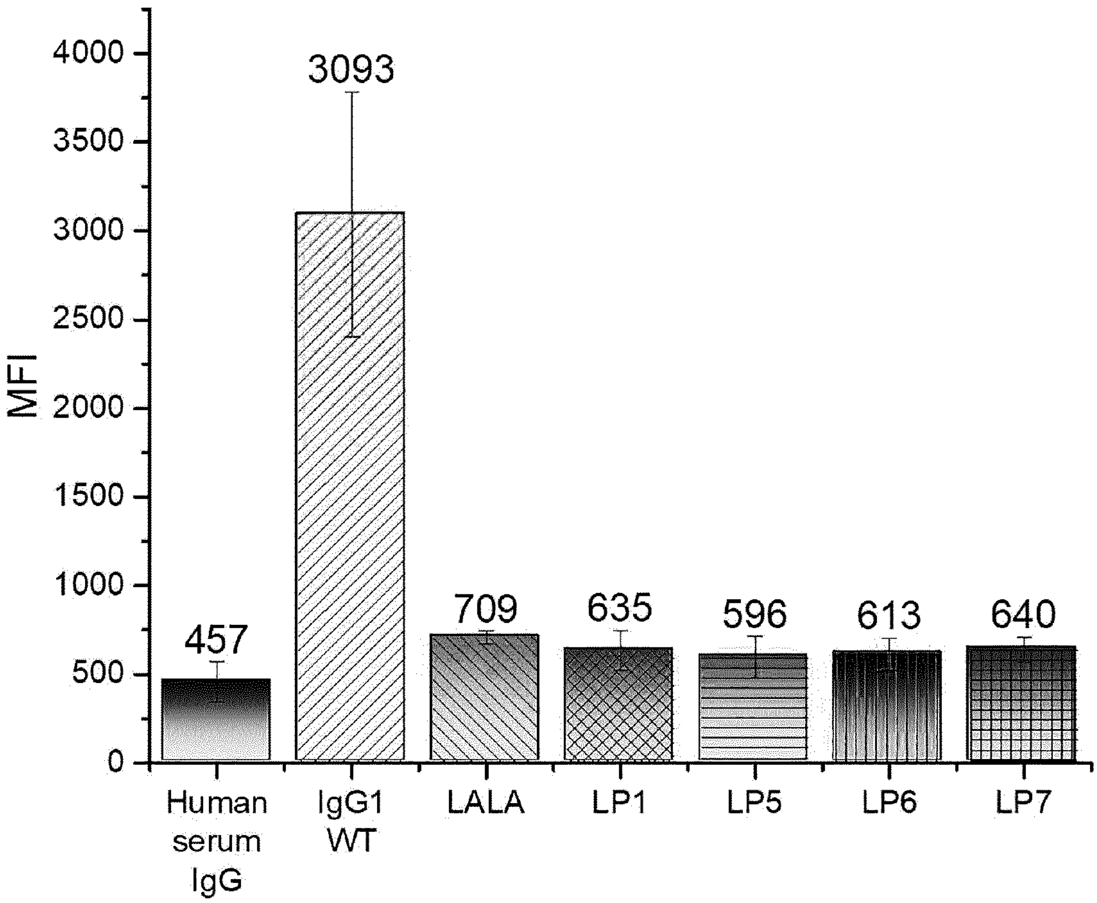
MFI
4000
3500
3000
2500
2000
1500
1000
500
0
457
3093
709
635
596
613
640
Human serum IgG
IgG1 WT
LALA
LP1
LP5
LP6
LP7

[FIG. 22]

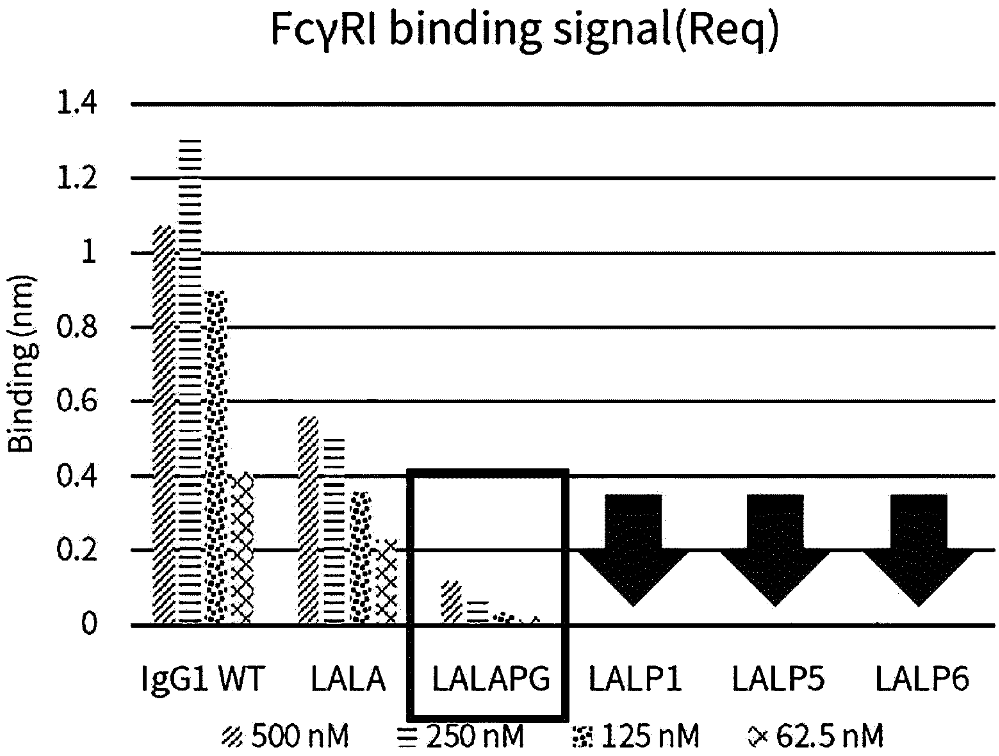
FcγRI binding signal(Req)
1.4
1.2
1
0.8
0.6
0.4
0.2
0
Binding (nm)
IgG1 WT
LALA
LALAPG
LALP1
LALP5
LALP6
500 nM
250 nM
125 nM
62.5 nM

[FIG. 23]

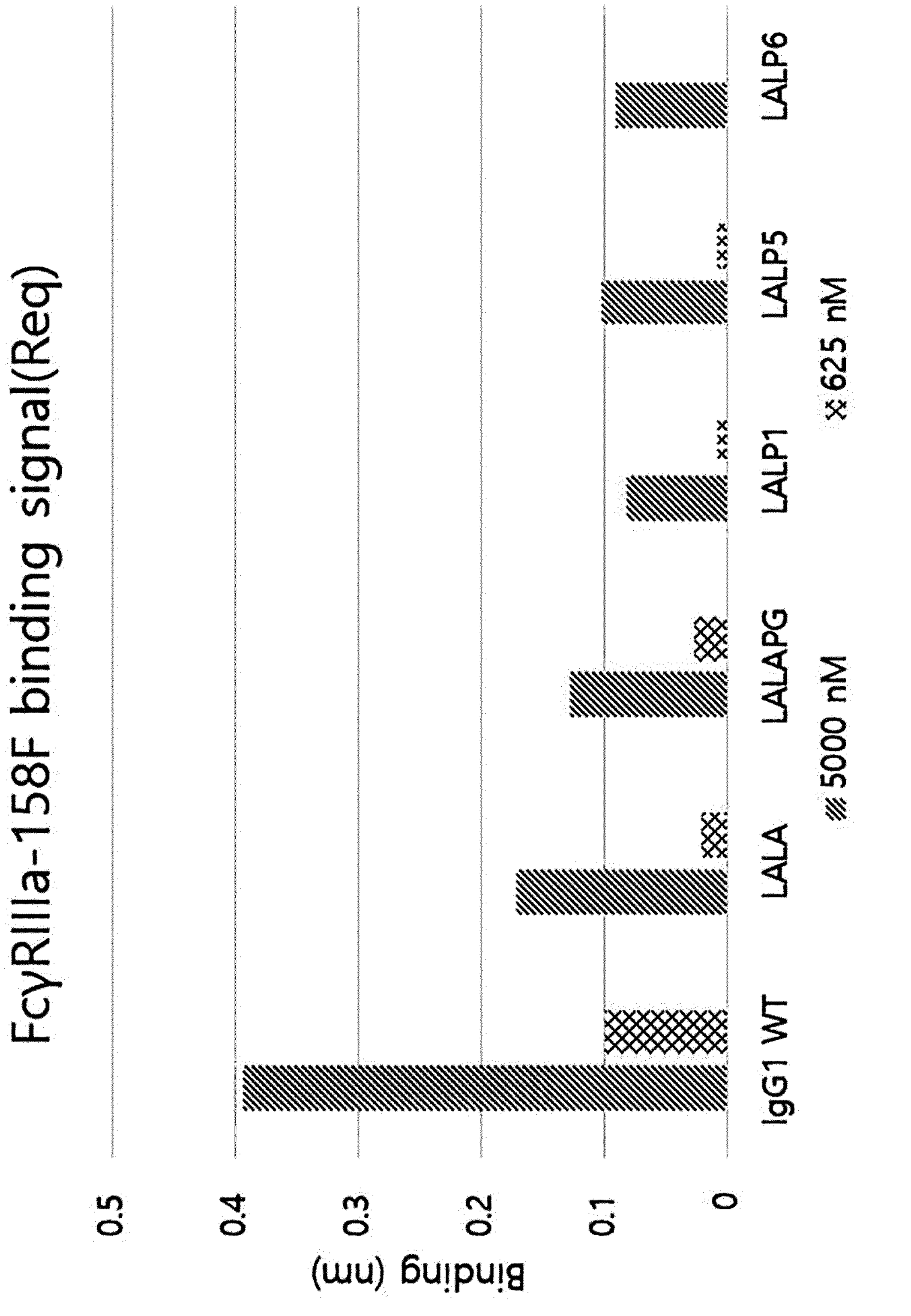
FcγRIIIa-158F binding signal(Req)
Binding (nm)
0
0.1
0.2
0.3
0.4
0.5
IgG1 WT
LALA
LALAPG
LALP1
LALP5
LALP6
5000 nM
625 nM

[FIG. 24]

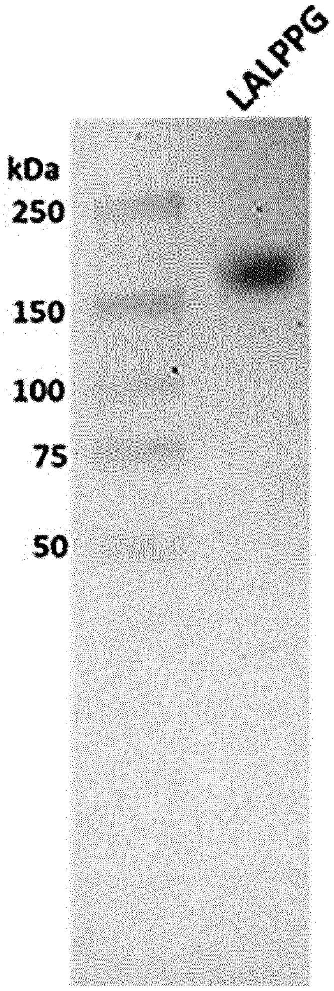


| Fc variants | Mutations |
|---|---|
| LALPPG | L234A/L235P/P329G |

[FIG. 25A]

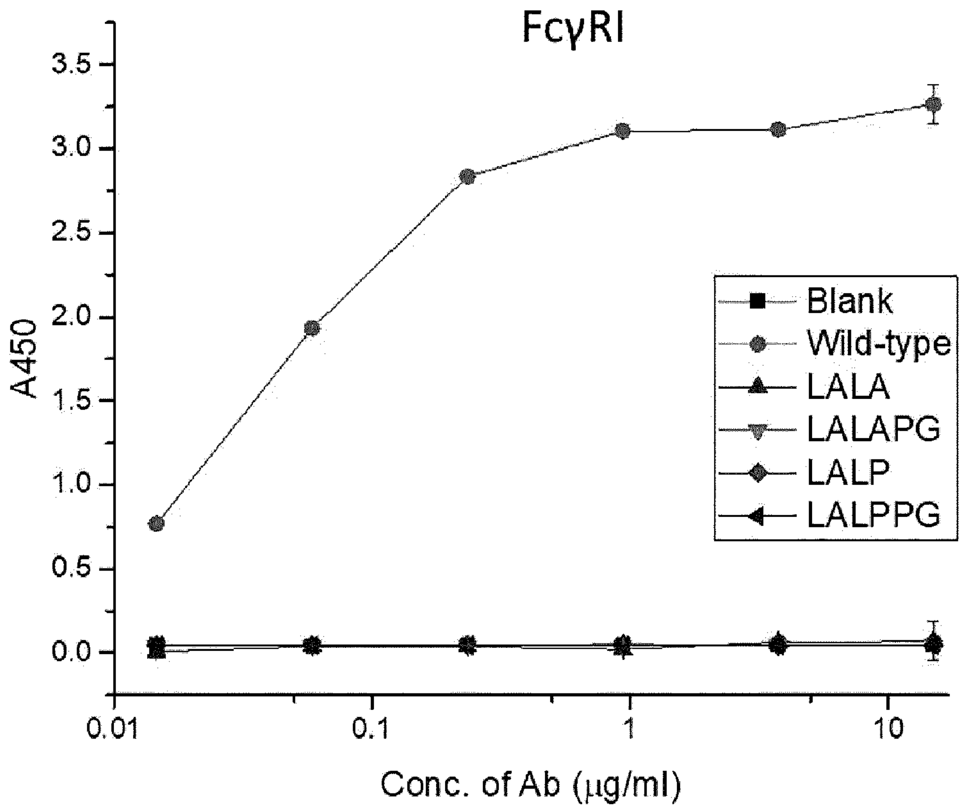
FcγRI
A450
3.5
3.0
2.5
2.0
1.5
1.0
0.5
0.0
0.01
0.1
1
10
Conc. of Ab (μg/ml)
Blank
Wild-type
LALA
LALAPG
LALP
LALPPG

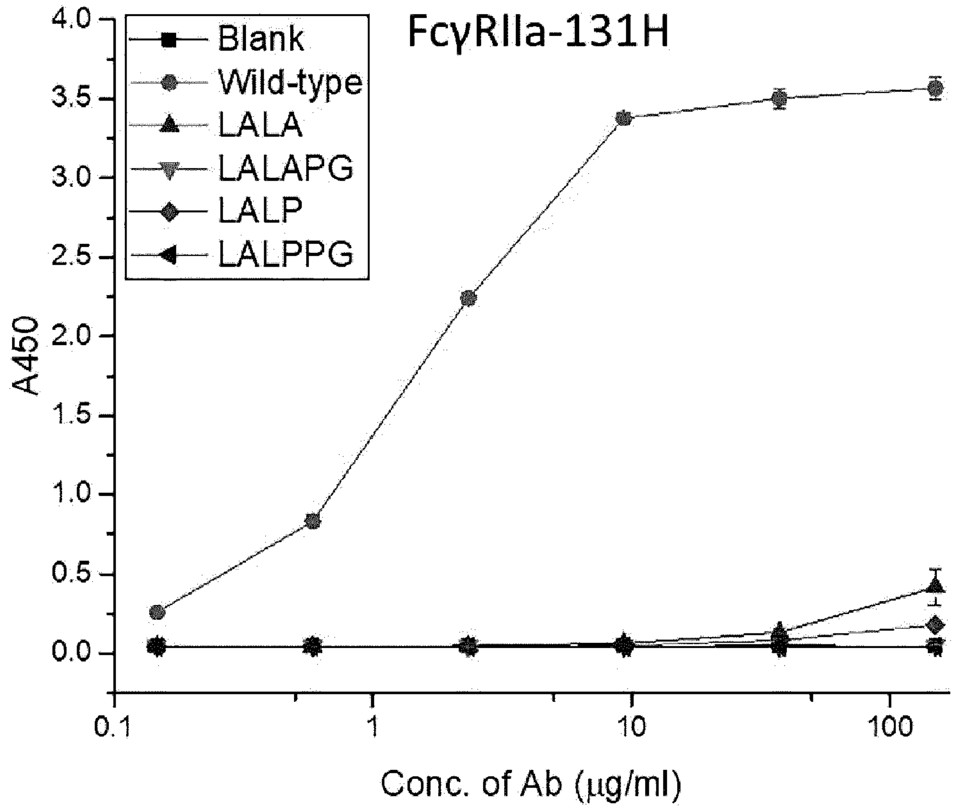
FcγRIIa-131H
A450
4.0
3.5
3.0
2.5
2.0
1.5
1.0
0.5
0.0
0.1
1
10
100
Conc. of Ab (μg/ml)
Blank
Wild-type
LALA
LALAPG
LALP
LALPPG

[FIG. 25B]

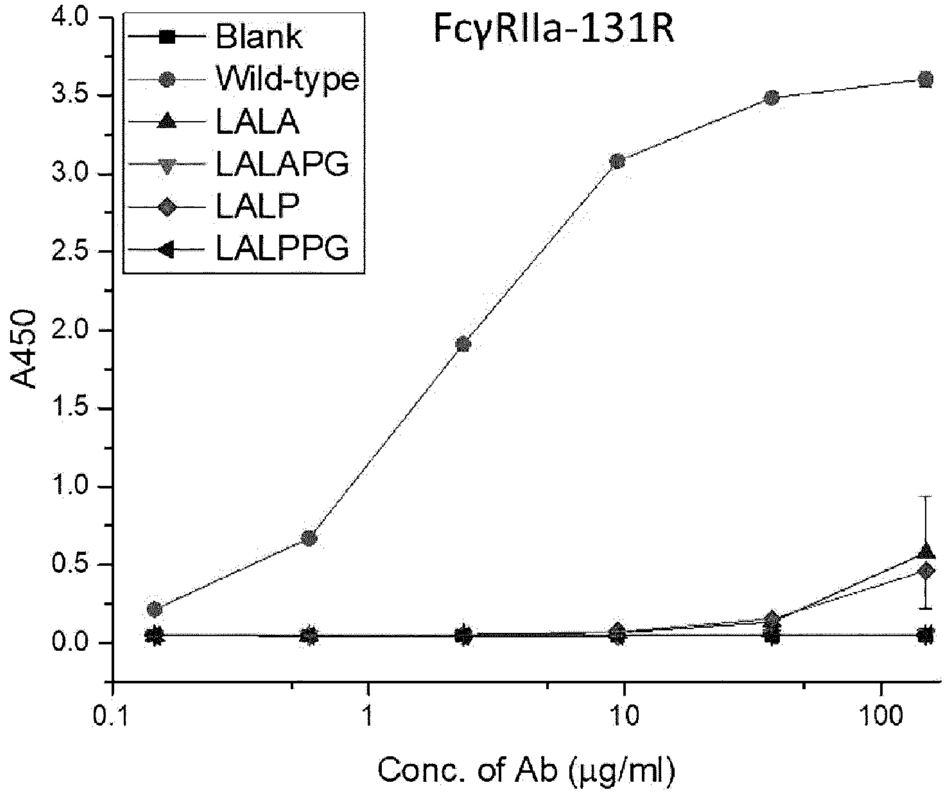
FcγRIIa-131R
Blank
Wild-type
LALA
LALAPG
LALP
LALPPG
A450
4.0
3.5
3.0
2.5
2.0
1.5
1.0
0.5
0.0
0.1
1
10
100
Conc. of Ab (µg/ml)

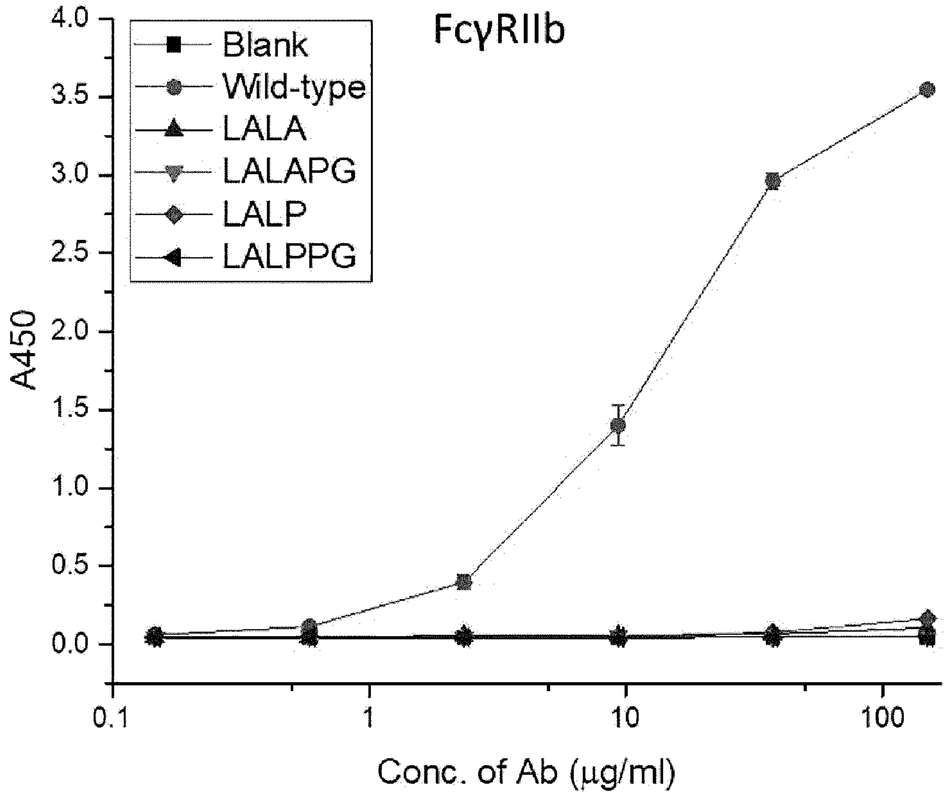
FcγRIIb
Blank
Wild-type
LALA
LALAPG
LALP
LALPPG
A450
4.0
3.5
3.0
2.5
2.0
1.5
1.0
0.5
0.0
0.1
1
10
100
Conc. of Ab (µg/ml)

[FIG. 25C]

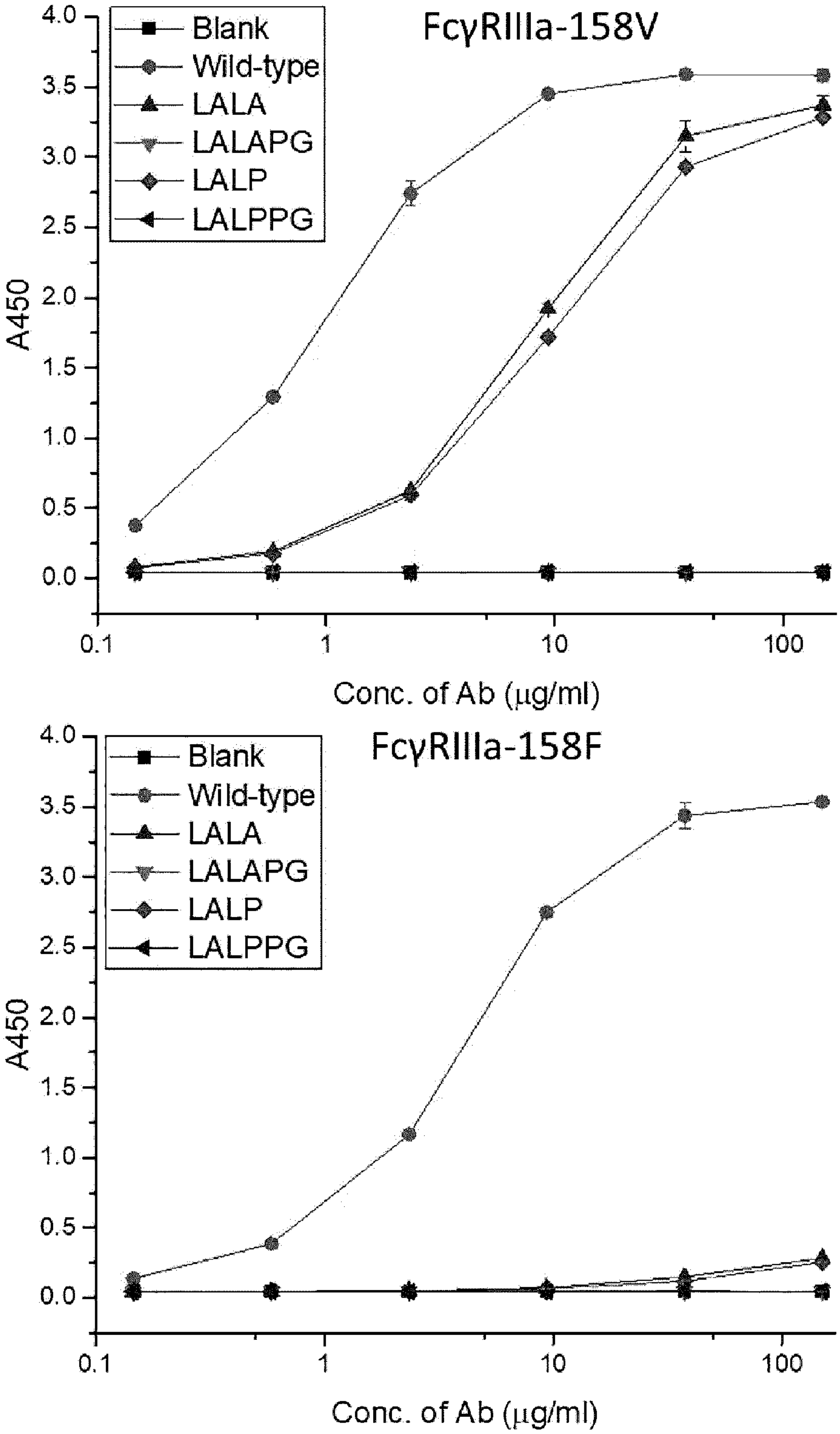
FcγRIIIa-158V
Blank
Wild-type
LALA
LALAPG
LALP
LALPPG
A450
4.0
3.5
3.0
2.5
2.0
1.5
1.0
0.5
0.0
0.1
1
10
100
Conc. of Ab (μg/ml)
FcγRIIIa-158F
Blank
Wild-type
LALA
LALAPG
LALP
LALPPG
A450
4.0
3.5
3.0
2.5
2.0
1.5
1.0
0.5
0.0
0.1
1
10
100
Conc. of Ab (μg/ml)

[FIG. 26]

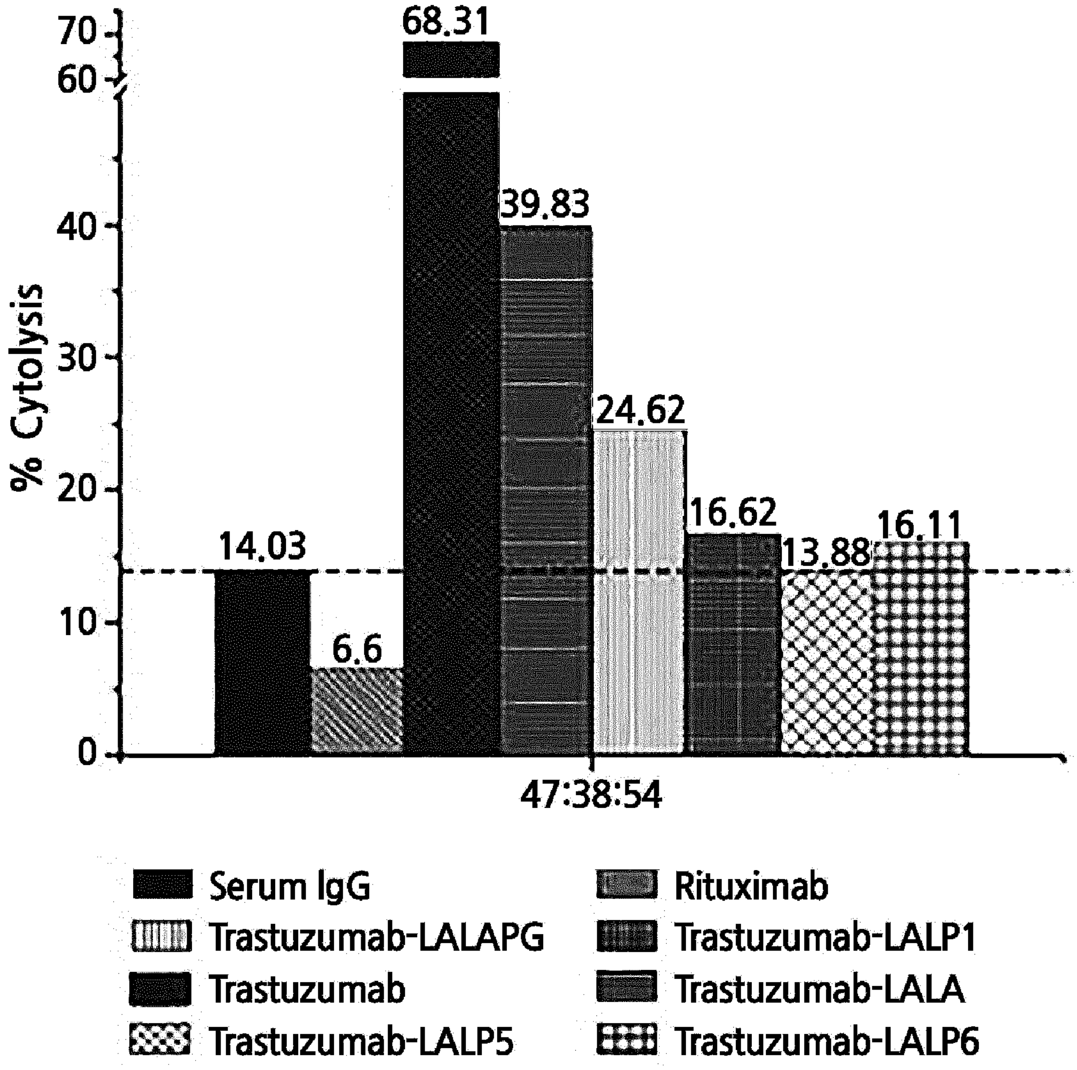
% Cytolysis
70
60
40
30
20
10
0
68.31
39.83
24.62
16.62
16.11
14.03
13.88
6.6
47:38:54
Serum IgG
Rituximab
Trastuzumab-LALAPG
Trastuzumab-LALP1
Trastuzumab
Trastuzumab-LALA
Trastuzumab-LALP5
Trastuzumab-LALP6

[FIG. 27A]

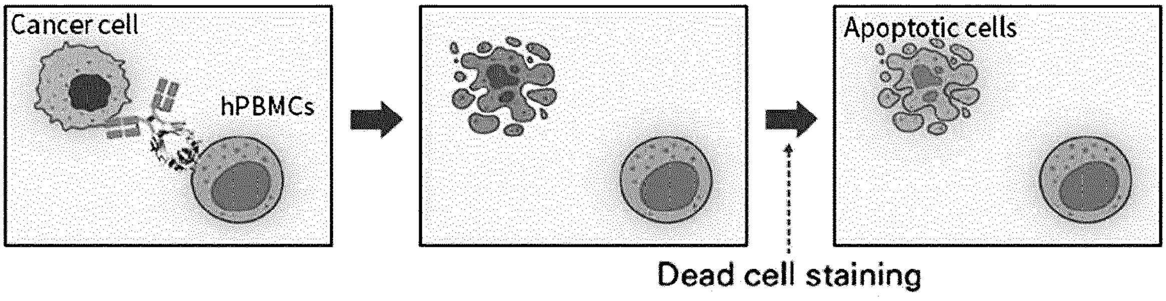


[FIG. 27B]

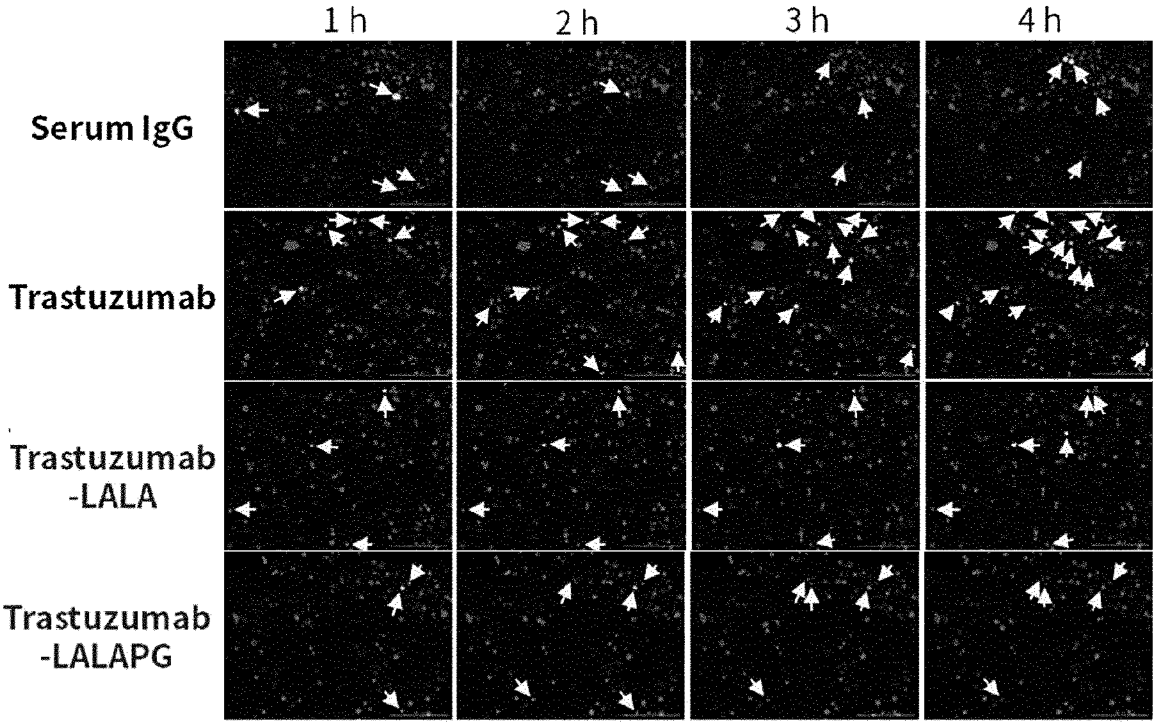

[FIG. 27C]

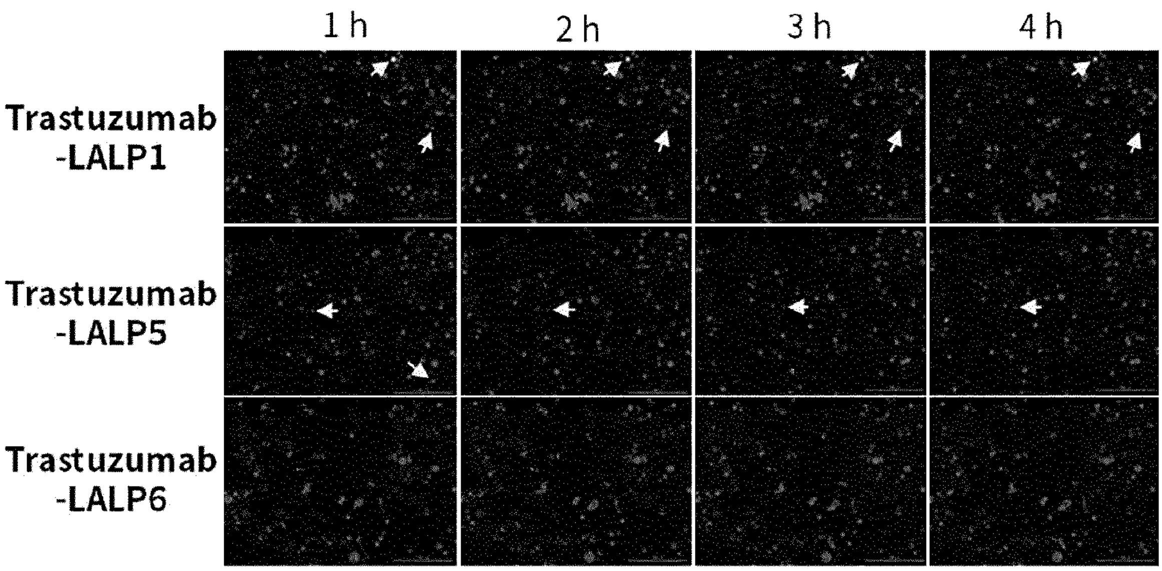
1 h
2 h
3 h
4 h
Trastuzumab -LALP1
Trastuzumab -LALP5
Trastuzumab -LALP6

[FIG. 27D]

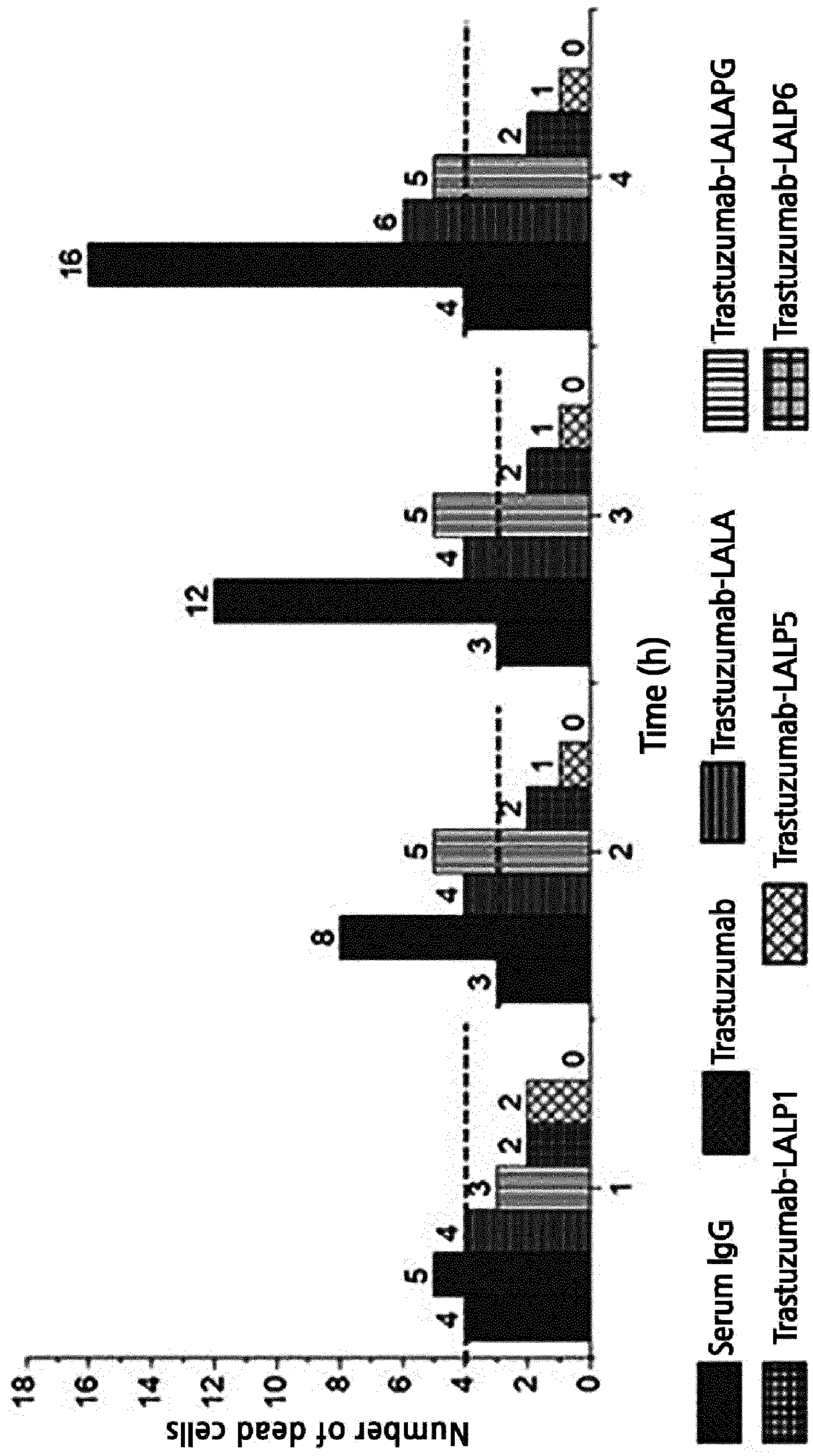
Number of dead cells
Time (h)
Serum IgG
Trastuzumab
Trastuzumab-LALA
Trastuzumab-LALAPG
Trastuzumab-LALP1
Trastuzumab-LALP5
Trastuzumab-LALP6

[FIG. 28]

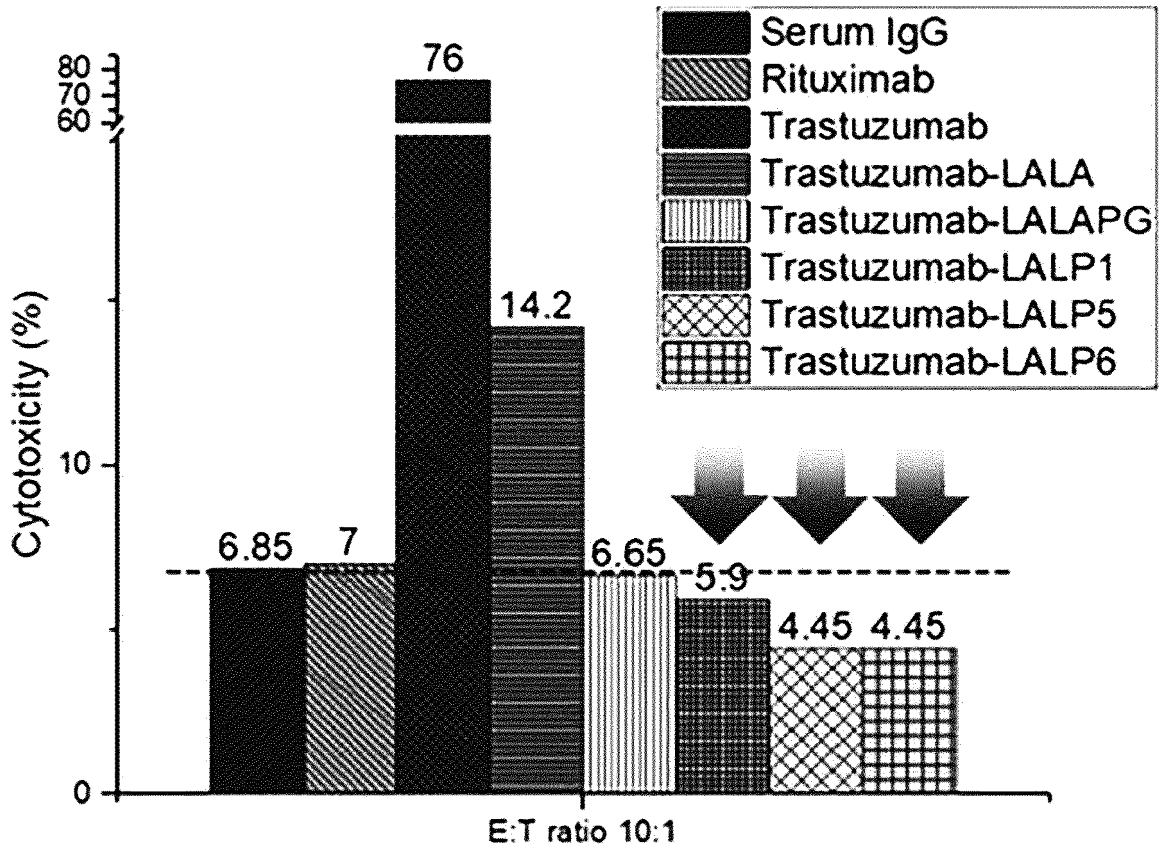
Serum IgG
Rituximab
Trastuzumab
Trastuzumab-LALA
Trastuzumab-LALAPG
Trastuzumab-LALP1
Trastuzumab-LALP5
Trastuzumab-LALP6
Cytotoxicity (%)
80
70
60
10
0
76
14.2
6.85
7
6.65
5.9
4.45
4.45
E:T ratio 10:1

[FIG. 29]

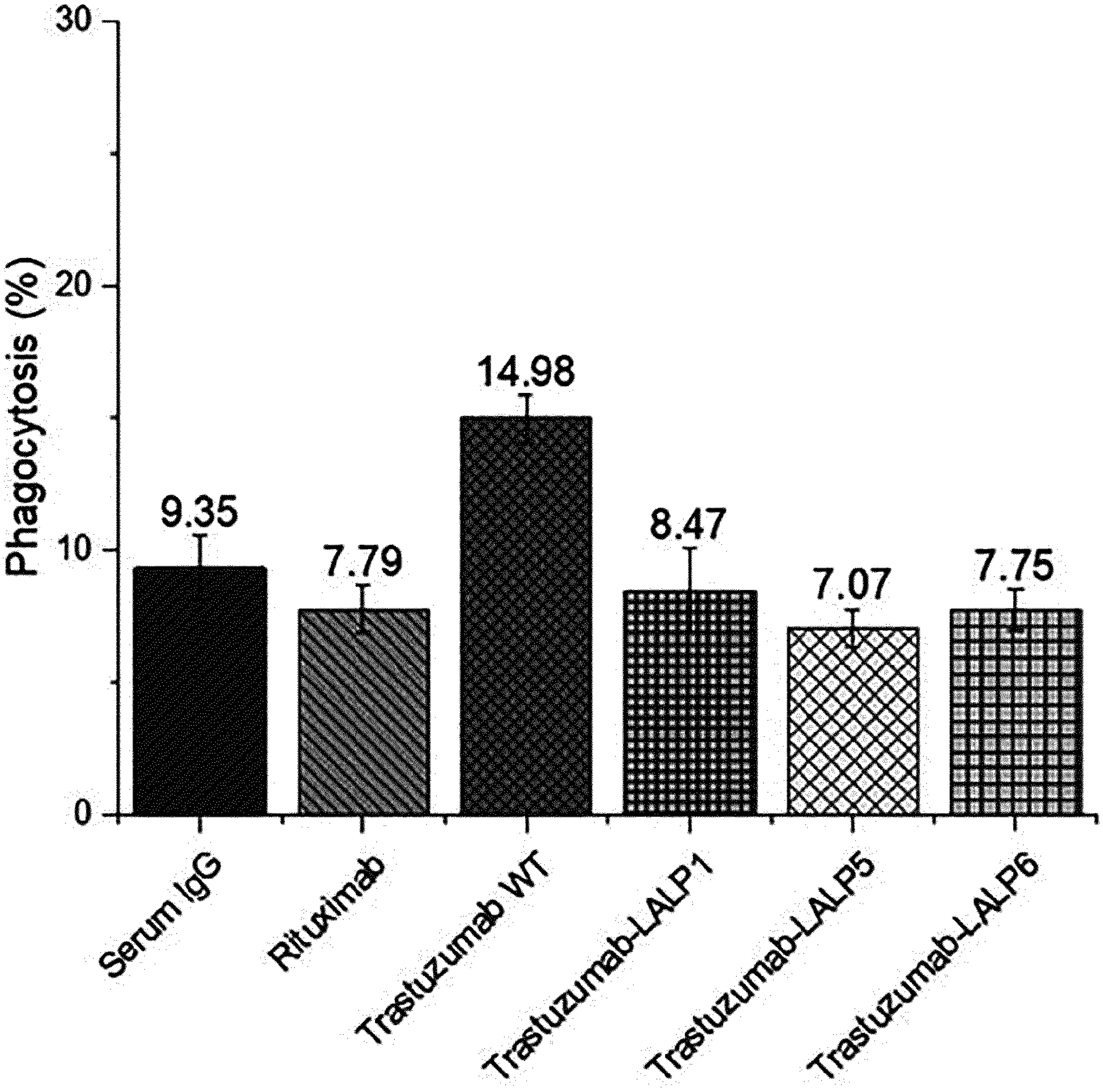
30
20
10
0
Phagocytosis (%)
9.35
7.79
14.98
8.47
7.07
7.75
Serum IgG
Rituximab
Trastuzumab WT
Trastuzumab-LALP1
Trastuzumab-LALP5
Trastuzumab-LALP6

[FIG. 30]

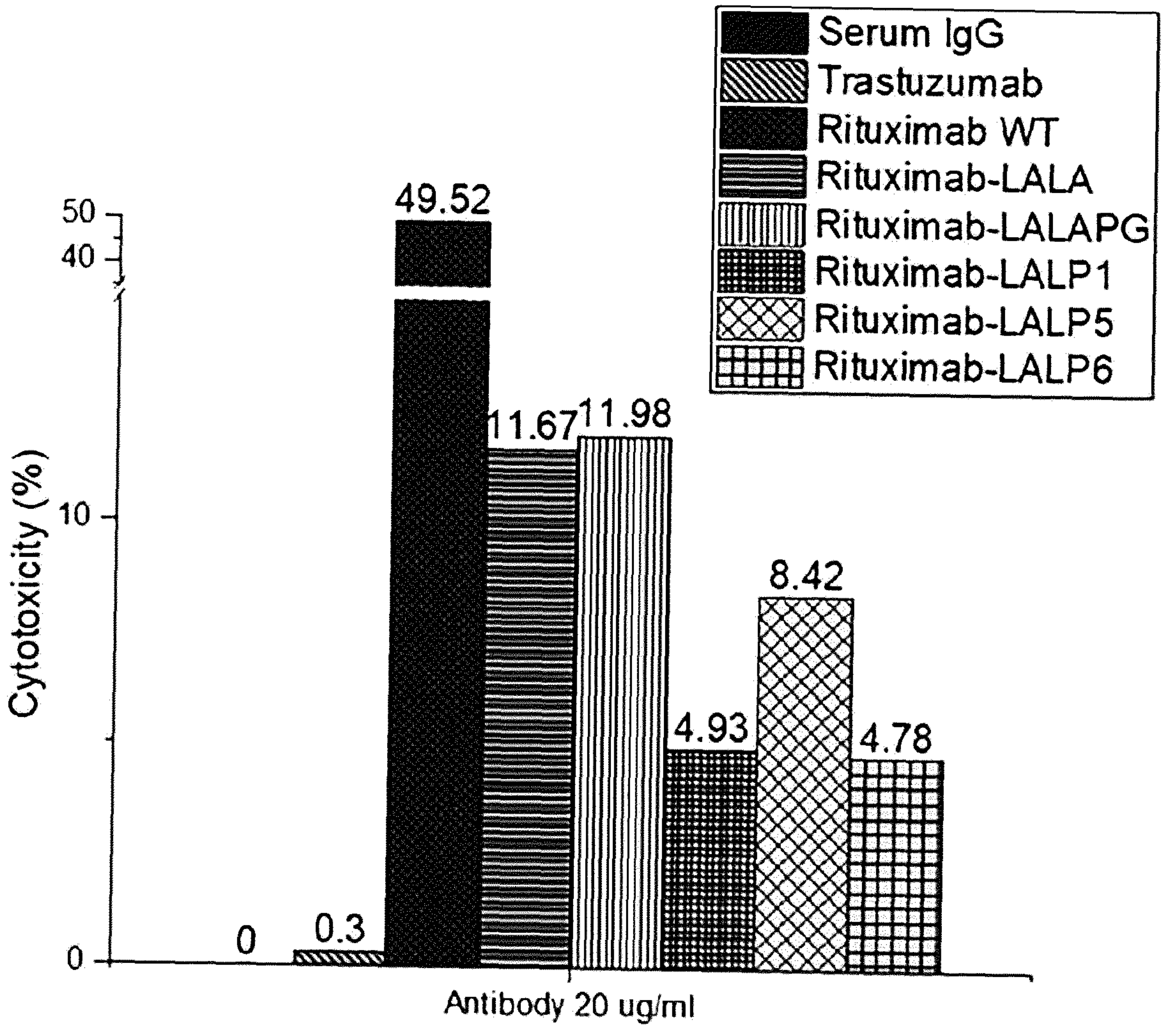
Serum IgG
Trastuzumab
Rituximab WT
Rituximab-LALA
Rituximab-LALAPG
Rituximab-LALP1
Rituximab-LALP5
Rituximab-LALP6
Cytotoxicity (%)
50
40
10
0
0
0.3
49.52
11.67
11.98
4.93
8.42
4.78
Antibody 20 ug/ml

[FIG. 31A]

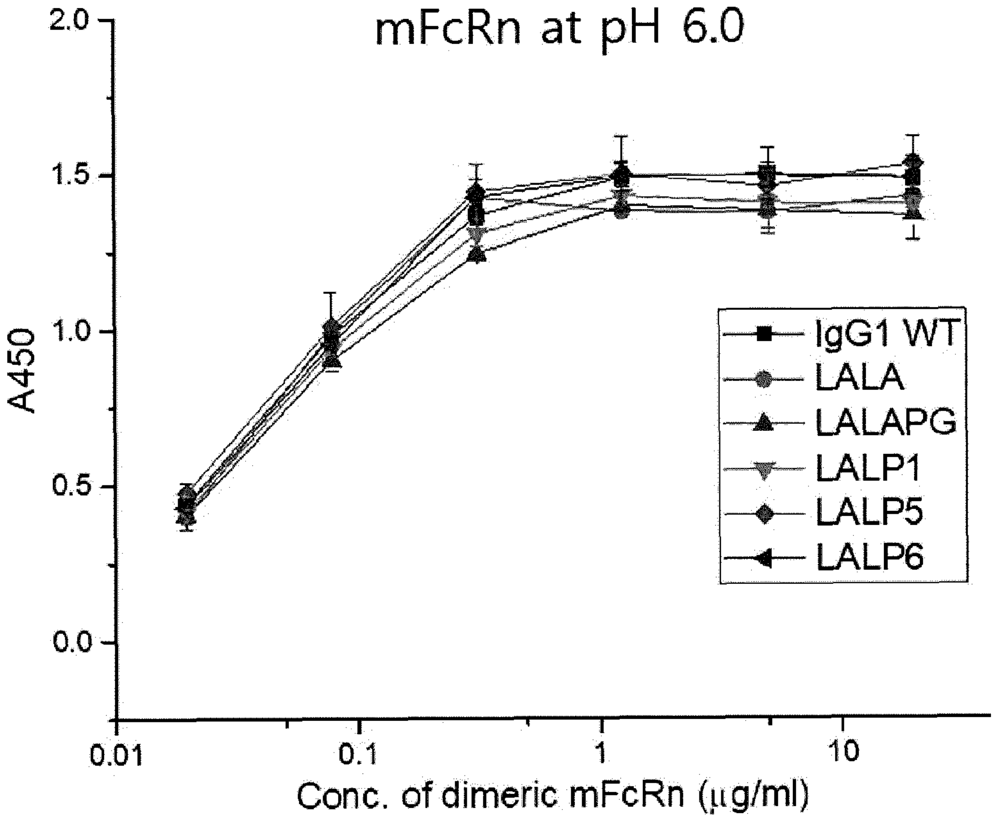
mFcRn at pH 6.0
A450
2.0
1.5
1.0
0.5
0.0
0.01
0.1
1
10
Conc. of dimeric mFcRn (μg/ml)
IgG1 WT
LALA
LALAPG
LALP1
LALP5
LALP6

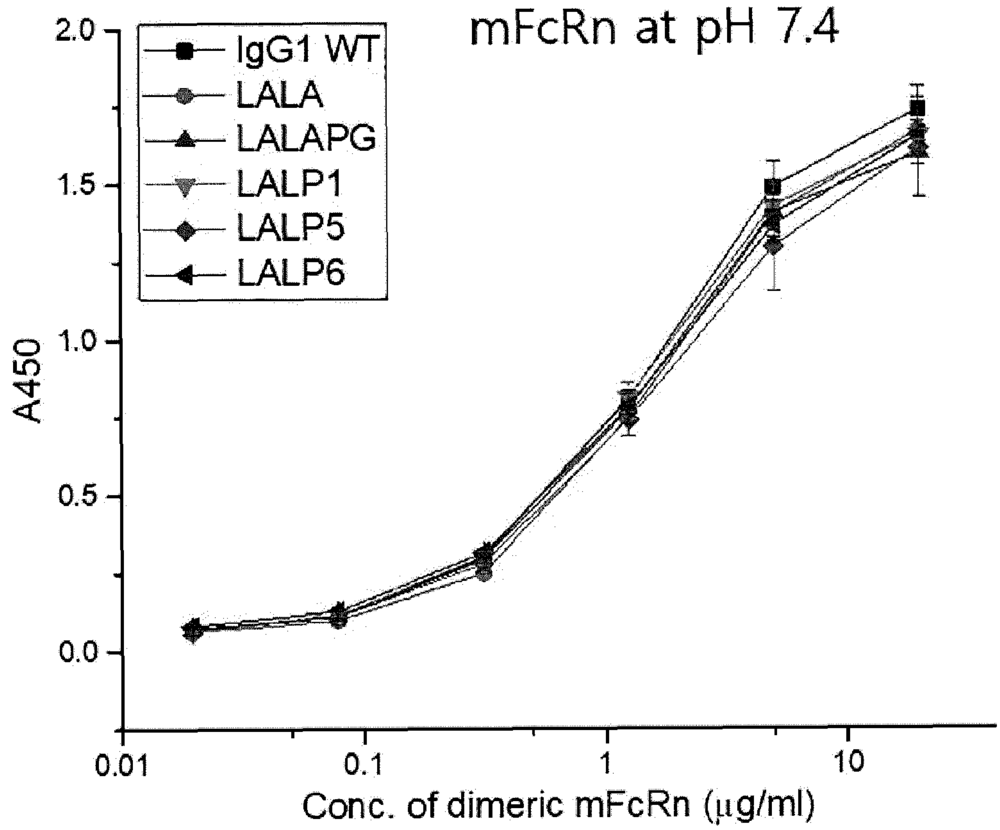
mFcRn at pH 7.4
A450
2.0
1.5
1.0
0.5
0.0
0.01
0.1
1
10
Conc. of dimeric mFcRn (μg/ml)
IgG1 WT
LALA
LALAPG
LALP1
LALP5
LALP6

[FIG. 31B]

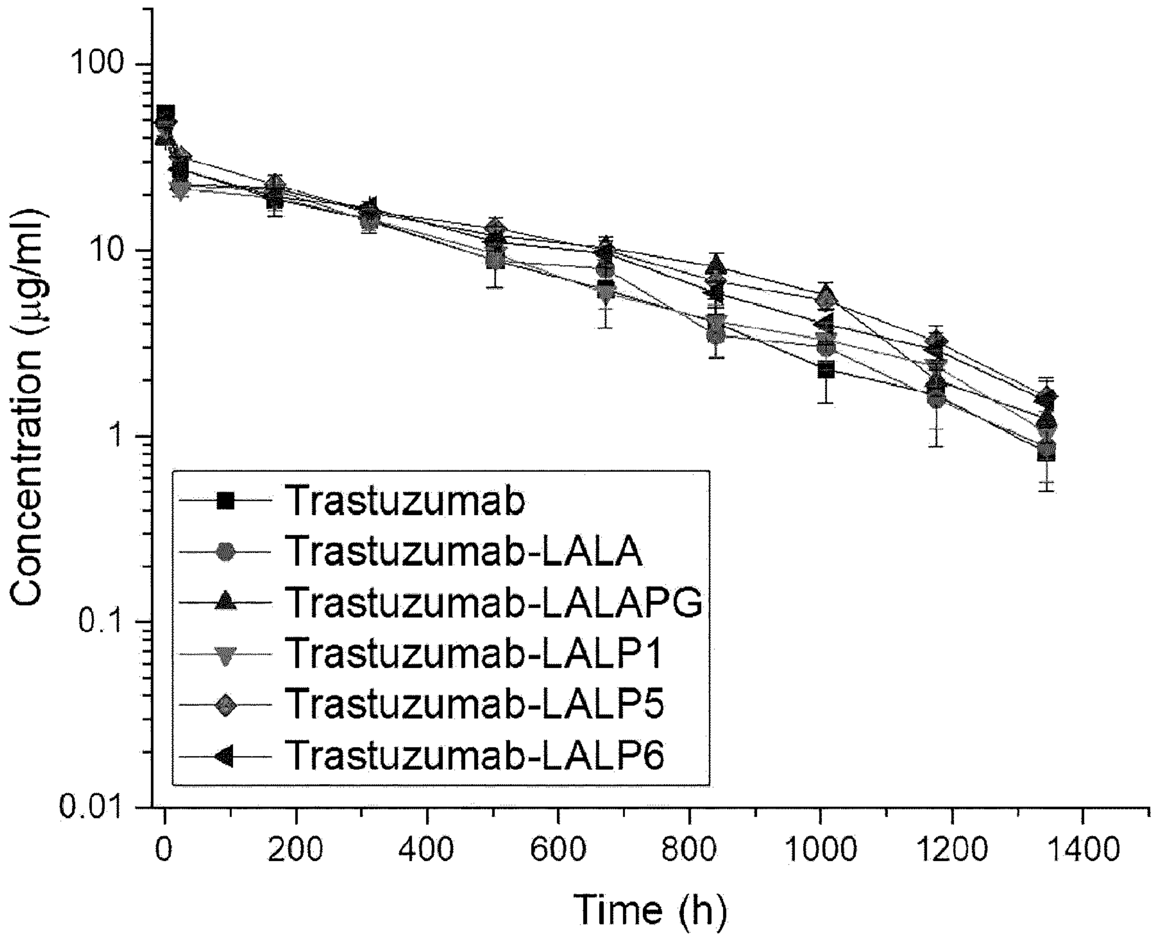

100
10
1
0.1
0.01
Concentration (μg/ml)
0
200
400
600
800
1000
1200
1400
Time (h)
Trastuzumab
Trastuzumab-LALA
Trastuzumab-LALAPG
Trastuzumab-LALP1
Trastuzumab-LALP5
Trastuzumab-LALP6

[FIG. 32A]

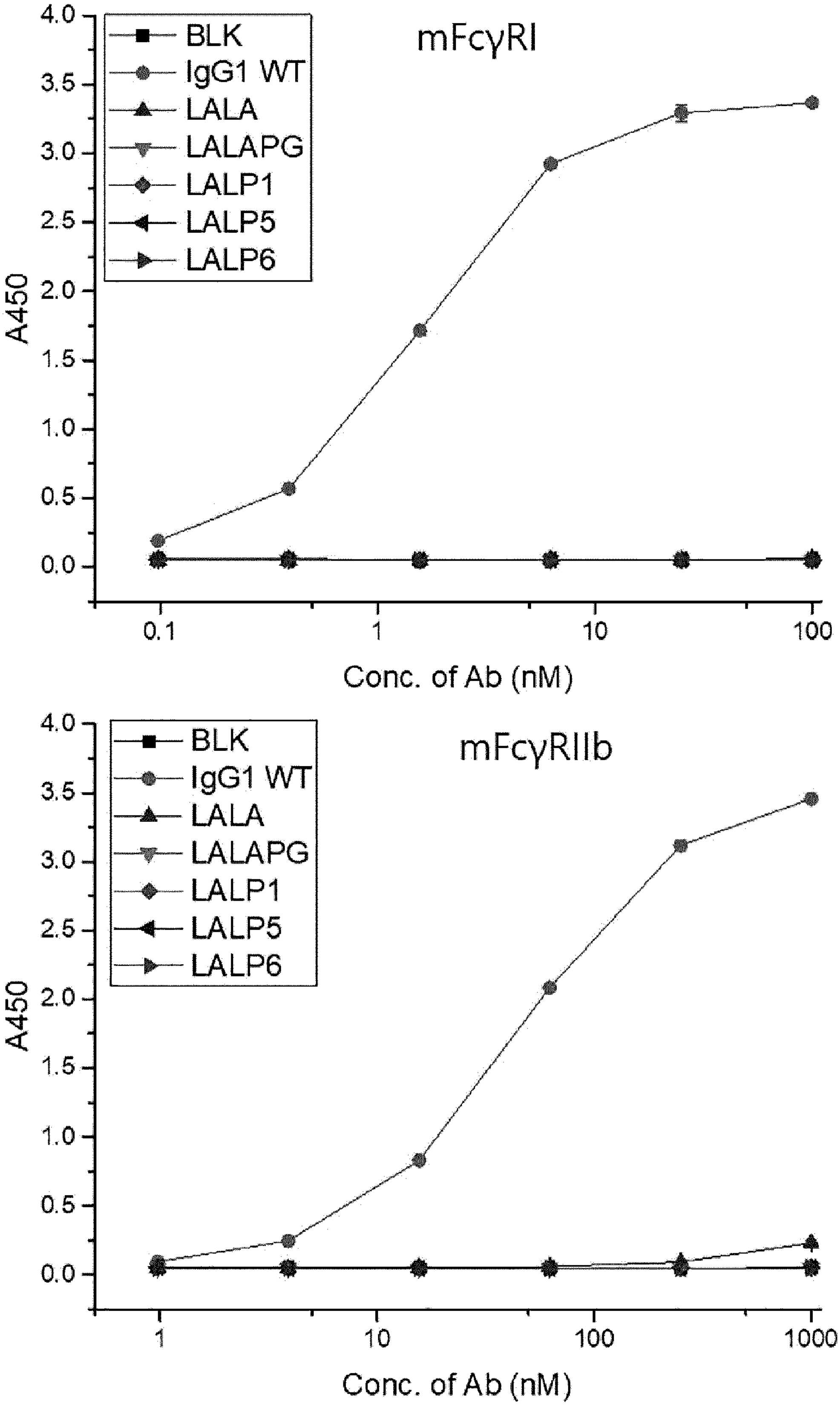
mFcγRI
BLK
IgG1 WT
LALA
LALAPG
LALP1
LALP5
LALP6
A450
Conc. of Ab (nM)
mFcγRIIb
BLK
IgG1 WT
LALA
LALAPG
LALP1
LALP5
LALP6
A450
Conc. of Ab (nM)

[FIG. 32B]

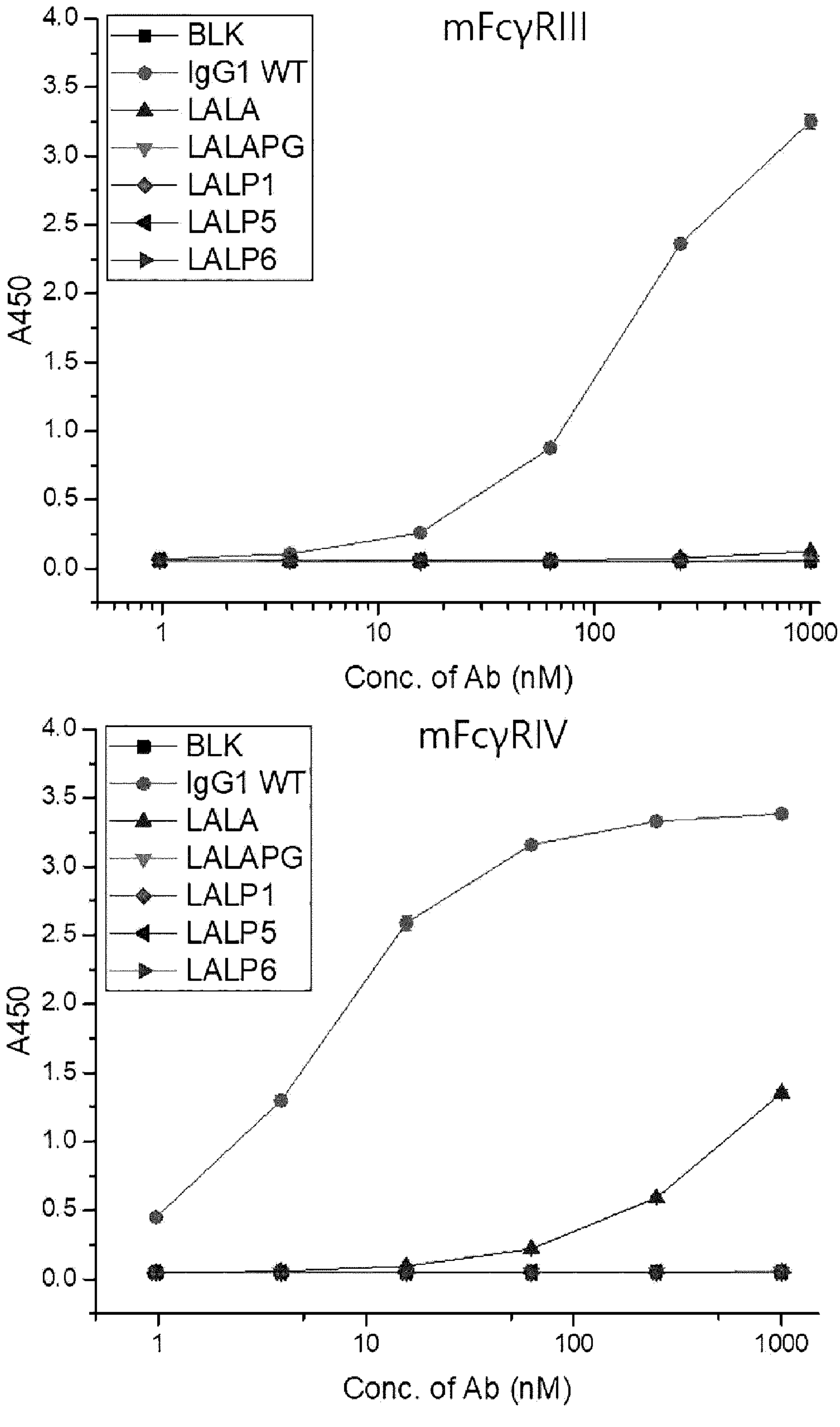
mFcγRIII
BLK
IgG1 WT
LALA
LALAPG
LALP1
LALP5
LALP6
A450
4.0
3.5
3.0
2.5
2.0
1.5
1.0
0.5
0.0
1
10
100
1000
Conc. of Ab (nM)
mFcγRIV
BLK
IgG1 WT
LALA
LALAPG
LALP1
LALP5
LALP6
A450
4.0
3.5
3.0
2.5
2.0
1.5
1.0
0.5
0.0
1
10
100
1000
Conc. of Ab (nM)

[FIG. 33A]

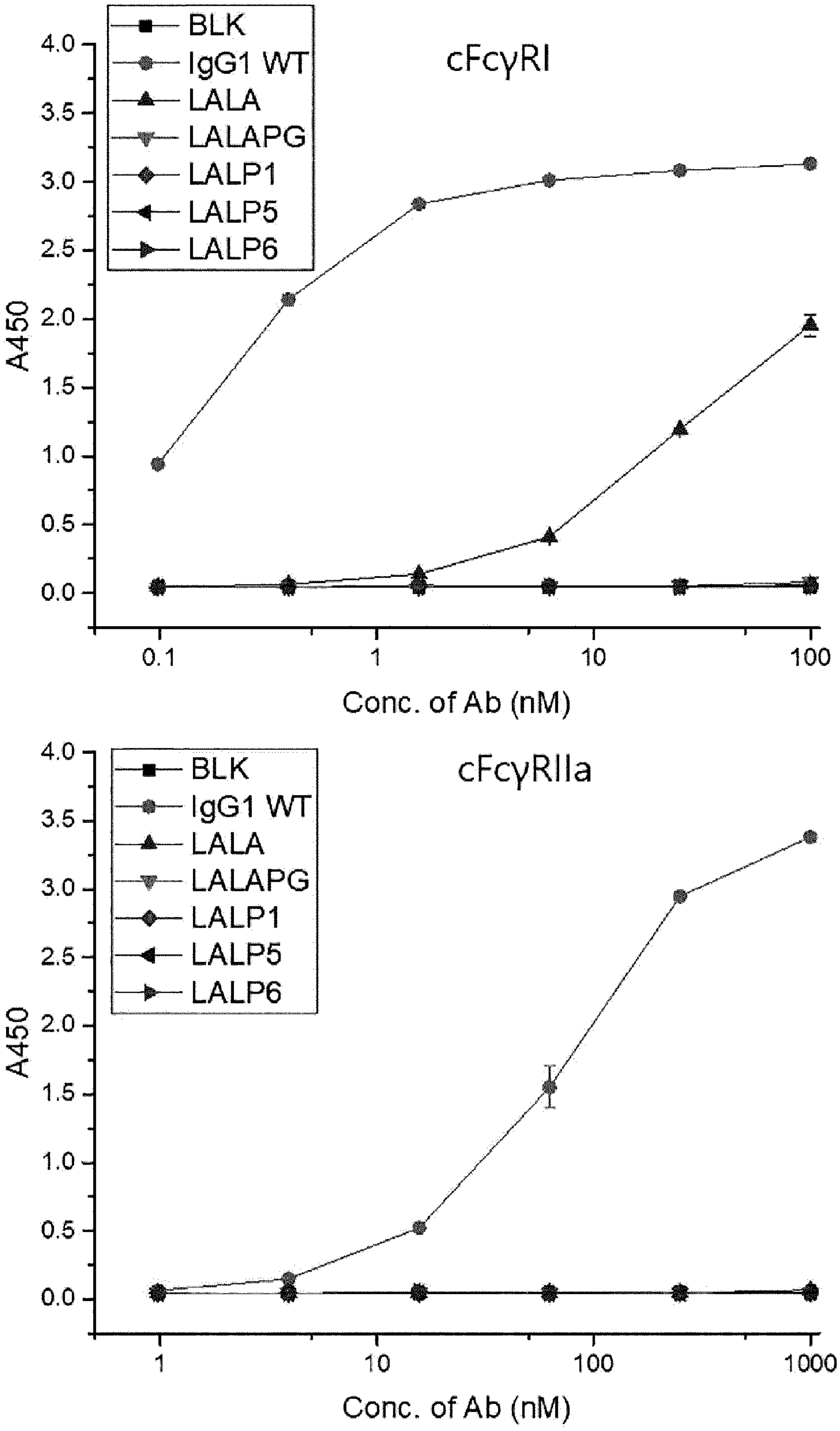
BLK
IgG1 WT
LALA
LALAPG
LALP1
LALP5
LALP6
cFcγRI
A450
Conc. of Ab (nM)
cFcγRIIa
A450
Conc. of Ab (nM)

[FIG. 33B]

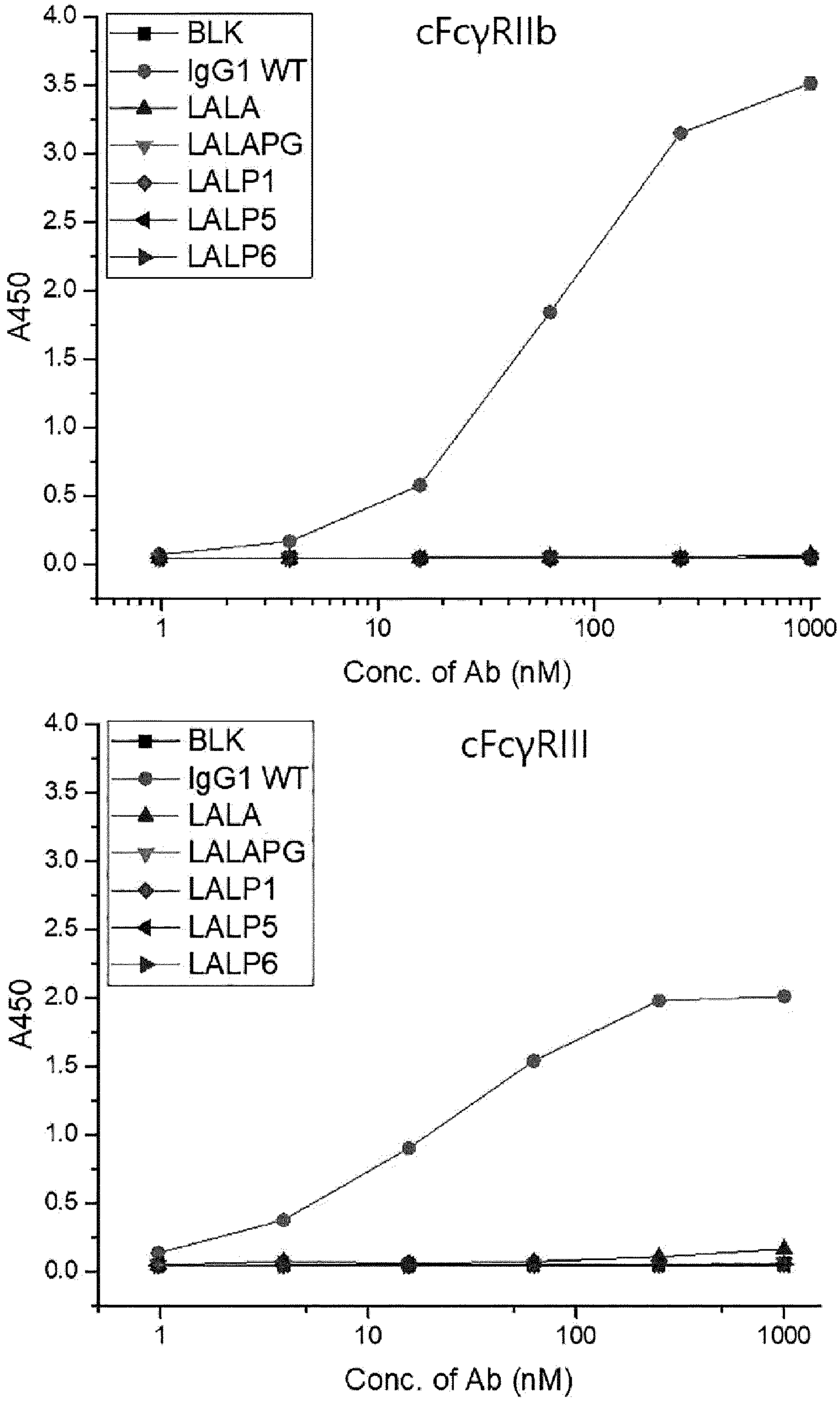
cFcγRIIb
BLK
IgG1 WT
LALA
LALAPG
LALP1
LALP5
LALP6
A450
Conc. of Ab (nM)
cFcγRIII
BLK
IgG1 WT
LALA
LALAPG
LALP1
LALP5
LALP6
A450
Conc. of Ab (nM)

# INTERNATIONAL SEARCH REPORT

International application No.
**PCT/KR2023/016105**

## A. CLASSIFICATION OF SUBJECT MATTER

**C07K 16/00**(2006.01)i; **C07K 16/32**(2006.01)i; **A61P 35/00**(2006.01)i; **A61K 39/00**(2006.01)i

According to International Patent Classification (IPC) or to both national classification and IPC

## B. FIELDS SEARCHED

Minimum documentation searched (classification system followed by classification symbols)

C07K 16/00(2006.01); A61K 38/08(2006.01); A61K 38/10(2006.01); C07K 16/18(2006.01); C07K 16/28(2006.01); C07K 16/42(2006.01); C12P 21/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 인간 항체(human antibody), Fc 도메인(Fc domain), 접합체(conjugate), 암(cancer), 진단(diagnosis)

## C. DOCUMENTS CONSIDERED TO BE RELEVANT

| Category* | Citation of document, with indication, where appropriate, of the relevant passages | Relevant to claim No. |
|---|---|---|
| X<br>Y | KR 10-0973564 B1 (XENCOR INC.) 03 August 2010 (2010-08-03)<br>See claims 4-6 and 19; and paragraphs [0073]-[0074] and [0083]-[0084]. | 1,3-15,18-19<br>2 |
| Y | US 7138370 B2 (OLINER, J. D. et al.) 21 November 2006 (2006-11-21)<br>See columns 78-79. | 2 |
| X | US 2011-0021755 A1 (LAZAR, G. A. et al.) 27 January 2011 (2011-01-27)<br>See paragraphs [0019]-[0020]. | 1,3-10 |
| X | US 8362210 B2 (LAZAR, G. A. et al.) 29 January 2013 (2013-01-29)<br>See paragraphs [0072]-[0073]. | 1,3-10 |
| A | US 2017-0058030 A1 (RESEARCH DEVELOPMENT FOUNDATION) 02 March 2017 (2017-03-02)<br>See entire document. | 1-15,18-19 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 January 2024** | **26 January 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2023/016105**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/KR2023/016105**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **16-17,20-21**
because they relate to subject matter not required to be searched by this Authority, namely:

Claims 16-17 and 20-21 pertain to a method for treatment of the human body, and thus pertain to a subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2023/016105**

| Patent document cited in search report | Publication date (day/month/year) | Patent family member(s) | Publication date (day/month/year) |
|---|---|---|---|
| KR 10-0973564 B1 | 03 August 2010 | CN 100867583 A | 22 November 2006 |
| | | CN 100867583 B | 23 May 2012 |
| | | EP 1620467 A2 | 01 February 2006 |
| | | JP 2007-525443 A | 06 September 2007 |
| | | JP 2009-209150 A | 17 September 2009 |
| | | JP 4578467 B2 | 10 November 2010 |
| | | JP 5226613 B2 | 03 July 2013 |
| | | KR 10-0890586 B1 | 25 March 2009 |
| | | KR 10-0956110 B1 | 10 May 2010 |
| | | KR 10-2005-0116400 A | 12 December 2005 |
| | | KR 10-2007-0116176 A | 06 December 2007 |
| | | KR 10-2009-0059170 A | 10 June 2009 |
| | | WO 2004-099249 A2 | 18 November 2004 |
| | | WO 2004-099249 A3 | 26 January 2006 |
| US 7138370 B2 | 21 November 2006 | CN 100596266 A | 16 March 2005 |
| | | CN 100596266 B | 28 April 2010 |
| | | CN 101787072 A | 28 July 2010 |
| | | CN 101787072 B | 15 July 2015 |
| | | CN 101812118 A | 25 August 2010 |
| | | EP 1434791 A2 | 07 July 2004 |
| | | EP 1434791 B1 | 07 October 2009 |
| | | EP 2070944 A1 | 17 June 2009 |
| | | EP 2070944 B1 | 26 September 2012 |
| | | EP 2157097 A1 | 24 February 2010 |
| | | EP 2311849 A1 | 20 April 2011 |
| | | EP 2311849 B1 | 16 August 2017 |
| | | EP 2316845 A1 | 04 May 2011 |
| | | EP 2316845 B1 | 16 January 2013 |
| | | JP 2005-514024 A | 19 May 2005 |
| | | JP 2009-273457 A | 26 November 2009 |
| | | JP 2010-189394 A | 02 September 2010 |
| | | JP 4573238 B2 | 04 November 2010 |
| | | JP 5432777 B2 | 05 March 2014 |
| | | KR 10-0976915 B1 | 18 August 2010 |
| | | KR 10-2004-0068119 A | 30 July 2004 |
| | | KR 10-2010-0038238 A | 13 April 2010 |
| | | US 2003-0229023 A1 | 11 December 2003 |
| | | US 2006-0122370 A1 | 08 June 2006 |
| | | US 2007-0072801 A1 | 29 March 2007 |
| | | US 2007-0093418 A1 | 26 April 2007 |
| | | US 2007-0093419 A1 | 26 April 2007 |
| | | US 2007-0225221 A1 | 27 September 2007 |
| | | US 2009-0054323 A1 | 26 February 2009 |
| | | US 2010-0286060 A1 | 11 November 2010 |
| | | US 2012-0141499 A1 | 07 June 2012 |
| | | US 2013-0158234 A1 | 20 June 2013 |
| | | US 2014-0275479 A1 | 18 September 2014 |
| | | US 7205275 B2 | 17 April 2007 |
| | | US 7666831 B2 | 23 February 2010 |
| | | US 7666832 B2 | 23 February 2010 |

Form PCT/ISA/210 (patent family annex) (July 2022)

**INTERNATIONAL SEARCH REPORT**
**Information on patent family members**

International application No.
**PCT/KR2023/016105**

| Patent document cited in search report | Publication date (day/month/year) | Patent family member(s) | Publication date (day/month/year) |
|---|---|---|---|
| | | US 7666839 B2 | 23 February 2010 |
| | | US 7723499 B2 | 25 May 2010 |
| | | US 7790674 B2 | 07 September 2010 |
| | | US 8129331 B2 | 06 March 2012 |
| | | US 9200040 B2 | 01 December 2015 |
| | | WO 03-057134 A2 | 17 July 2003 |
| | | WO 2003-057134 A3 | 29 April 2004 |
| US 2011-0021755 A1 | 27 January 2011 | US 10113001 B2 | 30 October 2018 |
| | | US 10584176 B2 | 10 March 2020 |
| | | US 2004-0110226 A1 | 10 June 2004 |
| | | US 2004-0132101 A1 | 08 July 2004 |
| | | US 2005-0054832 A1 | 10 March 2005 |
| | | US 2006-0024298 A1 | 02 February 2006 |
| | | US 2008-0051563 A1 | 28 February 2008 |
| | | US 2008-0057056 A1 | 06 March 2008 |
| | | US 2008-0154025 A1 | 26 June 2008 |
| | | US 2008-0161541 A1 | 03 July 2008 |
| | | US 2009-0010920 A1 | 08 January 2009 |
| | | US 2009-0215991 A1 | 27 August 2009 |
| | | US 2013-0261289 A1 | 03 October 2013 |
| | | US 2013-0273043 A1 | 17 October 2013 |
| | | US 2014-0073768 A1 | 13 March 2014 |
| | | US 2015-0010543 A1 | 08 January 2015 |
| | | US 2015-0030592 A1 | 29 January 2015 |
| | | US 2015-0031862 A1 | 29 January 2015 |
| | | US 2015-0232567 A1 | 20 August 2015 |
| | | US 2018-0208668 A1 | 26 July 2018 |
| | | US 2019-0071512 A1 | 07 March 2019 |
| | | US 7317091 B2 | 08 January 2008 |
| | | US 8188231 B2 | 29 May 2012 |
| | | US 8735547 B2 | 27 May 2014 |
| | | US 8753628 B2 | 17 June 2014 |
| | | US 8802823 B2 | 12 August 2014 |
| | | US 8937158 B2 | 20 January 2015 |
| | | US 9657106 B2 | 23 May 2017 |
| | | US 9663582 B2 | 30 May 2017 |
| US 8362210 B2 | 29 January 2013 | US 2011-0236375 A1 | 29 September 2011 |
| | | US 2013-0122001 A1 | 16 May 2013 |
| | | US 9475881 B2 | 25 October 2016 |
| | | WO 2011-091078 A2 | 28 July 2011 |
| | | WO 2011-091078 A3 | 01 December 2011 |
| US 2017-0058030 A1 | 02 March 2017 | US 10526408 B2 | 07 January 2020 |
| | | WO 2017-040380 A2 | 09 March 2017 |
| | | WO 2017-040380 A3 | 20 April 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 9209965 W
- WO 9413804 A
- WO 2012130831 A1


### Non-patent literature cited in the description


- Molecular Cloning - A Laboratory Manual. Cold Spring Harbor Laboratory Press, 2001
- Current Protocols in Molecular Biology. John Wiley & Sons
- Goodman and Gilman's The Pharmacological Basis of Therapeutics. Pergamon Press, 2001
- Remington's Pharmaceutical Sciences. Mack Publishing Co., 1990
- Remington's Pharmaceutical Science. Mack Publishing Company, 1990
- **S. T. JUNG et al.** Aglycosylated IgG variants expressed in bacteria that selectively bind FcyRI potentiate tumor cell killing by monocyte-dendritic cells. *Proceedings of the National Academy of Sciences USA (PNAS)*, 2010, vol. 107 (2), 604-609
- **S. T. JUNG et al.** Effective phagocytosis of low Her2 tumor cell lines with engineered, aglycosylated IgG displaying high FcyRIIa affinity and selectivity. *ACS Chemical Biology*, 2013, vol. 8 (2), 368-375
- **M. JO et al.** Engineered aglycosylated full-length IgG Fc variants exhibiting improved FcγRIIIa binding and tumor cell clearance. *mAbs*, 2018, vol. 2 (10), 278-289
- **H. W. YOON et al.** Optimal combination of beneficial mutations for improved ADCC effector function of aglycosylated antibodies. *Molecular Immunology*, 2019, vol. 114, 62-71